# EUROPEAN PATENT SPECIFICATION

(11) **EP 3 129 512 B1**
(45) Date of publication and mention of the grant of the patent: **28.03.2018**
(21) Application number: 15722601.0
(22) Date of filing: 11.04.2015
(51) Int. Cl.: C12Q 1/70

(54) **SCREENING TEST FOR DETECTING THE PRESENCE OF ONCOGENIC HPV VIRUSES**
SCREENINGTEST FÜR DEN NACHWEIS DER ANWESENHEIT ONKOGENER HPV-VIREN
EXAMEN DE DÉPISTAGE DESTINÉ À DÉTECTER LA PRÉSENCE DE VIRUS DU PAPILLOME HUMAIN (VPH)ONCOGÈNES

(30) Priority: 11.04.2014 PL 40786414
(43) Date of publication of application: 15.02.2017
(73) Proprietor: Centrum Onkologii - Instytut Im. Marii Sklodowskiej-Curie, 02-034 Warszawa (PL); CERVICO SP. Z O.O., 71-533 Szczecin (PL)
(72) Inventor: WYRWICZ,Lucjan, 02-863 Warszawa (PL); PODOLSKI, Jacek, 71-696 Szczecin (PL)
(74) Representative: Kondrat, Mariusz
(86) International application number: PCT/PL2015/050007
(87) International publication number: WO 2015/156693

(56) References cited:
- WO-A1-2011/094514
- WO-A2-2007/111998
- WO-A2-2009/129505
- WO-A2-2012/116220
- FATIH SAHINER ET AL: "Detection of major HPVs by a new multiplex real-time PCR assay using type-specific primers", JOURNAL OF MICROBIOLOGICAL METHODS, vol. 97, 1 February 2014 (2014-02-01), pages 44-50, XP055206690, ISSN: 0167-7012, DOI: 10.1016/j.mimet.2013.12.012
- C. E. DEPUYDT ET AL: "Comparison of MY09/11 consensus PCR and type-specific PCRs in the detection of oncogenic HPV types", JOURNAL OF CELLULAR AND MOLECULAR MEDICINE, vol. 11, no. 4, 1 July 2007 (2007-07-01), pages 881-891, XP055206847, ISSN: 1582-1838, DOI: 10.1111/j.1582-4934.2007.00073.x

## Description

### Technical Field

The invention relates to the field of diagnostics of cervical cancer, and in particular to a screening test for oncogenic HPV viruses, preferably based on LNA (locked nucleic acids) technology and an associated process, as a new tool in prevention and the early detection of cervical cancer.

### Background Art

Cervical cancer is a malignant tumour, which is still a major health and social problem worldwide. Every year approximately 500,000 new cases of this disease are diagnosed in the world, and more than half of the women diagnosed die of the disease. Although all women are vulnerable to this type of cancer, significant geographical differences of incidence and mortality have been noted.

Almost 80% of cases of cervical cancer are diagnosed in developing countries, in which this disease can even place first as a leading cause of death among women in comparison with other genital cancers. In these countries, an increase in the number of cases is expected in the coming years. Downward trends in incidence of cervical cancer and a lower mortality rate in developed countries are associated with the introduction of general and systematic prevention and better access to modem health care.

Poland belongs to those countries with a relatively high incidence and mortality rate of cervical cancer. It is the second most common malignant tumour in women up to 45 years of age. Every year approximately 3,500 Polish women are diagnosed with cervical cancer, and half of them die from this. The number of deaths of this type of cancer places Poland on one of the top positions in Europe.

The treatment of cervical cancer depends on the stage of the disease and comprises a local excision and radical surgery in early stages of the disease, radical radiotherapy in combination with brachytherapy and concurrent chemotherapy in advanced stages and palliative care in case of metastatic disease. Carboplatin and paclitaxel are most commonly used within chemotherapy treatment. Additional drugs may increase the survival time of patients in advanced cases. Recent reports suggest the possibility of the additional application of targeted therapy (bevacizumab) for this indication, which improves the parameters of life expectancy in palliative care; however, it significantly affects the cost of such treatment. In case of resistance to platinum, the second drug of choice is topotecan.

In 2006, vaccines aimed at preventing HPV infections, the major cause of incidences of cervical cancer, were placed on the market. A vaccine called Gardasil has been approved for use in the United States and within the European Union. Another vaccine against HPV infections is Cervarix.

Cervical cancer is one of the few malignant neoplasms, which, however, can be effectively prevented by proper prophylaxis and early diagnostics of medical conditions that are predisposed to its development (i.e. precancerous lesions, known as intraepithelial neoplasia). Cytological examinations of cervical smears aimed at detecting abnormal cells of stratified squamous or glandular epithelium regularly performed in women have been, so far, the basic and most effective method of such prophylaxis. Despite the undoubted value of cytological examination in a population-based screening for cervical cancer, the basic limitation of this method is its relatively low sensitivity of approx. 60%. According to many scientific studies, a combined cytological and molecular genetic examination for so-called oncogenic types of human papillomavirus (HPV), a group of 15 out of hundreds of known types of HPV, which directly cause the process of carcinogenesis in the epithelium of the cervix in some of the infected women (otherwise referred to as high-risk viruses, high-risk HPV, HR-HPV), is much more effective in detecting advanced precancerous lesions (so-called CIN2, CIN3, carcinoma *in situ).* In addition to the high sensitivity of a combined cytological and HPV examination (up to 100%), and only slightly lower specificity in comparison to cytological examination alone, the method is also characterised by a high so-called NPV (negative predictive value) index. Such combined examinations are especially recommended for women over 30 years of age. In addition to the use of HPV tests in primary screening for cervical cancer, as a supplement to cytological diagnostics, the determination of viral infections caused by the oncogenic HPV group is also recommended at the stage of so-called enhanced diagnostics in women with a detected presence of abnormal epithelial cells, especially in the case of diagnoses known as ASC-US and LSIL (recommendations of the Polish Gynaecological Society and international institutions). Execution of the test for the presence of oncogenic HPV types in women with certain abnormalities in a cytological preparation allows for a more specific further diagnosis and possible treatment.

Therefore, the common use of molecular genetic tests to detect oncogenic HPV types, as a supplement to population-based screening programmes based on cytological examinations of cervical smears, seems reasonable. Unfortunately, due to technical reasons (high complexity of tests, in which several dozen HPV types with low genome homology are analysed in a single assay) and the cost of commercially available HPV tests, such combined preventive exams are not common within population-based screening programmes, both in Poland and around the world, and they are limited exclusively to the stage of enhanced diagnostics in the case of detection of abnormal cells in cytological screening tests. Another problem is the lack of a uniform methodical standard for HPV tests.

The Hybrid-Capture (Digene) method is considered the gold standard for HPV diagnostics. However, this method has relatively low diagnostic sensitivity. In addition, the test allows for the detection of only some of the known oncogenic HPV types. Most of the tests available on the HPV diagnostics market are based on genotyping (determination of individual HPV types) methods, which generate high cost as well as time and labour consuming analyses and a reduction of specificity of the method for the detection of morphological abnormalities of the cervix. In recent years, a few tests approved for clinical use (CE-IVD-certified) based on different molecular genetic methods, using DNA amplification techniques (e.g., PCR) for detection of infections with HPV of the group characterised by a high-risk of cervical cancer development (with no distinction of type), appeared on the market. However, in general, they do not comprise an analysis of all oncogenic HPV types, and their high price prevents their common use in population-based screening tests of cervical cancer in Poland.

The PCR method in real time (real-time PCR) is now widely used in viral diagnostics, but it requires an individual approach for each investigated microorganism. To use this method for diagnostics, it is necessary to adequately select sequences, so-called primers and molecular probes, as well as reaction conditions, in order to ensure the highest clinical credibility of the test and, in particular, an adequate threshold of pathogen detection and diagnostic specificity. Preferably, the multiplex reaction allows for single-stage diagnosis of multiple HPV types, which is however an extremely difficult task and requires one to obtain a test characterised by a high sensitivity and specificity of detection.

Real-Time PCR assays in virology were reviewed by Mackay et al., 2002, Nucleic Acid Research, 30(6), 1292-1305. WO08/145366 (Univ. Milano Bicocca) described a method of identifying and quantifying oncogenic HPV nucleic acids. The method used 5 independent real-time PCR assays as a first line screen to identify the presence of HPV in a sample, and further TaqMan real-time PR assays performed on positive samples to determine the presence and the viral load of the most common oncogenic HPV types. In Quantitative Real-Time PCR: Methods and Protocols, Chapter 8 in Methods in Molecular Biology, vol. 1160:87-97 2014, Broccolo described a multiplex real-time PCR platform integrated into an automated extraction method for the rapid detection and measurement of oncogenic HPV DNA load from cervical samples. In this system, two multiplex qPCR assays (first line screening) were performed to detect and quantify HPV-16, -18, -31, -33, -45, -52, and -58 (HPV-18 and -45 measured together by single-fluorophore). In addition, a multiplex qPCR assay specific for HPV-18 and HPV-45 was used to type precisely the samples found to be positive for one of the two strains. Micalessi et al. described a TaqMan-based quantitative real time PCR assay for high-throughput detection of 17 HPV genotypes in seven multiplex reactions, and reported an analytical sensitivity of <100 copies (Clin. Chem. Lab. Med., 50(4):655-661 2011).

There is great demand for performing a test for the presence of oncogenic HPV types in order to implement the appropriate diagnostic and therapeutic procedure. The number of HPV tests performed is definitely not sufficient due to their high costs and limited availability on the market. The development of an inexpensive and clinically credible test for the presence of oncogenic HPV types will significantly improve the availability of modern diagnostics of medical conditions predisposing to cervical cancer. Therefore, the aim of the invention is to develop a diagnostic test which would allow for sensitive, specific and inexpensive detection of oncogenic HPV types in a cervical smear collected on the liquid medium (liquid based cytology - LBC).

### Disclosure of the Invention

The invention provides a method of determining if a sample tests positive for oncogenic human papillomavirus (HPV), the method comprising:
combining the sample with primers for real-time polymerase chain reaction (PCR) amplification of a target region of oncogenic HPV genomes, and a labelled pan-HPV probe capable of emitting a signal upon amplification of the target region, providing conditions for real-time PCR, and measuring for a signal emitted from the labelled pan-HPV probe, wherein detection of the signal indicates that the sample tests positive for oncogenic
   HPV,
characterised in that the pan-HPV probe is a labelled locked nucleic acid (LNA) molecule comprising sequence ACTTTCGTTT (SEQ ID NO: 25) or the reverse complement thereof.

Preferably, the pan-HPV probe is a labelled 10-nucleotide molecule of sequence consisting of ACTTTCGTTT (SEQ ID NO: 25) or the reverse complement thereof and/or the pan-HPV probe is labelled at its 5' end with a fluorophore and at its 3' end with a quencher, and wherein the signal emitted upon amplification of the target region is fluorescence.

Preferably, the primers are for amplification of a target region of any of HPV16, HPVI8, HPV31, HPV33, HPV35, HPV39, HPV45, HPV51, HPV52, HPV56, HPV58, HPV59, HPV66 or HPV68 or/and comprising combining the sample with a combination of primers for amplification of a target region of all of HPV16, HPV18, HPV31, HPV33, HPV35, HPV39, HPV45, HPV51, HPV52, HPV56, HPV58, HPV59, HPV66 orHPV68.

The primers may comprise one or more of the following primer pairs:
a) for HPV16 SEQ ID NO: 1 and SEQ ID NO: 14
b) for HPVI8 SEQ ID NO: 2 and SEQ ID NO: 15
c) for HPV33/33n SEQ ID NO: 3 and SEQ ID NO: 14
d) for HPV31 SEQ ID NO: 4 and SEQ ID NO: 14
e) for HPV35 SEQ ID NO: 5 and SEQ ID NO: 14
f) for HPV39 SEQ ID NO: 6 and SEQ ID NO: 14
g) for HPV45 SEQ ID NO: 7 and SEQ ID NO: 15
h) for HPV51 SEQ ID NO: 8 and SEQ ID NO: 16
i) for HPV52 SEQ ID NO: 9 and SEQ ID NO: 14
j) for HPV56/66 SEQ ID NO: 10 and SEQ ID NO: 14
k) for HPV58 SEQ ID NO: 11 and SEQ ID NO: 14 1) for HPV59 SEQ ID NO: 12 and SEQ ID NO: 17 m) for HPV68 SEQ ID NO: 13 and SEQ ID NO: 18
or/and wherein the primers comprise all of the primer pairs (a) to (m).

A method in accordance with the invention may comprise combining the sample with primers for amplification of a target region of HPV16, HPVI8, HPV31, HPV33, HPV35, HPV39, HPV45, HPV51, HPV52, HPV56, HPV58, HPV59, HPV66 and HPV68, detecting a signal from the labelled pan-HPV probe, and
concluding that the sample tests positive for HPV16, HPV18, HPV31, HPV33, HPV35, HPY39, HPY45, HPY51, HPY52, HPV56, HPY58, HPV59, HPY66 orHPV68.

A method in accordance with the invention may comprise combining the sample with primers for amplification of a target region of HPV16, HPV18, HPV31, HPV33, HPV35, HPY39, HPV45, HPV51, HPV52, HPV56, HPV58, HPV59, HPV66 and HPV68, detecting no signal from the labelled pan-HPY probe, and
concluding that the sample tests negative for HPV16, HPV18, HPV31, HPV33, HPY35, HPY39, HPY45, HPY51, HPV52, HPY56, HPV58, HPY59, HPV66 and HPY68.

A method according to the invention may further comprise
combining the sample with primers for amplification of a target region of HPV33n, and a labelled 33n probe capable of emitting a signal upon amplification of the target region, wherein the 33n probe and the pan-HPY probe carry the same label, providing conditions for real time-PCR, and
measuring for a signal emitted from the labelled 33n probe and / or the pan-HPV
   probe,
wherein detection of the signal indicates that the sample tests positive for oncogenic
   HPV,
characterised in that the 33n probe is a labelled locked nucleic acid (LNA) molecule comprising sequence ACTTTCGCTT (SEQ ID NO: 26) or the reverse complement thereof.

In such methods the 33n probe may be a labelled 10-nucleotide molecule of sequence consisting of ACTTTCGCTT (SEQ ID NO: 26) or the reverse complement thereof or/and the 33n probe is a labelled 10-nucleotide molecule of sequence consisting of ACTTTCGCTT (SEQ ID NO: 26), or the reverse complement thereof.

A method according to the invention, may comprise determining whether the sample tests positive for HPV16 or HPV18, the method comprising
combining the sample with primers for real-time PCR amplification of a target region of HPV16 and HPV18 genomes, and a labelled 16/18 probe capable of emitting a signal upon amplification of the target region,
providing conditions for real time PCR, and measuring for a
signal from the labelled 16/18 probe,
wherein detection of a signal indicates that the sample tests positive for HPVI6 or HPV18,
characterised in that the 16/18 probe is a labelled locked nucleic acid (LNA) molecule comprising sequence ATATCWGATGACGAG (SEQ ID NO: 27) or the reverse complement thereof. The 16/18 probe may be a labelled 15-nucleotide molecule of sequence consisting of ATATCWGATGACGAG (SEQ ID NO: 27) or the reverse complement thereof.

The 16/18 probe may carry a different label from the pan-HPV probe.

The 16/18 probe may be labelled at its 5' end with a fluorophore and at its 3' end with a quencher, and wherein the signal emitted upon amplification of the target region is fluorescence.

The 16/18 probe may emit a fluorescent signal at a wavelength different from that of the pan- HP V probe.

The primers for use in a method according to the invention for determining whether the sample tests positive for HPV16 or HPV18 may comprise one or both of the following primer pairs:
a) for HPV16 SEQ ID NO: 19 and SEQ ID NO: 20
b) for HPVI8 SEQ ID NO: 21 and SEQ ID NO: 22.

A method according to the invention may further comprise conducting a positive control for the real-time PCR, comprising
combining the sample with primers for real-time PCR amplification of a control DNA sequence, preferably human DNA_present in the sample, and a labelled control probe capable of emitting a signal upon amplification of the control DNA sequence, preferably human DNA
providing conditions for real-time PCR, and measuring
for a signal from the control probe,
   wherein detection of the signal indicates that the real-time PCR is functional.

In a method according to the invention the sample may be a liquid-based cytology sample from a human cervical smear.

The invention further provides a composition comprising a labelled nucleic acid probe comprising nucleotide sequence ACTTTCGTTT (SEQ ID NO: 25) or the reverse complement thereof, and a detectable label. The probe may comprise LNA nucleotides. Preferably, the probe is a 10-nucleotide molecule of sequence consisting of ACTTTCGTTT (SEQ ID NO: 25) or the reverse complement thereof, labelled with a fluorophore and a quencher. The probe may be labelled at its 5' end with a fluorophore and at its 3' end with a quencher.

The invention additionally provides the use of a composition according to the invention in performing real-time PCR for detection of HPV.

Surprisingly, the inventors realised the above objective to obtain a diagnostic test for the presence of oncogenic HPVs, characterised by many advantages, such as ease of implementation, low cost, favourable parameters: high sensitivity, clinical effectiveness, specificity, and a clinically desired detection threshold for HPV16/18 and other oncogenic HPV types. Moreover, as demonstrated below, this test can potentially allow for resignation from a cytological examination in a large population of HR-HPV-negative women.

The HPV genome has approximately 8 thousand nucleotides, and the length of continuous sequences with full homology between oncogenic types does not exceed several dozen base pairs. Such great diversity of HPVs does not allow for the design of a minimal amount of hybridisation probes, which would enable the detection of all oncogenic HPV viruses in one reaction. This problem can be solved using modified LNA (locked nucleic acids) nucleotides in a molecular probe. LNA nucleotides contain an additional methylene bridge within the molecule of deoxyribose, which, due to the change of conformation and rigidifying the molecule, increases the melting point of oligomers built from such nucleotides and the stability of the bonds between complementary nucleotides in the hybridisation process. Properties of LNA were reviewed by Vester & Wengel, Biochemistry 43(42): 13233-13241 2004. According to the invention, thanks to the use of LNA, it is possible to advantageously apply significantly shorter, in comparison to traditional ones, TaqMan type molecular probes, with a high specificity of hybridisation into the PCR amplification product at a temperature optimal for the reaction, in a PCR. The use of significantly shorter 8-10 nucleotide long LNA-TaqMan probes (in comparison to 20-30 nucleotides in traditional probes) allows for their easier fitting into short (of even 10-12 base pairs) homologous regions between different oncogenic HPV types and consequently for the development of a new diagnostic test.

There is a high demand for tests using LNA technology, which would allow for obtaining clinically relevant expertise within the field of prevention and diagnostics of cervical cancer. The patients also want to be subjected to modern preventive exams that would be highly sensitive and financially available.

Accordingly, a test called HR-HPV, preferably based on a multiplex real-time PCR technique (multiplex real-time PCR), performed in a single sealed test tube, using TaqMan type molecular probes, specially designed with bioinformatic methods, was developed. The probes employed LNA innovative technology, which had not been used in the tests for the presence of HPV before.

Preferably, the reduction of the length of the probes to 8-10 nucleotides allowed for a significant decrease of their number, from 15-16 to merely several (optimally 2-3). Preferably, it also enabled the carrying out of a multiplex real-time PCR reaction in a single test tube in contrary to most of the tests present on the market, which are carried out in several test tubes. In one embodiment of the invention, LNA probes were optimally labelled with maximally 3 different universal fluorescent dyes with different emission spectra of fluorescent light, which allows for performing the tests in most commercially available PCR apparatuses, and not only in devices designed for the test of a specific producer.

In order to implement the invention, a kit of primers and probes allowing for identification and amplification of homologous regions in all oncogenic HPV types and, preferably, an internal control, being a selected sequence of human DNA and/or a synthetic fragment of DNA, was designed and fitted. In one embodiment of the invention, preferably, the reaction was conducted in a single test tube, and preferably, the readout of the reaction was performed in maximally 4 detection channels used in the majority of real-time PCR apparatuses present on the market.

Two 10-nucleotide LNA-TaqMan probes allowing for detection of infections with high-risk HPV (HR-HPV) in one real-time PCR reaction were designed. The first of them, HRP (pan- HPV), is complementary to all oncogenic HPV viruses, while the second one, HRP33n, is complementary to the identified variant of HPV33n, comprising polymorphism of one A/G nucleotide at the site where the probe is attached. Preferably, the 5' ends of HRP pan-HPV and HRP33n probes were labelled with a FAM (fluorescein) fluorescent dye, while the 3' ends with a BHQ1 quencher (Black Hole Quencher 1).

The readout of a real-time PCR for HRP pan-HPV and HRP33n probes was performed at a wavelength of 530 nm in a channel designed to read out FAM dye.

Moreover, preferably, an internal control for the PCR test was designed. A system consisting of a synthetic short DNA fragment was used and an oligonucleotide comprising 77 base pairs was designed as a target. A system of 4 primers and a 10-nucleotide LNA-TaqMan (ICP) probe was designed for amplification and detection of the synthetic internal control. An optimal combination of 2 primers for the PCR was selected. The ICP probe was labelled with ROX dye at its 5' end, and with a BHQ2 quencher at its 3' end. The readout of the real-time PCR for the ICP probe was performed at a wavelength of 610 nm (channel of the ROX dye).

The aim of the study was to possibly design a small kit of primers which would enable the efficient amplification of all oncogenic HPV types in clinical samples with sufficient sensitivity and specificity, with a possible complete elimination of any adverse effects which may occur during PCR, preferably conducted in real time, preferably in several detection channels.

As a result of conducted research, a common pool of primers for high-risk HPV was developed. The sequences were selected in a way so that the total number of primers was as small as possible. Therefore, some sequences of primers are common to several HPV types. Thus, the common reverse primer ("reverse") for 16, 31, 33, 33n, 35, 39, 52, 56, 58, HPV types and the common reverse primer ("reverse") for 18 and 45 HPV types were selected. Furthermore, common forward primers ("forward") were selected for 56 and 66 types and 33 and 33n types, respectively.

According to the embodiment of the invention, preferably, the final mixture of the HR-HPV test of the invention comprises 18 primers (13 forward primers and 5 reverse primers) and 2 LNA-TaqMan probes for the detection of 15 oncogenic HPV types, 2 primers and 1 TaqMan probe for synthetic internal control.

Using selected clinical samples, previously examined with the well-known PapilloCheck test, which detects 24 types of HPV (approx. 50 clinical samples containing the DNA of a single oncogenic HPV type, 30 clinical samples containing the DNA of a non-oncogenic HPV type, 50 clinical samples containing co-infections and 30 negative samples (free from 24 types of HPV, including all oncogenic types), it was demonstrated that an adequate combination of primers and molecular probes, according to the invention, allows for the specific detection of oncogenic HPV types (including the HPV33n variant) and produces negative results in the case of clinical samples containing a non-oncogenic virus type and negative clinical samples. Moreover, the reaction using the above-mentioned 24 primers, 3 LNA probes and one internal control (IC) TaqMan Probe did not generate products of the type primer-dimer or other non-specific PCR amplification products.

### Description of the Drawings

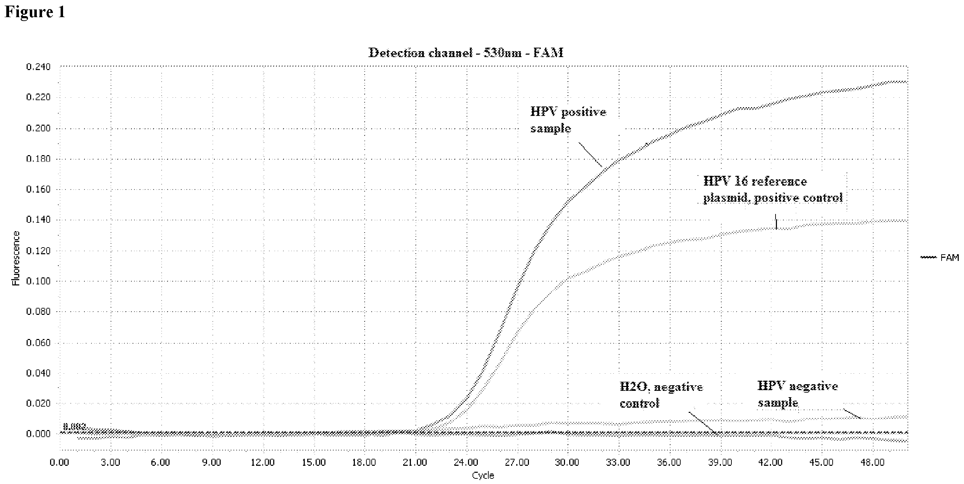

An embodiment of the subject of the invention was shown in the drawing, in which figure 1 presents the results obtained in an exemplary test using a real-time PCR for all groups of oncogenic HP Vs, with a positive control sample, negative control sample and negative clinical sample. A detection channel of 530 nm was used.
Figure 2 shows the results obtained in an exemplary embodiment of a test using a real-time PCR for 16 and 18 HPV types, with a positive control sample, negative control sample and negative clinical sample. A detection channel of 560 nm was used.
Figure 3 shows the results obtained in an exemplary embodiment of a test using a real-time PCR for the internal control (IC) of the test, with a positive control sample, negative control sample and negative clinical sample. A detection channel of 610 nm was used.
Figures 4A - D show sequences of example probes and primers according to various embodiments of the invention, and corresponding regions of the HPV genome to which they hybridise.

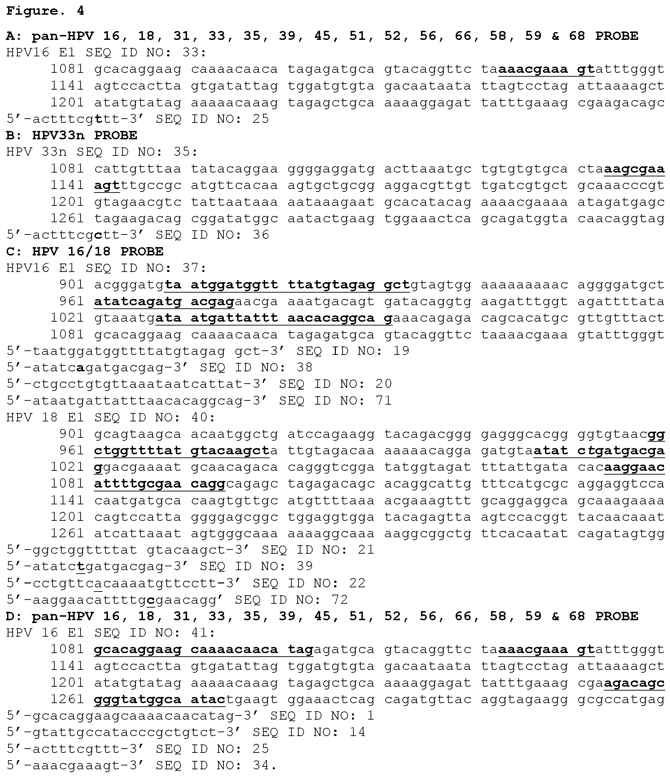

Figure 4A: shows a region of the HPV16 El gene (SEQ ID NO: 33) that is amplified and targeted by the HR-HPV (pan-HPV) probe (SEQ ID NO: 25) for detection of HPV 16, 18, 31, 33, 35, 39, 45, 51, 52, 56, 66, 58, 59 and 68; the target sequence to which the probe hybridizes (SEQ ID NO: 34) is shown in bold and underlined. The sequence of the HR-HPV (pan-HPV) Probe (5'-actttcgttt-3', SEQ ID NO: 25 / 29), which is the reverse complement of the target sequence, is also shown.
Figure 4B shows a region of the HPV 33n El gene (SEQ ID NO: 34) that is amplified and targeted by the HPV 33n probe for detection of HPV33n; the target sequence (SEQ ID NO: 36) to which the probe hybridizes is shown in bold and underlined). The sequence of the HR- HPV 33n Probe (5'-actttcgctt-3', SEQ ID NO: 26 / 30) which is the reverse complement of the target sequence, is also shown.
Figure 4C shows the regions of the HPV 16 El gene (SEQ ID NO: 37) and HPV 18 El gene (SEQ ID NO: 40) that are amplified for targeting by the HPV16/18 probe. The target sequence to which Primer HPV 16 F HEX (SEQ ID NO: 19) and Primer HPV 16 R HEX (SEQ ID NO: 20) hybridise for amplification and the target sequence (SEQ ID NO: 71) to which the HPV 16/18 probe (SEQ ID NO: 38) hybridizes are shown in bold and underlined. The target sequences to which Primer HPV 18 F HEX (SEQ ID NO: 21) and Primer HPVI8 HEX (SEQ ID NO: 22) hybridise for amplification and the target sequence to which the HPV16/18 probe (SEQ ID NO: 39) hybridizes are shown in bold and underlined. Note that the HPV16/18 probe shown (5'-atatctgatgacgag-3' (SEQ ID NO: 39)) has a "t" nucleotide as marked, whereas the HPV16/18 probe SEQ ID NO: 38 has an "a" residue at this position. The reverse primer HPV 18 R HEX has an "a" nucleotide where indicated, whereas for the target sequence shown, the complement would normally be a "g" nucleotide; the native HPV sequence has a "t" nucleotide at this position, hence the reverse primer used to target this sequence has an "a" at this position.
Figure 4D also shows the region of the HPV16 El gene (SEQ ID NO: 41) that is amplified and targeted by the HR-HPV (pan-HPV) probe for detection of HPV 16, 18, 31, 33, 35, 39, 45, 51, 52, 56, 66, 58, 59 and 68. The target sequence to which the primers HPV 16 F (SEQ ID NO: 1) and HPV 16 / 31/ 33 / 33n / 35 / 39 / 52 / 56 / 58 / 66 R (SEQ ID NO: 14) hybridise are shown in bold and underlined. The target sequence (SEQ ID NO: 34) to which the pan-HPV probe (SEQ ID NO: 25) hybridizes is shown in bold and underlined.

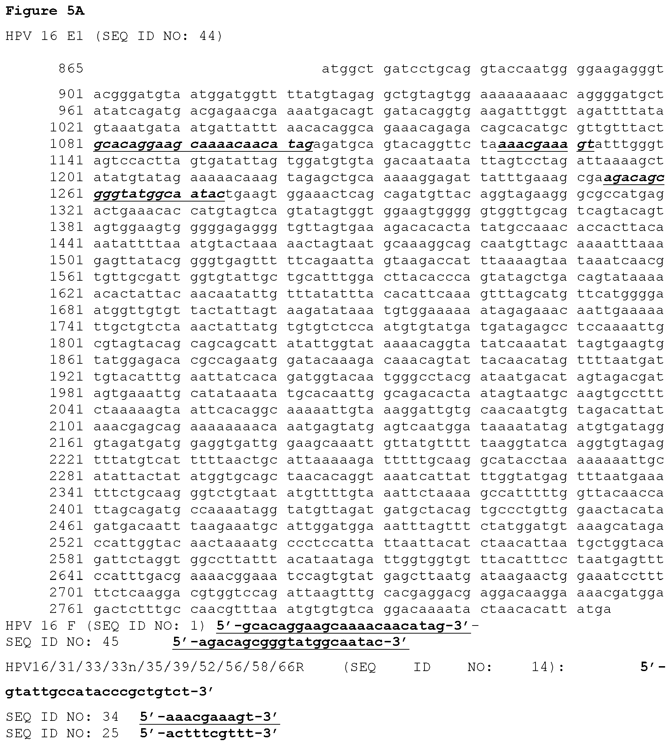

Figures 5A - M show sequences of the high risk HPV genome El regions for HPV strains 16, 18, 33, 31, 35, 39, 45, 51, 52, 56, 58, 59, 66 and 68, with the sequences of probes and primers according to various embodiments of the invention, and corresponding regions of the HPV genome to which they hybridise for detection in the first detection channel 530nm FAM. Figure 5A shows the HPV 16 El gene nucleotides 865 to 2814 (SEQ ID NO: 44), marked to show the positions of the Forward primer HPV 16 (SEQ ID NO: 1)
   5'-gcacaggaagcaaaacaacatag-3' and the region of the HPV 16 El gene targeted by the reverse primer HPV 16/31/33/33n/35/39/52/56/58/66R 5'-agacagcgggtatggcaatac-3' (SEQ ID NO: 45). Also shown is the Reverse primer HPV16/31/33/33n/35/39/52/56/58/66R 5'- gtattgccatacccgctgtct-3 ' (SEQ ID NO: 14) which is the reverse complement of the region of the HPV 16 El gene shown in SEQ ID NO: 45. The 5'-aaacgaaagt-3' sequence is the pan- HPV Probe target sequence (SEQ ID NO: 34) for hybridization of the 5'-actttcgttt-3' pan- HPV Probe (SEQ ID NO: 25, which is the reverse complement of SEQ ID NO: 34). The target region of HPVI6 amplified for detection with the pan-HPV probe is nucleotides 1081 to 1274.

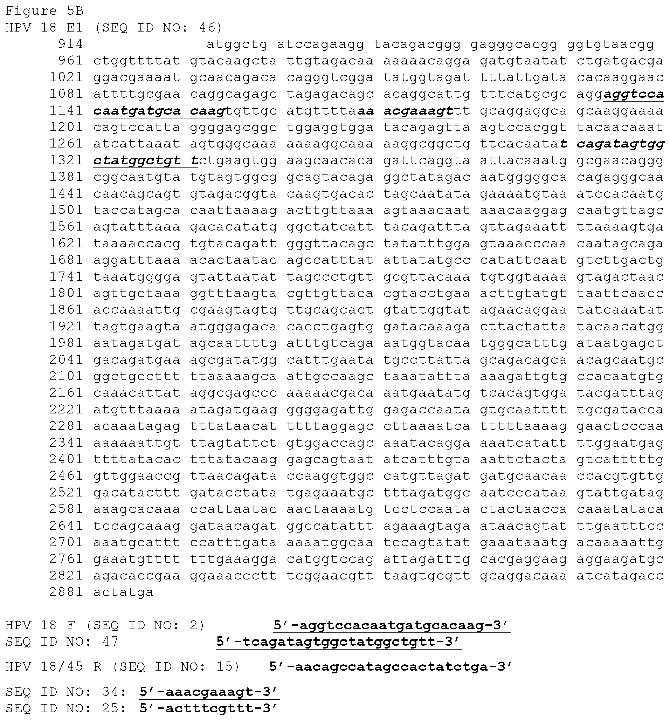

Figure 5B shows the HPV 18 El gene nucleotides 914 to 2887 sequence (SEQ ID NO: 46), marked to show the positions of the Forward primer HPV 18 5'-aggtccacaatgatgcacaag-3' (SEQ ID NO: 2) and the target region of the HPVI8 El genome 5'-tcagatagtggctatggctgtt-3' (SEQ ID NO: 47) that is targeted by the reverse primer. The Reverse primer HPV 18/45 R 5'- aacagccatagccactatctga-3 ' (SEQ ID NO: 15), is shown, this is the reverse complement of the target region of the HPV18 El genome shown in SEQ ID NO: 47. Also shown is the 5'- aaacgaaagt-3' (SEQ ID NO: 34) pan-HPV probe target sequence for hybridization of the 5'- actttcgttt-3' (SEQ ID NO: 25) pan-HPV Probe (SEQ ID NO: 34 is the reverse complement of
   SEQ ID NO: 25). The target region of HPV16 amplified for detection using the pan-HPV probe is nucleotides 1134 to 1331.

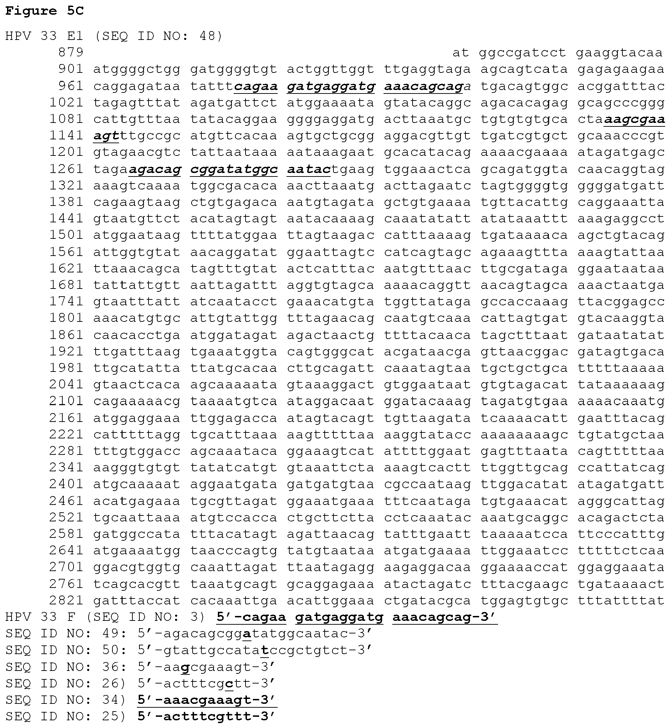

Figure 5C shows the HPV 33 El gene nucleotides 879 to 2813 (SEQ ID NO: 48) marked to show the Forward primer for HPV 33 5'-cagaagatgaggatgaaacagcag-3' (SEQ ID NO: 3), and the target binding site of the reverse primer HPV16/31/33/33n/35/39/52/56/58/66R 5'agacagcggatatggcaatac3'(SEQ ID NO: 49) in which there is a base difference a/g as marked relative to the target sequence for the reverse primer HPV 16/31/35/39/52/56/58/66R, thus 5'-gtattgccatatccgctgtct-3' (SEQ ID NO: 50) shows a mismatch c/t in the reverse complement when compared to the primer (SEQ ID NO: 14) that is used for amplification. The sequence 5'-aagcgaaagt-3' (SEQ ID NO: 36) is the HR-HPV 33n region targeted by the HPV 33n Probe, this has a nucleotide difference a/g compared to the consensus region targeted by the pan-HPV probe (SEQ ID NO: 25). The sequence 5'-actttcgctt-3' (SEQ ID NO: 26) is the HR-HPV Probe 33n (reverse complement of SEQ ID NO: 36) which has a nucleotide difference t/c when compared to the pan-HPV probe (SEQ ID NO: 25). The sequence 5'-aaacgaaagt-3' (SEQ ID NO: 34) is the target sequence for the pan-HPV Probe used for detection of HPV 33. The sequence 5'-actttcgttt-3' (SEQ ID NO: 25) is the pan- HPV Probe used for detection of HPV 33 (SEQ ID NO: 25 is the reverse complement of SEQ ID NO: 34). The target region of HPV 33 (and 33n) amplified for detection using the pan- HPV probe is nucleotides 976 to 1285.

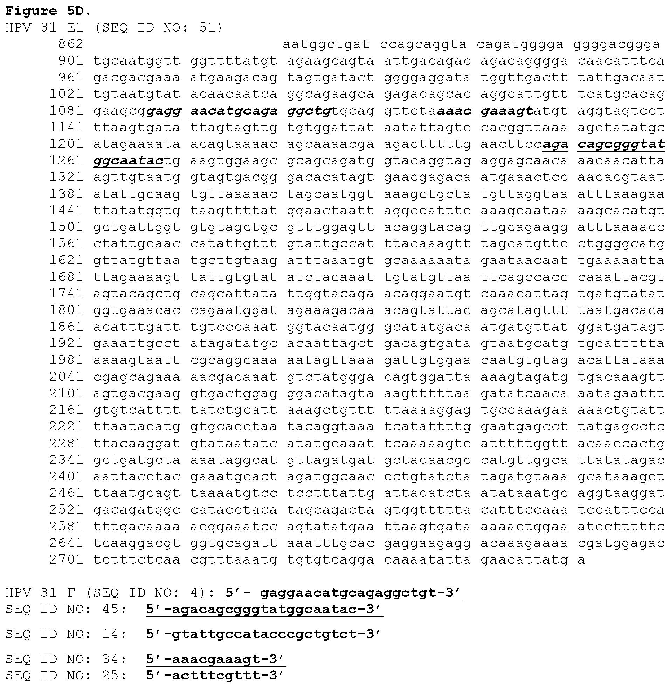

Figure 5D shows the HPV 31 El gene nucleotides 862 to 2751 nucleotides (SEQ ID NO: 51), marked to show the positions of the Forward primer HPV 31 5'-gaggaacatgcagaggctgt-3' (SEQ ID NO: 4) and the target sequence for the Reverse primer HPV16/31/33/33n/35/39/52/56/58/66R 5'-agacagcgggtatggcaatac-3' (SEQ ID NO: 45). The sequence 5'-gtattgccatacccgctgtct-3' (SEQ ID NO: 14) is the reverse primer for HPV16/31/33/33n/35/39/52/56/58/66R (SEQ ID NO: 14 is the reverse complement of the target region shown in SEQ ID NO: 45). The sequence 5'-aaacgaaagt-3' (SEQ ID NO: 34) is the target sequence for the pan-HPV Probe used for detection of HPV 31. The sequence 5'- actttcgttt-3 ' (SEQ ID NO: 25) is the pan-HPV Probe used for detection of HPV 31 (SEQ ID NO: 25 is the reverse complement of SEQ ID NO: 34). The target region amplified for detection of HPV 31 is nucleotides 1087 to 1268.

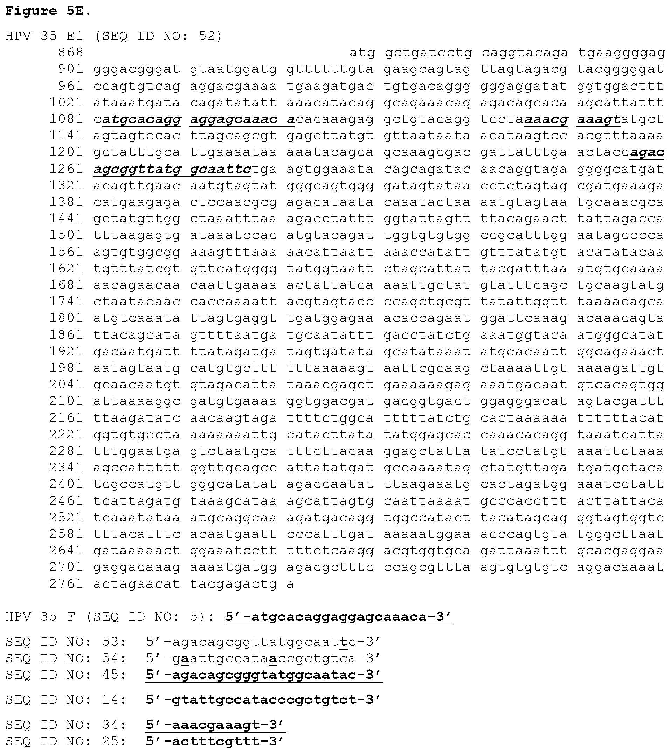

Figure 5E shows the HPV 35 El gene nucleotides 868 to 2781 nucleotides (SEQ ID NO: 52), marked to indicate the positions of the Forward primer HPV 35 5'-atgcacaggaggagcaaaca-3' (SEQ ID NO: 5), and the target region for the Reverse primer HPV16/31/33/33n/35/39/52/56/58/66R, the genomic sequence 5'-agacagcggttatggcaattc-3' (SEQ ID NO: 53) shows the two nucleotide differences t/g and t/a between the target sequence of HPV35 and the target sequence for the reverse primer. The sequence 5'-gaattgccataaccgctgtca-3' (SEQ ID NO: 54), complementary to SEQ ID NO: 53, shows the two nucleotide differences t/a and a/c relative to the reverse primer (SEQ ID NO: 14). The sequence 5'-agacagcgggtatggcaatac-3' (SEQ ID NO: 45) is the target sequence for the reverse primer HPV16/31/33/33n/35/39/52/56/58/66R. The sequence 5'- gtattgccatacccgctgtct-3 ' (SEQ ID NO: 14) is the Reverse primer HPV16/31/33/33n/35/39/52/56/58/66R used for amplification of HPV 35 (SEQ ID NO: 14 is the reverse complement of SEQ ID NO: 45). The sequence 5'-aaacgaaagt-3' (SEQ ID NO: 34) is the target consensus sequence for the pan-HPV Probe used for detection of HPV 35. The sequence 5'-actttcgttt-3' (SEQ ID NO: 25) is the pan-HPV Probe used for detection of HPV 35 (which is the reverse complement of SEQ ID NO: 34). The target region of the HPV 35 El gene amplified for detection is nucleotides 1082 to 1276.

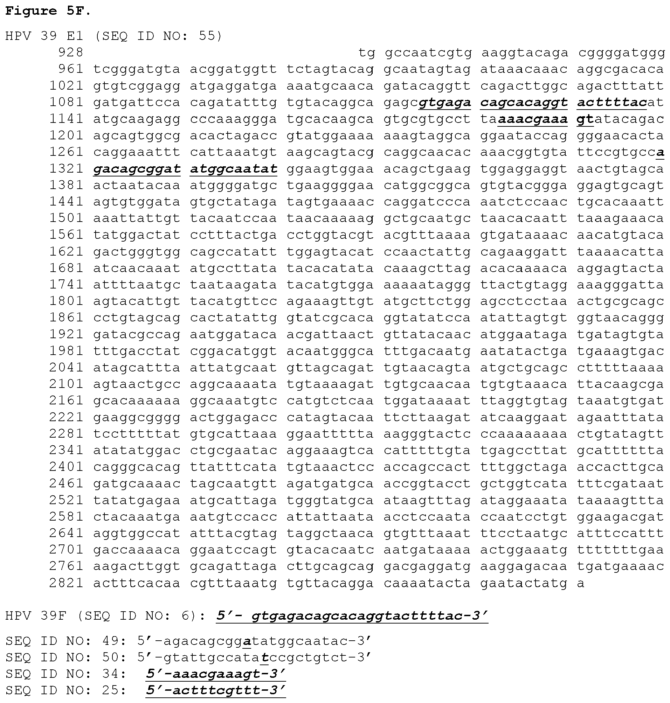

Figure 5F shows the HPV39 El gene nucleotides 928 to 2871 (SEQ ID NO: 55), marked to show the position of the Forward primer 39F 5'- gtgagacagcacaggtacttttac-3' (SEQ ID NO: 6) and the target region for the Reverse primer HPV16/31/33/35/39/52/56/58/66R 5'-agacagcggatatggcaatac-3' (SEQ ID NO: 49) which shows the nucleotide difference a/g between the target sequence of HPV 39 and the target sequence for the reverse primer. The sequence 5'-gtattgccatatccgctgtct-3' (SEQ ID NO: 50) shows the nucleotide difference c/t in the reverse complement of SEQ ID NO: 49, the reverse primer used for amplification of HPV16/31/33/35/39/52/56/58/66R (SEQ ID NO: 14) has a "c" nucleotide at the marked position. The sequence 5'-aaacgaaagt-3' (SEQ ID NO: 34) is the target consensus sequence for the pan-HPV Probe used for detection of HPV 39. The sequence 5'-actttcgttt-3' (SEQ ID NO: 25) is the pan-HPV Probe used for detection of HPV 39 (SEQ ID NO: 25 is the reverse complement of SEQ ID NO: 34). The target amplified for detection of HPV 39 is nucleotides 1115-1339.

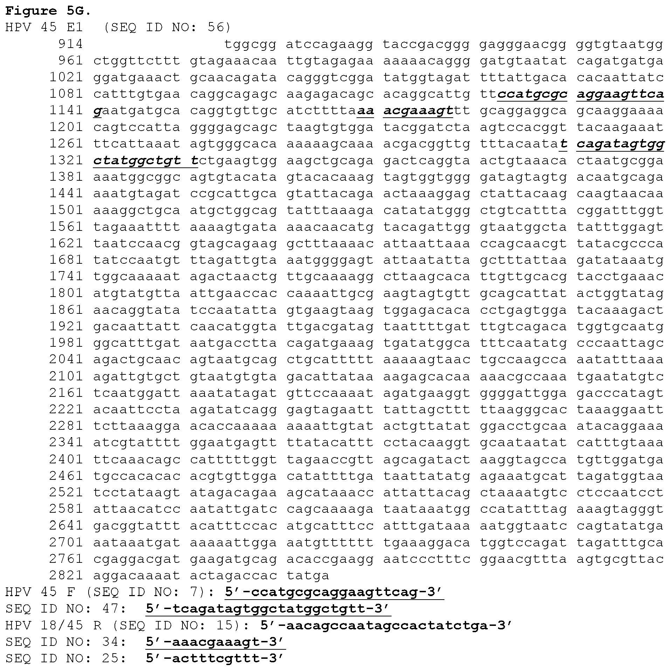

Figure 5G shows the HPV 45 El gene nucleotides 914 to 2845 (SEQ ID NO: 56), marked to indicate the Forward primer HPV 45 F 5'-ccatgcgcaggaagttcag-3' (SEQ ID NO: 7), and the target region for the Reverse primer HPV 18/45 R 5'-tcagatagtggctatggctgtt-3' (SEQ ID NO: 47), this region is targeted by the reverse primer for both HPV 18 and 45. The sequence 5'- aacagccaatagccactatctga-3' (SEQ ID NO: 15) is the reverse primer for HPV 18/45 R (reverse complement of SEQ ID NO: 47). The sequence 5'-aaacgaaagt-3' is the target consensus sequence for the pan-HPV Probe used for detection of HPV 45. The sequence 5'-actttcgttt-3' (SEQ ID NO: 25) is the pan-HPV Probe used for detection of HPV 45 (SEQ ID NO: 25 is the reverse complement of SEQ ID NO: 34). The target amplified for detection of for HPV 45 is nucleotides 1123-1331.

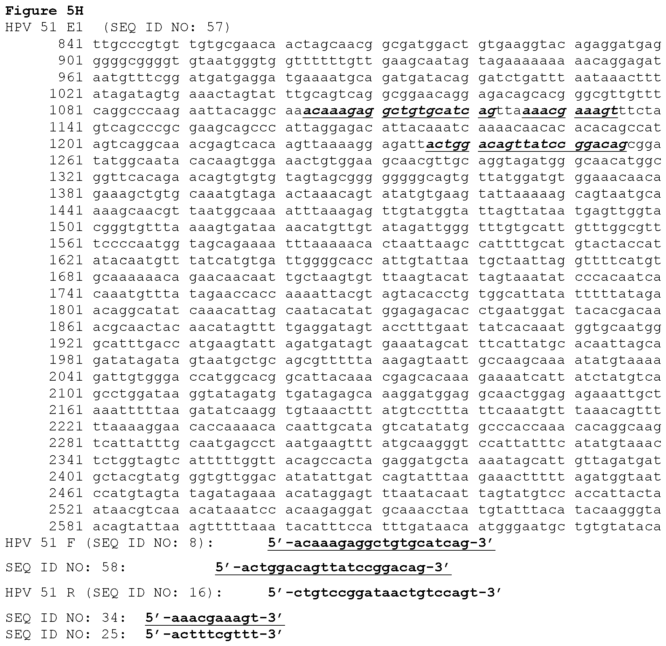

Figure 5H shows the region of the HPV51 genome that contains the HPV51 gene (SEQ ID NO: 57), marked to show the Forward primer HPV 51 F 5'-acaaagaggctgtgcatcag-3' (SEQ ID NO: 8) and the target sequence for the Reverse primer HPV 51 R 5'-actggacagttatccggacag- 3' (SEQ ID NO: 58). The sequence 5'-ctgtccggataactgtccagt-3' (SEQ ID NO: 16) is the reverse primer for HPV 51 R (reverse complement of SEQ ID NO: 58). The sequence 5'- aaacgaaagt-3' (SEQ ID NO: 34) is the target consensus sequence for the pan-HPV Probe used for detection of HPV 51. The sequence 5'-actttcgttt-3' (SEQ ID NO: 25) is the pan-HPV Probe used for detection of HPV 51 (SEQ ID NO: 25 is the reverse complement of SEQ ID NO: 34). The target HPV 51 El region amplified for detection is nucleotides 1103-1256.

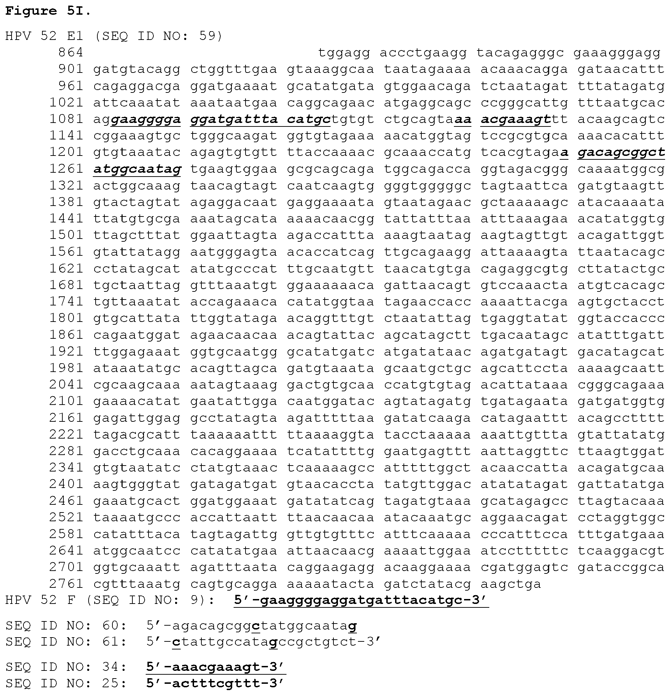

Figure 5I shows the HPV 52 El gene nucleotides 864 to 2807 marked to show the positions of the Forward primer HPV 52 5'-gaaggggaggatgatttacatgc-3' (SEQ ID NO: 9) and the target region for the Reverse primer HPV16/31/33/33n/35/39/52/56/58/66R 5'- agacagcggctatggcaatag-3' (SEQ ID NO: 60), there are two nucleotide differences g/c and c/g as shown between the HPV52 target sequence and the target sequence for the reverse primer. The sequence 5'ctattgccatagccgctgtct-3' (SEQ ID NO: 61) is the reverse complement of SEQ ID NO: 60, there are two nucleotide differences as shown, c/g and g/c, between the reverse complement and the Reverse primer HPV16/31/33/33n/35/39/52/56/58/66R (SEQ ID NO: 14) used for amplification of HPV52. The sequence 5'-aaacgaaagt-3' (SEQ ID NO: 34) is the target consensus sequence for the pan-HPV Probe used for detection of HPV 52. The sequence 5'-actttcgttt-3' (SEQ ID NO: 25) is the pan-HPV Probe used for detection of HPV 52 (SEQ ID NO: 25 is the reverse complement of SEQ ID NO: 34). The target region amplified for detection of the HPV 52 El gene is nucleotides 1083-1269.

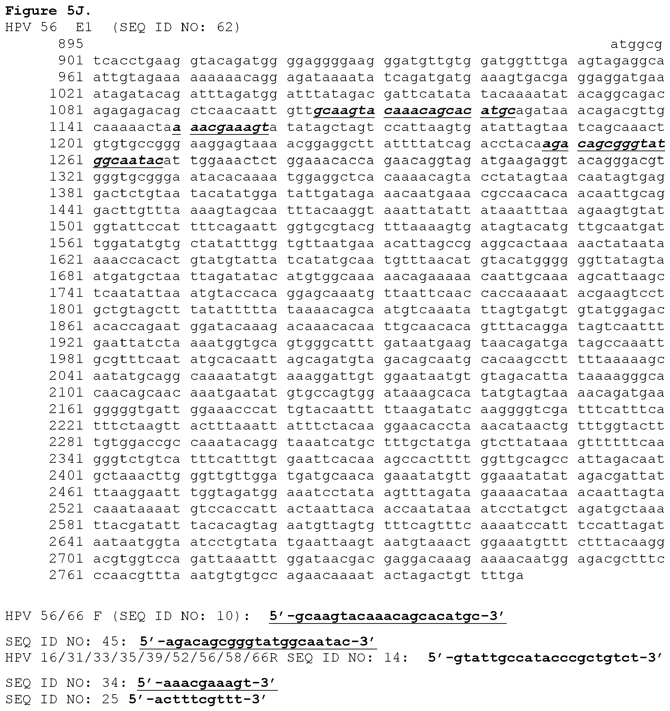

Figure 5J shows the HPV 56 El gene nucleotides 895 to 2805 (SEQ ID NO: 62), marked to show the positions of the Forward primer HPV 56/66 5'-gcaagtacaaacagcacatgc-3' (SEQ ID NO: 10) and the target for the Reverse primer HPV 16/31/33/33n/35/39/52/56/58/66R 5'- agacagcgggtatggcaatac-3' (SEQ ID NO: 45). The sequence 5'-gtattgccatacccgctgtct-3' (SEQ ID NO: 14) is the sequence of the reverse primer for HPV 16/31/33/33n/35/39/52/56/58/66R (reverse complement of SEQ ID NO: 45). The sequence 5'-aaacgaaagt-3' (SEQ ID NO: 34) is the target consensus sequence for the pan-HPV Probe used for detection of HPV 56. The sequence 5'-actttcgttt-3' (SEQ ID NO: 25) is the pan-HPV Probe used for detection of HPV 52 (the reverse complement of SEQ ID NO: 34). The target amplified for detection of HPV 56/66 is nucleotides 1104 to 1267.

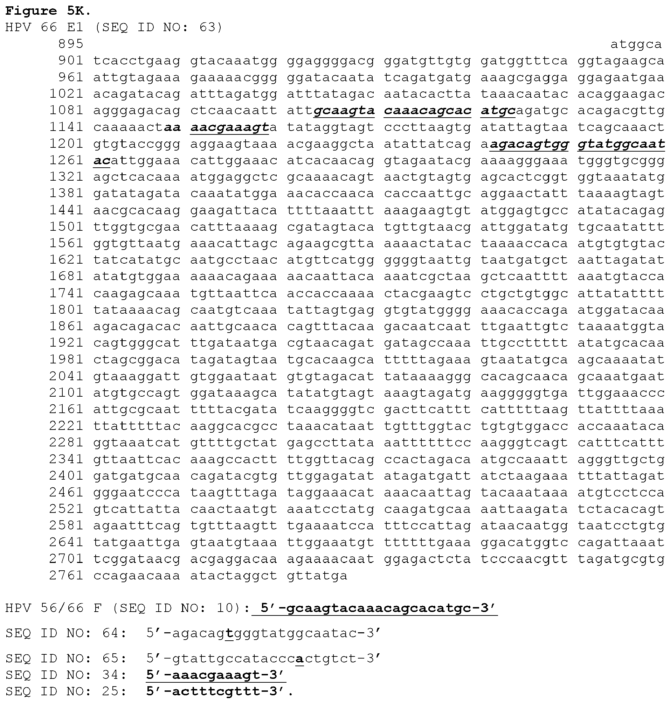

Figure 5K shows the HPV66 El gene nucleotides 895 to 2787 nucleotides (SEQ ID NO: 63), marked to indicate the position of the Forward primer HPV 56/66 5'-gcaagtacaaacagcacatgc- 3' (SEQ ID NO: 10) and the target for the Reverse primer HPV16/31/33/33n/35/39/52/56/58/66R 5'-agacag/gggtatggcaatac-3' (SEQ ID NO: 64), note the nucleotide difference c/t between the target sequence for the reverse primer and the target sequence of HPV 66. The sequence 5'-gtattgccataccc«ctgtct-3' (SEQ ID NO: 65) shows the nucleotide difference g/a between the reverse complement of SEQ ID NO: 64 and the reverse primer HPV16/31/33/33n/35/39/52/56/58/66R (SEQ ID NO: 14) that is used for amplification of HPV 66. The sequence 5'-aaacgaaagt-3' (SEQ ID NO: 34) is the target consensus sequence for the pan-HPV Probe used for detection of HPV 66. The sequence 5'- actttcgttt-3' (SEQ ID NO: 25) is the pan-HPV Probe used for detection of HPV 56 (the reverse complement of SEQ ID NO: 34). The target region of the HPV 56/66 El gene is nucleotides 1104-1262.

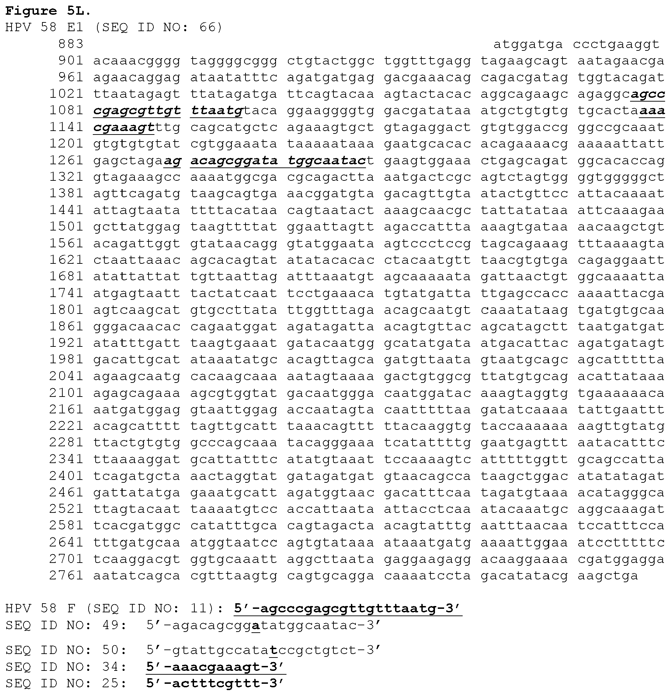

Figure 5L shows the HPV 58 El gene nucleotides 883 to 2817 (SEQ ID NO: 66), marked to show the positions of the Forward primer 58 F 5'-agcccgagcgttgtttaatg-3' (SEQ ID NO: 11), and the target region for the Reverse primer HPV16/31/33/33n/35/39/52/56/58/66R. The sequence 5'-agacagcggatatggcaatac-3' (SEQ ID NO: 49), shows the nucleotide difference g/a relative to the target sequence for the reverse primer for HPV16/31/33/33n/35/39/52/56/58/66R. The sequence 5'-gtattgccataiccgctgtct-3' (SEQ ID NO: 50) shows the nucleotide difference c/t between the reverse complement of SEQ ID NO: 49 and the reverse primer for HPV16/31/33/33n/35/39/52/56/58/66R that is used to amplify HPV 58. The sequence 5'-aaacgaaagt-3' (SEQ ID NO: 34) is the target consensus sequence for the pan-HPV Probe used for detection of HPV 58. The sequence 5'-actttcgttt-3' (SEQ ID NO: 25) is the pan-HPV Probe used for detection of HPV 58 (the reverse complement of SEQ ID NO: 34). The target amplified for detection of HPV 58 is nucleotides 1077 to 1289.

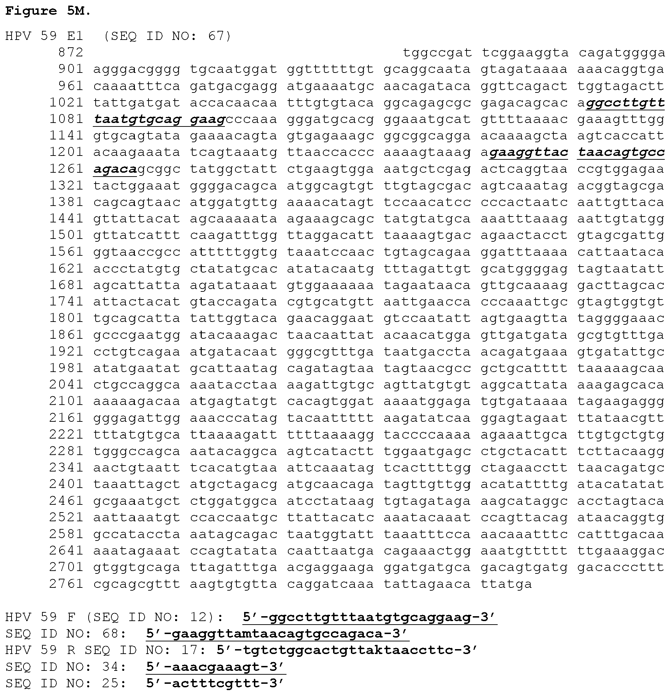

Figure 5M shows the HPV 59 El gene nucleotides 872 to 2806 (SEQ ID NO: 67) marked to show the position of the Forward primer HPV 59 F 5'-ggccttgtttaatgtgcaggaag-3' (SEQ ID NO: 12) and the region targeted by the Reverse primer HPV 59 R 5'- gaaggttamtaacagtgccagaca-3' (SEQ ID NO: 68), in which m denotes a nucleotide change c/a. The sequence 5'-tgtctggcactgttaktaaccttc-3' (SEQ ID NO: 17) is the reverse primer used for amplification of HPV 59 (reverse complement of SEQ ID NO: 68), k denotes a change of nucleotide g/t. The sequence 5'-aaacgaaagt-3' (SEQ ID NO: 34) is the target consensus sequence for the pan-HPV Probe used for detection of HPV 59. The sequence 5'-actttcgttt-3' (SEQ ID NO: 25) is the pan-HPV Probe used for detection of HPV 59 (the reverse complement of SEQ ID NO: 34). The target region amplified for detection of the HPV 59 El gene is nucleotides 1172-1265.

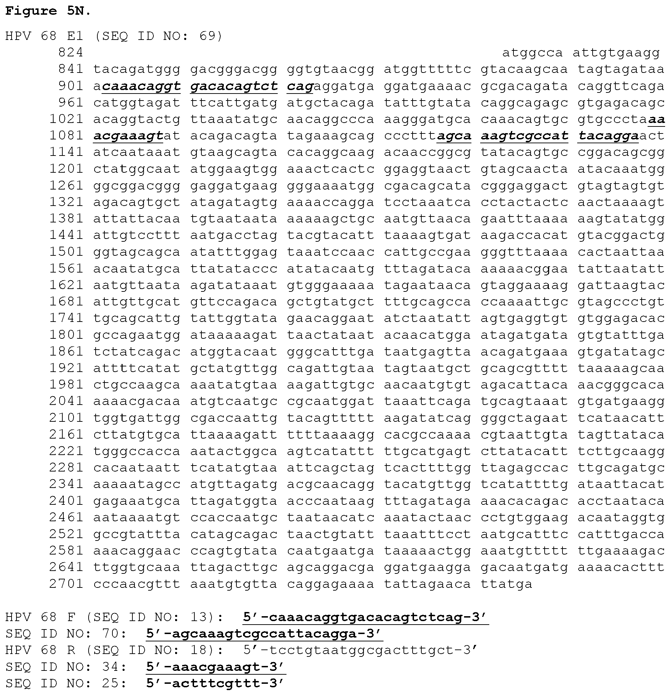

Figure 5N shows HPV 68 El nucleotides 824 to 2746 (SEQ ID NO: 69) marked to show the position of the Forward primer HPV 68 F 5'-caaacaggtgacacagtctcag-3' (SEQ ID NO: 13) and the target sequence for the Reverse primer HPV 68 R 5'-agcaaagtcgccattacagga-3' (SEQ ID NO: 70). The sequence 5'-tcctgtaatggcgactttgct-3' (SEQ ID NO: 18) is the reverse primer for HPV68 (the reverse complement of SEQ ID NO; 70). The sequence 5'-aaacgaaagt-3' (SEQ ID NO: 34) is the target consensus sequence for the pan-HPV Probe used for detection of HPV 68. The sequence 5'-actttcgttt-3' (SEQ ID NO: 25) is the pan-HPV Probe used for detection of HPV 68 (the reverse complement of SEQ ID NO: 34). The target region amplified for detection of HPV 68 El is nucleotides 902-1137.

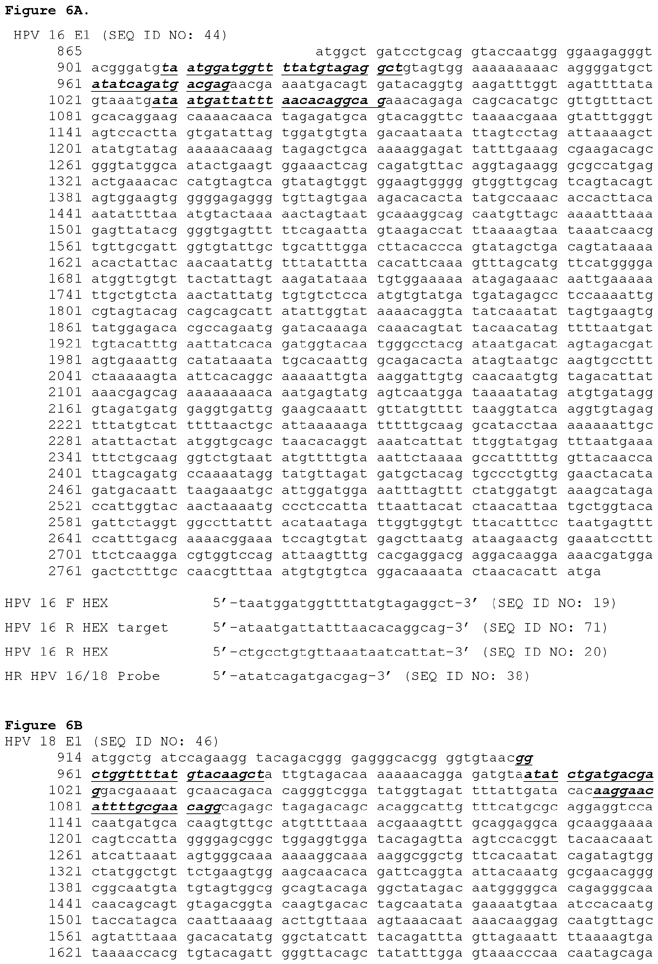

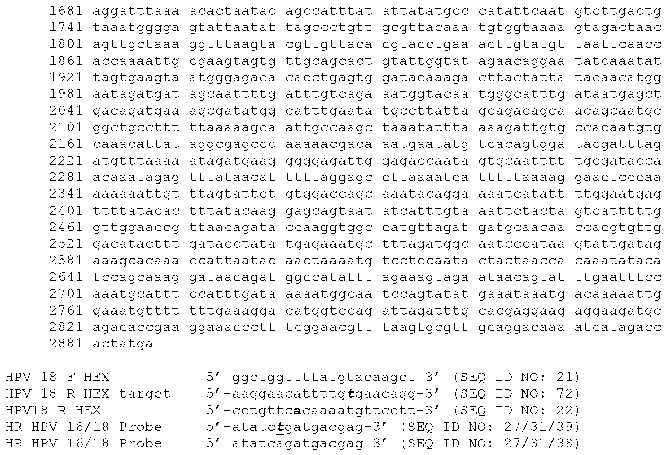

Figures 6A and 6B show the sequences of the high risk HPV genome El regions for HPV strains 16 and 18 with the sequences of probes and primers according to various embodiments of the invention, and corresponding regions of the HPV genome to which they hybridise for detection in the second detection channel 560nm HEX.
Figure 6A shows the HPV 16 El gene sequence nucleotides 865 to 2814 nucleotides (SEQ ID NO: 44) marked to show the position of the Forward primer HPV 16 F HEX 5'taatggatggttttatgtagaggct-3' (SEQ ID NO: 19) for amplification of HPV16 for the second detection channel (560nm) and the target sequence for the Reverse primer HPV 16 R HEX
   5'-ataatgattatttaacacaggcag-3' (SEQ ID NO: 71). The sequence 5'-ctgcctgtgttaaataatcattat-3' (SEQ ID NO: 20) is the HPV 16 R HEX primer sequence (SEQ ID NO: 20 is the reverse complement of SEQ ID NO: 71). The sequence 5'-atatcagatgacgag-3' (SEQ ID NO: 27/31/38) is sequence of the HR HPV 16/18 Probe. The target for amplification and detection of HPV 16 in the second detection channel (560 nm HEX) is nucleotides 909-1051.
Figure 6B shows the HPV 18 El gene nucleotides 914 to 2887 (SEQ ID NO: 46) marked to show the positions of the Forward primer HPV 18 F HEX 5'-ggctggttttatgtacaagct-3' (SEQ ID NO: 21) and the region targeted by the Reverse primer HPV 18 R HEX
   5'-aaggaacattttgigaacagg-3' (SEQ ID NO: 72), note there is a nucleotide difference c/t relative to the corresponding HPV 18 El genomic sequence shown in SEQ ID NO: 46.

The sequence 5'-cctgttcacaaaatgttcctt-3' reverse complement (SEQ ID NO: 22) is the HPV18R HEX primer sequence use for amplification of HPV 18 in the second detection channel (560nm HEX). The sequence 5'-atatcigatgacgag-3' (SEQ ID NO: 27/31/39) is the sequence of the HPV 18 showing the nucleotide difference A/T relative to the HR HPV 16/18 Probe 5'-atatcagatgacgag-3' (SEQ ID NO: 27/31/38). The target region for HPV 18 in the second detection channel (560 nm HEX) is nucleotides 959-1097.

### Detailed Description

As described above, and presented in more detail in the accompanying examples and drawings, one aspect of the invention is a composition comprising a nucleic acid probe for HPV disclosed herein. The probe may be labelled with any label suitable for use in real time PCR. Typical labels are a fluorophore and quencher, located at opposite ends of the linear probe, for example the fluorophore may be attached at the 5' end of the probe and the quencher at the 3' end. The probe may have a nucleotide sequence comprising or consisting of a probe sequence set out herein, or its reverse complement sequence (which corresponds to the target sequence of the El region of the HPV genome to which the probe binds). For example, the nucleotide sequence may be the pan-HPV probe ACTTTCGTTT (SEQ ID NO: 25) which is also referred to herein as the HR-HPV probe. The reverse complement of this sequence is AAACGAAAGT (SEQ ID NO: 42).

Preferably the probe is an LNA probe molecule, in which the nucleotide sequence comprises LNA nucleotides. Advantageously, probes comprising LNA allow a shorter sequence length to be employed while still conferring high specificity of hybridisation. HPV probes according to the present invention are generally 8, 9, 10, 11, 12, 13, 14, 15 or 16 nucleotides in length. For example, probes may be up to 15 nucleotides in length and comprise a probe sequence set out herein or its reverse complement. As noted above, LNA nucleotides contain an additional methylene bridge within the molecule of deoxyribose. These additional methylene bridges are optionally present in at least 8, 9 or 10 of the nucleotides in the probe. Methylene bridges may be absent in some nucleotides in the probe. Examples of exact locations of methylene bridges in probe sequences are presented in Example 1 (d) below.

One of the key findings underlying the present invention is that such short LNA probes may be directed to regions of homology among oncogenic HPV types, and may hybridise with sufficient specificity that they permit a large range of oncogenic HPV strains to be specifically detected using a single probe or a small number of probes such as 1, 2 or 3 probes. Thus, a probe according to the present invention may be a pan-HPV probe, able to hybridise to a plurality of oncogenic HPV. Oncogenic HPV strains include HPV16, HPVI8, HPV31, HPV33, HPV33n, HPV35, HPV39, HPV45, HPV51, HPV52, HPV56, HPV58, HPV59, HPV66 or HPV68. Preferably, pan-HPV probes according to the present invention target a region of the HPV genome in the El gene, for example as shown below in Table 1.

**Table 1.**

| HPV strain | Target region of the HPV El gene for amplification / detection (e.g., in first detection channel 530nm FAM) |
|---|---|
| 16 | 1081 - 1274 |
| 18 | 1134 - 1331 |
| 31 | 1087- 1268 |
| 33 / 33n | 976 - 1285 |
| 35 | 1082 - 1276 |
| 39 | 1115 - 1339 |
| 45 | 1123 - 1331 |
| 51 | 1103 - 1256 |
| 52 | 1083 - 1269 |
| 56 | 1104- 1267 |
| 58 | 1077 - 1289 |
| 59 | 1172 - 1265 |
| 66 | 1104 - 1262 |
| 68 | 902- 1137 |

Preferably, HPV 16/18 probes according to the present invention target a region of the HPV genome in the El gene, for example as shown below in Table 2.

**Table 2.**

| HPV strain | Target region of the HPV El gene for amplification / detection (e.g., in second detection channel 560nm HEX) |
|---|---|
| 16 | 909-1051 |
| 18 | 959-1097 |

The probes described herein may be provided for use in detecting HPV, including as part of diagnostic methods to detect the presence of oncogenic HPV in samples or to specifically determine whether a sample tests positive or negative for oncogenic HPV. A sample may be any material to be tested for oncogenic HPV, such as a sample comprising biological material from a subject e.g., from a human patient. The biological material may be obtained from a cervical smear. Preferably, the sample is a liquid-based cytology sample from a human cervical smear. The probes are optimised for use in HPV detection by real-time PCR. Primers suitable for use in combination with the probes are also described and represent further aspects of the invention.

Accordingly, one aspect of the invention is a method of determining whether a sample tests positive for oncogenic HPV, the method comprising:
combining the sample with primers for real time PCR amplification of a target region of oncogenic HPV genomes, and a labelled pan-HPV probe capable of emitting a signal upon amplification of the target region,
providing conditions for real time PCR, and
measuring for a signal emitted from the labelled pan-HPV probe,
wherein detection of the signal indicates that the sample tests positive for oncogenic
   HPV.

Examples of pan-HPV probes are described herein, such as the labelled LNA molecule comprising sequence ACTTTCGTTT (SEQ ID NO: 25) or its reverse complement. The pan-HPV probe may be a labelled 10-nucleotide molecule of sequence consisting of ACTTTCGTTT (SEQ ID NO: 25). Examples of suitable primers are also described herein. Preferably the primers are for amplification of a target region of any of HPV16, HPV18, HPV31, HPV33, HPV35, HPV39, HPV45, HPV51, HPV52, HPV56, HPV58, HPV59, HPV66 or HPV68. The method may comprise combining the sample with a combination of primers for amplification of all of these HPV types, thus allowing detection of any such HPV if it is present in the sample.

As the pan-HPV probe may be one that is designed to specifically detect any HPV belonging to a particular set of oncogenic HPV (e.g., the set of HPV16, HPV18, HPV31, HPV33, HPV35, HPV39, HPV45, HPV51, HPV52, HPV56, HPV58, HPV59, HPV66 and HPV68), detection of a signal from the labelled pan-HPV probe in an assay with all such primers therefore indicates that the sample tests positive for at least one of these oncogenic HPV, whereas the absence of a signal in these circumstances indicates that the sample tests negative for these oncogenic HPV. The test may not provide absolute certainty, in view of the limits of detection of the assay, discussed in the examples below, but methods of the invention have been shown to provide excellent accuracy and consistency with alternative methods of testing for oncogenic HPV. Methods of the invention may, if desired, be combined with one or more further techniques, such as visual examination of cells in the sample, to provide additional confidence as to the presence or absence of oncogenic HPV.

Use of a plurality of probes of different HPV specificity can extend the range of HPV detected and/or provide information regarding the type of HPV present in the sample.
For example, the pan-HPV probe of SEQ ID NO: 25 may bind more weakly to the target region of HPV33n than the target region of other oncogenic HPV, due to differences in the HPV33n genome sequence in this region. Accordingly, the invention provides a supplementary probe for HPV 33n to hybridise more strongly to the target region in HPV33n. Exemplary 33n probes described herein comprise sequence ACTTTCGCTT (SEQ ID NO: 26) or its reverse complement. The 33n probe may be a labelled 10-nucleotide molecule of sequence consisting of ACTTTCGCTT (SEQ ID NO: 26). It may be an LNA probe. A probe for HPV33n may be combined with a pan-HPV probe in methods of the invention. Conveniently, if both probes are identically labelled (e.g., with the same fluorophore) then they will emit a common signal (e.g., fluorescence of the same wavelength). Detection of this signal then indicates the presence of any HPV bound by the pan-HPV probe or the 33n probe, for instance it may indicate the presence of any oncogenic HPV belonging to the set of HPV16, HPV18, HPV31, HPV33, HPV33n, HPV35, HPV39, HPV45, HPV51, HPV52, HPV56, HPV58, HPV59, HPV66 and HPV68.

Thus, a method of determining whether a sample tests positive for oncogenic HPV may comprise additionally combining the sample with primers for amplification of a target region of HPV33n, and a labelled 33n probe capable of emitting a signal upon amplification of the target region, wherein the 33n probe and the pan-HPV probe carry the same label,
providing conditions for-real time PCR, and
measuring for a signal emitted from the labelled 33n probe or the pan-HPV probe, wherein
detection of the signal indicates that the sample tests positive for oncogenic
   HPV.

Another aspect of the invention is a method of specifically testing a sample for HPV16 and/or HPV18. Detection of these strains of HPV is of particular importance, as it is believed that HPV 16 and HPV 18 have the highest oncogenic potential and are associated with the greatest risk of cervical cancer. A method of detecting HPV16/18 may be performed alone, but it is advantageous to combine it with the pan-HPV detection method above, to provide the additional information as to whether HPV present in the sample is of the type HPV 16 or HPV 18 or belongs to a different strain of the virus.

Accordingly the method of determining whether a sample tests positive for oncogenic HPV may comprise additionally determining whether the sample tests positive for HPV16 or HPV18, and may comprise:
combining the sample with primers for real time PCR amplification of a target region of HPV 16 and HPV 18 genomes, and a labelled 16/18 probe capable of emitting a signal upon amplification of the target region,
providing conditions for real time PCR, and measuring for a
signal from the labelled 16/18 probe,
wherein detection of a signal indicates that the sample tests positive for HPVI6 or HPV18.

Examples of probes for HPVI6 and HPVI8 are set out herein, and include an LNA molecule comprising sequence ATATCWGATGACGAG (SEQ ID NO: 27) or its reverse complement.

To facilitate resolution of the HPV 16/18 signal from the pan-HPV signal in a combined detection method, the 16/18 probe may be labelled differently (e.g., with a different fluorophore) so that it emits a different signal (e.g. fluorescence at a different wavelength) from the pan-HPV probe.

Positive and / or negative controls may be included in the method as desired. Typically, conducting a positive control for the real time PCR will comprise combining the sample with primers for real time PCR amplification of a control DNA sequence (e.g., human
DNA) present in the sample, and a labelled control probe capable of emitting a signal upon amplification of the control DNA sequence,
providing conditions for real time PCR, and measuring for a
signal from the control probe,
wherein detection of the signal indicates that the real time PCR is functional. Preferably, as illustrated in the Examples, methods of the invention are performed in multiplex. Thus, multiple probes (any two or more, or all, of: pan-HPV probe, 33n probe, 16/18 probe and the control probe) and their corresponding primers for the real time PCR may be included in a single reaction mixture. The use of different labels and detection of signals with different channels (e.g., fluorescence at different wavelengths) allows the signals from different probes to be distinguished. The reaction mixture will contain suitable buffers and other reagents for the real-time PCR. Examples of reagents and reaction conditions for PCR are well-known and are provided in the Examples

Embodiments of the invention are presented below.

### Example I. Embodiment of the diagnostic test

In this embodiment, the presence of all studied oncogenic HPV viruses and the presence of 16 and 18 HPV types were determined in a test sample.

Preferably, the positive control sample was prepared of a HPV 16 nucleotide sequence and a fragment of a sequence of human beta globin.

Preferably, one control positive sample and one control negative sample were applied for each assay. In this embodiment, the positive control sample indicates the correctness of the conducted reaction (lack of degradation of the positive control sample, appropriate activity of reagents, correct thermal profile of the device, no inhibition of the reaction). A negative control sample allows for verification of whether a contamination of the DNA eluate and reagents with biological material containing HPV or amplified PCR products occurred during the isolation of DNA and preparation of the amplification reaction. If a positive signal in at least one detection channel is detected for the negative control sample, it means that a contamination of the negative control sample or reagents occurred.

Furthermore, an internal control (IC), comprising a fragment of a sequence of human beta globin applied to confirm whether each clinical test sample contains a sufficient amount of DNA, needed to detect HR HPV, was used. Moreover, a signal from the internal control
indicates properly conducted DNA isolation and lack of reaction inhibitors. Preferably, the positive control sample comprises a sequence of the internal control. The negative control sample should produce negative results in three detection channels.

### 1. Sequences of used primers, probes and control samples of the invention

a)

**oligonucleotide sequence of the primer for the first FAM channel at 530 nm:**

| SEQ ID NO. : | PRIMER | ORIENTATION | PRIMER SEQUENCE |
|---|---|---|---|
| 1 | HPV 16 F | Forward | 5'-GCACAGGAAGCAAAACAACATAG-3' |
| 2 | HPV 18 F | Forward | 5'-AGGT C CACAAT GAT GCACAAG-3' |
| 3 | HPV 33/33n F | Forward | 5'-CAGAAGATGAGGATGAAACAGCAG-3' |
| 4 | HPV 31 F | Forward | 5'-GAGGAACATGCAGAGGCTGT-3' |
| 5 | HPV 35 F | Forward | 5'-ATGCACAGGAGGAGCAAACA-3' |
| 6 | HPV 39 F | Forward | 5'-GTGAGACAGCACAGGTACTTTTAC-3' |
| 7 | HPV 45 F | Forward | 5'-C CAT GC GCAGGAAGTT CAG-3' |
| 8 | HPV 51 F | Forward | 5'-ACAAAGAGGCT GT GCAT CAG-3' |
| 9 | HPV 52 F | Forward | 5'-GAAGGGGAGGATGATTTACATGC-3' |
| 10 | HPV 56/66 F | Forward | 5'-GCAAGTACAAACAGCACAT GC-3' |
| 11 | HPV 58 F | Forward | 5'-AGCCCGAGCGTTGTTTAATG-3' |
| 12 | HPV 59 F | Forward | 5'-GGCCTT GTTTAATGTGCAGGAAG-3' |
| 13 | HPV 68 F | Forward | 5'-CAAACAGGT GACACAGT CT CAG-3' |
| 14 | HPV 16/31/33/ 33n/35/39/52 /56/58/66R | Reverse | 5'-GTATTGCCATACCCGCTGTCT-3' |
| 15 | HPV 18/45 R | Reverse | 5'-AACAGC CATAGC CACTAT CT GA-3' |
| 16 | HPV 51 R | Reverse | 5'-CTGTCCGGATAACTGTCCAGT-3' |
| 17 | HPV 59 R | Reverse | 5'-TGTCTGGCACTGTTAKTAACCTTC-3' |
| 18 | HPV 68 R | Reverse | 5'-TCCTGTAATGGCGACTTTGCT-3' |

| | | | |
|---|---|---|---|
| wherein "K" signifies a change of base G/ | | | |

b)

**oligonucleotide sequence of the primer for the second HEX channel at 560 nm:**

| SEQ ID NO.: | PRIMER | ORIENTATION | PRIMER SEQUENCE |
|---|---|---|---|
| 19 | HPV 16 F HEX | Forward | 5'-TAATGGATGGTTTTATGTAGAGGCT-3' |
| 20 | HPV 16 R HEX | Reverse | 5'-CTGCCTGTGTTAAATAAT CATTAT-3' |
| 21 | HPV 18 F HEX | Forward | 5'-GGCTGGTTTTATGTACAAGCT-3' |
| 22 | HPV 18 R HEX | Reverse | 5'-CCTGTTCACAAAATGTTCCTT-3' |

c)

**oligonucleotide sequence of the primer for the third ROX channel at 610 nm - internal control:**

| SEQ ID NO : | PRIMER | ORIENTATION | PRIMER SEQUENCE |
|---|---|---|---|
| 23 | HBE1 IC F | Forward | 5'-GAGAACTTCAAGGTGAGTTCAGGT-3 ' |
| 24 | HBE1 IC R | Reverse | 5'-GAAGTCTGCTGTTCTAACATCAAG-3' |

d)

**oligonucleotide sequence of the probe:**

| Probe | Sequence of the probe |
|---|---|
| HR-HPV (pan-HPV) SEQ ID NO.: 29 | 5'-6FAM-A+C+T+T+T+C+G+T+TT-BHQ-1-3' |
| 33n SEQ ID NO.: 30 | 5'-6FAM-A+C+T+T+T+C+G+C+TT-BHQ-1-3' |
| 16/18 SEQ ID NO.: 31 | 5'-HEX-AT+AT+CW+GA+T+G+AC+G+AG-BHQ-1-3' |
| IC SEQ ID NO.: 32 | 5'-ROX-TGACATTAATTGAAGCTCATAATCTTATTG-BHQ-2-3' |

| | |
|---|---|
| wherein "+" signifies a LNA nucleotide containing an additional methylene bridge within the deoxyribose; "W" signifies a change of base A/T. | |

### 2.Applied concentrations of primers and probes in the reaction mixture of the invention

a)

**Final concentrations of the primers in the reaction mixture for the first FAM channel at 530 nm equalled:**

| No. | PRIMER | Final concentration in the mixture [pM] | Initial concentration [pM] |
|---|---|---|---|
| 1 | HPV 16 F | 0.22 | 4 |
| 2 | HPV 18 F | 0.22 | 4 |
| 3 | HPV 31 F | 0.22 | 4 |
| 4 | HPV 33/33n F | 0.22 | 4 |
| 5 | HPV 35 F | 0.22 | 4 |
| 6 | HPV 39 F | 0.22 | 4 |
| 7 | HPV 45 F | 0.22 | 4 |
| 8 | HPV 51 F | 0.22 | 4 |
| 9 | HPV 52 F | 0.22 | 4 |
| 10 | HPV 56/66 F | 0.22 | 4 |
| 11 | HPV 58 F | 0.22 | 4 |
| 12 | HPV 59 F | 0.22 | 4 |
| 13 | HPV 68 F | 0.22 | 4 |
| 14 | HPV 16/31/33/33n/35/3 9/52/56/58/66R | 0.22 | 4 |
| 15 | HPV 18/45 R | 0.22 | 4 |
| 16 | HPV 51 R | 0.22 | 4 |
| 17 | HPV 59 R | 0.22 | 4 |
| 18 | HPV 68 R | 0.22 | 4 |

b)

**Final concentrations of the primers in the reaction mixture for the second HEX channel of HPV 16/18 at 560 nm equalled:**

| No. | PRIMER | Final concentration in the mixture [pM] | Initial concentration [pM] |
|---|---|---|---|
| 19 | HPV 16 F HEX | 0.06 | 8 |
| 20 | HPV 16 R HEX | 0.06 | 8 |
| 21 | HPV 18 F HEX | 0.06 | 8 |
| 22 | HPV 18 R HEX | 0.06 | 8 |

c)

**Final concentrations of the primers in the reaction mixture for the third ROX at 610 nm - internal control - equalled:**

| No. | PRIMER | Final concentration in the mixture [pM] | Initial concentration [pM] |
|---|---|---|---|
| 23 | HBE1 IC F | 0.2 | 8 |
| 24 | HBE1 IC R | 0.2 | 8 |

d)

**Final concentrations of the probes in the reaction mixture equalled:**

| No. | PROBE | Final concentration in the mixture [}iM] | Initial concentration [\|iM] |
|---|---|---|---|
| 25 | HR-HPV | 0.36 | 8 |
| 26 | 33n | 0.36 | 8 |
| 27 | 16/18 | 0.06 | 8 |
| 28 | IC | 0.2 | 3.6 |

Appropriate amounts of primers and probes at the above concentrations were prepared.
Before the mixture was prepared, all ingredients were vortexed and centrifuged. Subsequently, the mixture was divided into appropriate portions, which were placed into test tubes.

### 3. Description of the Master MIX MabPROBE analysis

Real-Time Master Mix MabProbe is a 2.083x concentrated mixture for Real-Time PCR. It contains a Taq polymerase blocked with a monoclonal antibody and other ingredients required for Real-Time PCR. The ingredients of the mixture were combined in appropriate portions into the final mixture, whose use reduces the risk of contamination and human error. The kit comprises:
- Master MIX MabPROBE
- A mixture of probes and primers
- A positive control sample
- A negative control sample
- Water

Composition of the Master MIX MabPROBE mixture:
- Run MAb polymerase 0.104 U/[jl
- Reaction buffer: Tris-HCl 20.83 mM pH 8.0,
   KC1 104 mM MgCl₂ - 10.42 mM
- dNTPs - 2.75 mM (0.69 mM of each dNTP)
- PCR stabilizers

All components of the test should be stored at -20°C in a dark place. Re-thawing and refreezing of components does not cause any deterioration of their activity. They must be transported on dry ice.

### 4. Test protocol:

**a.** All ingredients should be placed on ice. Primers, probes and the Master Mix MabPROBE mixture, positive control sample and negative control sample should be vortexed and briefly centrifuged.
**b.** In the following table, the quantities of the ingredients for one reaction are shown. In order to perform more analyses, it is necessary to appropriately recalculate the composition of the mixture, taking into consideration test samples, positive control and negative control. The addition of a UNG enzyme in a dilution of 1:200 is optional, and it may be replaced with water. Appropriate amounts of individual components should be mixed together in a 1.5 ml test tube of the Eppendorf type, and then the resulting mixture should be vortexed and briefly centrifuged.

**Composition of mixture for a single reaction in 25 jil**

| Ingredients | | | Quantity for one reaction in 25 j_il |
|---|---|---|---|
| MASTER MIX MAB | | | 12 |
| PRIMERS+PROBES | | | 6 |
| H₂0 or optionally 1IHNTG (1:200) | | | 1 |
| DNA | | | 6 |

| | Time | Temperature °C | Number of cycles |
|---|---|---|---|
| UNG hydrolysis | 20 minutes | 37°C | 1 |
| Dénaturation | 15 minutes | 95°C | 1 |
| Amplification | 20 seconds | 95°C | 40-50* |
| | 75 seconds | 60°C | the number of cycles should be optimised |
| Cooling | 30 seconds | 37°C | 1 |

The test was verified on the following apparatuses:

LightCycler 2.0, LightCycler Roche 96, LightCycler Roche 480, Biorad CFXTouch. The devices, which require colour compensation, are provided by the manufacturer of the test with a file in the correct format or a kit of dyes for self-performed compensation.

### 5. Interpretation of the results

According to the invention, the presence of the virus in a specific sample is evidenced by the amplification of a product comprising its specific pair of primers.
1 The UNG enzyme is not provided with the kit. It can be purchased through the Fermentas company at a concentration of 1 (J.1/U.

The recommended quantity of DNA matrix is 10 pg-1 jag. The maximal volume of the added DNA eluate is 6 jal. The quantity and volume of the added DNA eluate depends on the DNA isolation and is subjected to optimisation. In case the added quantities are below 6 jal, the missing quantity should be replaced with water.
**c.** Place 19 [j,l of the mixture into test tubes dedicated to the apparatus used for conducting the reaction.
**d.** Next, successively add to each tube 6 jal of the test DNA, positive control and negative control, repipetting several times. The mixture prepared in this way should be briefly centrifuged.
**e.** Place the samples into a thermal cycler.
   Thermal cycler programme:

In the present embodiment, the reaction product, which indicates the presence of a HPV virus, was identified by an analysis of a logarithmic growth curve formed as a result of the observed change of fluorescence for the test sample. Positive samples were characterised by a logarithmic increase of the fluorescence level with respect to the relatively constant level of fluorescence in negative samples. Preferably, the analysis was conducted using appropriate controls: negative, positive and internal ones. The resulting image is shown in Fig. 1-3.

In Fig. 1, the results of a real-time PCR for all oncogenic test HPV types, preferably using a positive control sample, a negative and a positive control sample and a negative clinical sample, were presented. According to the embodiment of the invention, the presence or lack of oncogenic HPV in the investigated samples is read out in a detection channel of 530 nm. A logarithmic increase of the PCR product in the positive clinical sample indicates the presence of at least one from the group of all investigated oncogenic HPV types. No logarithmic growth of the PCR product in the negative clinical sample demonstrates a lack of presence of oncogenic HPVs. Preferably, a positive and a negative control sample were
applied in the detection channel at 530 nm. In order to facilitate the interpretation of the results, a comparison of the control sample with clinical test samples is preferable.

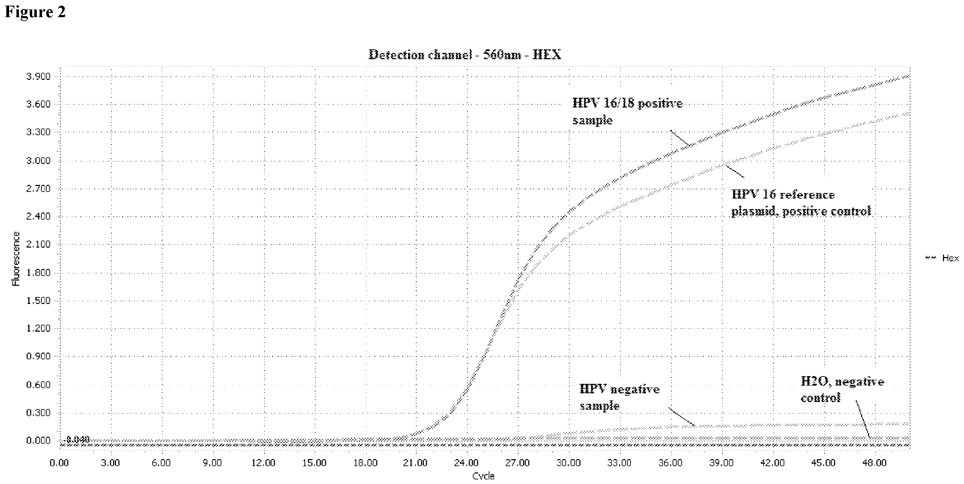

In Fig. 2, the results of a real-time PCR for 16 and 18 HPV types, preferably using a positive control sample, a negative and a positive control sample and a negative clinical sample, were presented. According to the embodiment of the invention, the presence or lack of 16 or 18 HPV types in the investigated samples is read out in a detection channel of 560 nm. A logarithmic increase of the PCR product in the positive clinical sample indicates the presence of at least one from the investigated oncogenic 16 and 18 HPV types. No logarithmic growth of the PCR product in the negative clinical sample demonstrates a lack of presence of oncogenic 16 and 18 HPVs. Preferably, a positive and a negative control sample were applied in the detection channel at 560 nm. In order to facilitate the interpretation of the results, a comparison of the control sample with clinical test samples is preferable.

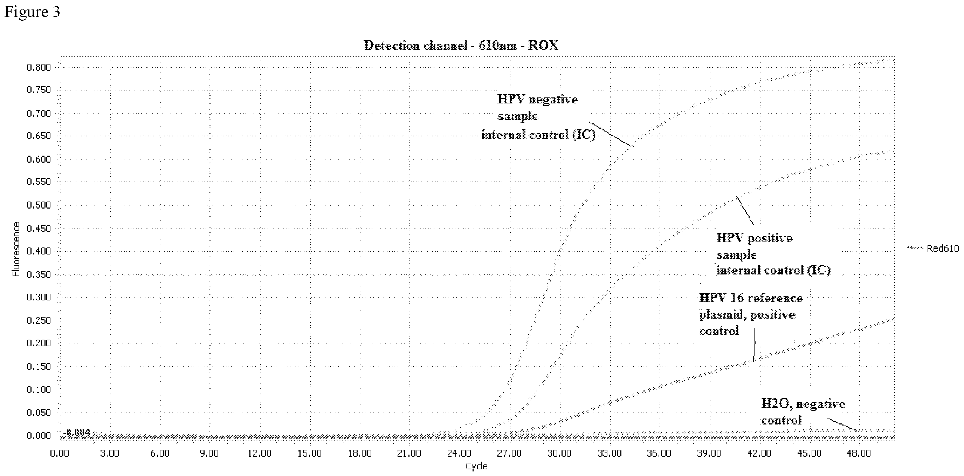

In Fig. 3, the results of a real-time PCR for the internal control (IC), preferably using a positive control sample, a negative and a positive control sample and a negative clinical sample, were presented. According to the embodiment of the invention, the presence or lack of the internal control (IC) in the investigated samples is read out in a detection channel of 610 nm. A logarithmic growth of the PCR product in the positive control sample and in the positive and negative clinical samples indicates the presence of a fragment of human beta globin and provides evidence for the correct course of the reaction and the appropriate quantity of DNA needed to detect all the investigated oncogenic HPV types. The absence of logarithmic growth of the PCR product in the negative clinical sample is expected, which suggests no presence of contamination with human DNA or the amplified PCR product. In order to facilitate the interpretation of the results, a comparison of the control sample with clinical test samples is preferable.

The present embodiment is not an example limiting the scope of the invention. The reaction product may be identified by any methods known to individuals skilled in the art.

Preferably, it can be sequenced to identify any specific sequence of the virus present in the investigated sample.
An exemplary PCR product obtained from the pair of primers HPV 16 F and HPV 16/31/33/33n/35/39/52/56/58/66R has been presented below (SEQ ID NO: 43):
HPV 16 F
HPV16/31/33/33n/35/39/52/56/58/66R

### Example II. Evaluation of the effectiveness of different kits of primers and molecular probes for the detection of oncogenic HPV types

The first step involved a modification of the composition of primers and molecular probes described above, so as to separate an additional detection channel for 16/18 HPVs. According to epidemiological data, HPV 16 and HPV 18 are characterised by the highest oncogenic potential and risk of development of cervical cancer in infected women. Therefore, it is recommended for HPV tests used for clinical purposes and, in particular, for population- based screening tests to allow not only for identification of an infection with oncogenic HPVs, but also for determination of the presence of HPV 16/18 in the investigated sample. Therefore, an additional kit of primers and one molecular probe allowing for discrimination of HPV 16/18 in a single reaction with a system for a group of all oncogenic HPV types and an internal control was designed. As a result of the conducted studies, 4 primers for HPV 16 and HPV 18 and one short probe TaqMan-LNA labelled with HEX fluorescent dye, allowing for the detection of amplicons in the channel of 560 nm, were obtained. The El region of the HPV genome, situated at a distance of approximately 700 bp from the consensus region for all oncogenic HPV types, was selected as a target sequence for primers and a common LNA probe. In this case, the internal control system was favourably modified for the real-time PCR. The synthetic nucleotide was eliminated in favour of a reference human gene for beta globin. 2 primers and a TaqMan probe labelled with ROX dye (detection channel at 610 nm) were designed for single-stage amplification of this gene together with HPV particles, potentially present in the sample. As a result, the target reaction mixture contained 26 primers and 4 molecular probes. As a consequence of performing the test on previously genotyped cytological samples, neither products of the primer-dimer type nor other non-specific PCR amplification products were observed.

The aim of the analysis was to confirm whether the selected configuration of primers and LNA-TaqMan probes is characterised by the highest efficiency in the detection of oncogenic HPV types in clinical cytological material. Comparative analyses of the new test comprising a potentially optimal system of primers and probes were conducted by evaluation of parameters such as sensitivity and specificity of the detection of oncogenic HPV types, the detection threshold for individual HPV types, including all oncogenic types, and the correlation of the obtained results with the clinically validated HPV PapilloCheck test (Greiner Bio-One), which is approved for clinical use with the CE-IVD certificate.

The analysis comprises 1256 pairs of the results of the new test for the presence of oncogenic HPV types (hrHPV) and validated PapilloCheck test. The material was collected from women, residents of the Zachodniopomorskie province and Wielkopolska province, who presented for routine preventive cytological exams, including 720 (57.3%) patients with normal cytological results and 536 (42.7%) selected women whose cytological exam revealed the presence of abnormal epithelial cells, belonging to diagnostic categories ASC-US (n=249; 19.8%), ASC-H (n=20; 1.6%), L-SIL (n=232; 18.5%) and H-SIL (n=34; 2.7%) and L-SIL/H- SIL (n=1; *0.1%)*. The average age of the investigated women equalled 33.4 ± 9.6 years of age (range: 16-66 years of age). DNA for the real-time PCR was isolated from cytology samples (cervical smears) collected into a SurePath liquid medium (Becton Dickinson), using a Cervex Brush Combi brush (Rovers). DNA isolation was performed automatically using magnetic beads in a NucliSense EasyMAG apparatus (Biomerieux). DNA amplification was performed in a Light Cycler 96 apparatus (Roche). Thin-layer cytological slides, prepared using a PrepStain robot (Becton Dickinson), were evaluated by an experienced team of cytomorphologists and pathologists using computer-assisted screening (Focal Point GS system, Becton Dickinson). The results of cytological and molecular genetic exams carried out using hrHPV and PapilloCheck tests were subjected to a statistical analysis. The detection threshold for selected HPVs (a minimal number of copies of viral particles/genomes) in the designed hrHPV test was determined. For this purpose, HPV16 and HPV18 reference materials with a known number of copies of the mentioned viruses (1st WHO International Standard for Human Papillomavirus, National Institute for Biological Standards and Control, UK) were purchased.

11 serial dilutions of HPVI6 and HPVI8 standards were performed to determine standard curves in three series of repetitions in order to define the following detection thresholds using real-time PCR:
HPV16 standard: HR-HPV (FAM) channel - control dilution 1:128 - 2 x 10⁴ copies of the virus 16/18 HPV channel (HEX) - control dilution 1:20 48 - 3.33 x 10³ copies of the virus
HPV18 standard: HR-HPV channel (FAM) - control dilution 1:128 - 5 x 10⁴ copies of the virus 16/18 HPV channel (HEX) - control dilution 1:2048 - 1.92 x 10³ copies of the virus

It is worth noting that the detection threshold for 16/18 HPVs in the second detection channel is lower in comparison to the group of all oncogenic HPVs in the first detection channel, which seems to be a favourable phenomenon in terms of the clinical efficacy of the test (targeting HPV types with a higher oncogenic potential). It should also be emphasized that taking into account the above-described correlation coefficients of the new test and the reference PapilloCheck test, it can be concluded that the detection threshold of oncogenic HPV types is similar for both tests.

Possible cross-reactions of the components of the hrHPV test with other microorganisms were also assessed. For this purpose, based on a review of the available literature (including: P. Gajer et al:. Temporal dynamics of the Human vaginal microbiota, Science Translational Medicine 2012, 4, 132ra52; S.S. Witkin, W.J. Ledger: Complexities of the uniquely human vagina. Science Translational Medicine 2012, 4, 132fsll), 42 bacterial strains, typical physiological inhabitants of the reproductive tract in women, and pathogens causing the most common infections of the genitourinary system were selected. Reference strains of the selected bacteria were purchased in the ATCC: The Global Bioresource Center, and they comprised: *Escherichia coli* ATCC 11775, *Escherichia coli* ATCC 8739, *Proteus vulgaris* ATCC 6380, *Proteus mirabilis* ATCC 7002, *Staphylococcus epidermidis* ATCC 49461, *Streptococcus agalactiae* (B) ATCC 12386, *Listeria monocytogenes* (4e) ATCC 19118, *Listeria monocytogenes* (4e) ATCC 19118, *Listeria innocua* (6a) ATCC 33090, *Lactobacillus gasseri* ATCC 19992, *Lactococcus lactis* ATCC 19435, *Lactobacillus acidophilus* ATCC 4356, *Candida albicans* ATCC 10231, *Corynebacterium urealyticum* ATCC 43044, *Oligella urethralis* ATCC 17960, *Aerococcus viridans* ATCC 700406, *Neisseria gonorrhoeae* ATCC 49226, *Gardnerella vaginalis* ATCC 14018, *Veillonella parvula* ATCC 10790, *Fusobacterium nucleatum* subsp. nucleatum ATCC 25586, *Gemella morbillorum* ATCC 27824, *Actinomyces odontolyticus* ATCC 17929, *Peptostreptococcus anaerobius* ATCC 27337, *Parvimonas micra* ATCC 33270, *Ureaplasma parvum* ATCC 27813, *Peptoniphilus asaccharolyticus* ATCC 14963, *Finegoldia magna* ATCC 29328, *Porphyromonas levii* ATCC 29147, *Prevotella loescheii* ATCC 15930.

In the course of the performed experiments, no cross-reaction between the designed system of primers and probes and the above-mentioned microorganisms was obtained in the hrHPV test.

To conclude: the study resulted in the development of an optimal kit of primers and reaction mixture favourable for a single-stage amplification of oncogenic HPV types, HPV 16/18 and an internal control in three detection channels.
Comparative studies of the correlation of the new test with the results of the reference test (PapilloCheck) in the group of 1256 women were performed for the purpose of further statistical analysis.
A clinically desired detection threshold for HPV 16/18 and other oncogenic HPV types similar to the reference test was obtained.
No cross-reactions of the new test with other microorganisms present in the reproductive tract of women were observed, which proves the high specificity of the test.

### Example III. Results analysis and statistics for the effectiveness of the new HR-HPV test for detecting the presence of oncogenic HPV viruses

In order to determine the diagnostic value of the new test for HR-HPV, its sensitivity and specificity in relation to the validated PapilloCheck test (Greiner Bio-One) were measured. It was assumed that the latter test is characterised by an optimal diagnostic accuracy for HR-HPV infections, including HPV 16/18, which is well described in the literature. Therefore, it was used as a reference for the new test. In addition, correlation coefficients for both tests were determined. This is a standard procedure to apply in the case of validation of qualitative tests, which allows for determining whether the currently used gold standard could be replaced with a new tool. The analysis was carried out on the basis of the results of cytological and molecular genetic exams, obtained by the NZOZ Meditest.

The level of correlation between the results of the new tests and the results of the reference test was evaluated based on the value of Kendall's tau b correlation coefficient. The sensitivity and specificity of the new test compared to the reference test, understood as the
percentage of the results of true positives and true negatives, respectively, were assessed on the basis of ROC analysis. All statistical calculations were performed using the Statistica 10 software package (StatSoft, Tulsa, OK, United States).

### Detection of HR-HPV in the entire investigated group

A total of the results of 1256 pairs of tests was analysed. The new test produced 630 (50.2%) positive results and 626 (49.8%) negative results. The reference test produced 647 (51.5%>) positive results and 609 (48.5%>) negative results.

| Test/result | Reference test positive result | Reference test negative result | Total |
|---|---|---|---|
| New test - positive result | 612 | 18 | 630 |
| New test - negative result | 35 | 591 | 626 |
| Total | 647 | 609 | 1256 |

In the case of 18 (2.9%) positive results of the new test, the reference test produced a negative result. In the case of 35 (5.6%>) negative results of the new test, the reference test produced a positive result. Kendall's tau b correlation coefficient equalled for both tests
b=0.9159327.

ROC analysis showed that the new test is characterised by 94.6%> sensitivity and 97.0%) specificity compared to the reference test.

### Detection of HPV 16/18 in the entire investigated group

A total of the results of 1256 pairs of tests was analysed. The new test produced 230 (18.3%>) positive results and 1026 (81.7%>) negative results. The reference test produced 238 (18.9%>) positive results and 1018 (81.1%) negative results.

| Test/result | Reference test positive result | Reference test negative result | Total |
|---|---|---|---|
| New test - positive result | 223 | 7 | 230 |
| New test - negative result | 15 | 1011 | 1026 |
| Total | 238 | 1018 | 1256 |

In the case of 7 (3.0%) positive results of the new test, the reference test produced a negative result. In the case of 15 (1.5%) negative results of the new test, the reference test produced a positive result. Kendall's tau b correlation coefficient for both tests equalled
b=0.9424401.

ROC analysis showed that the new test is characterised by 93.1% sensitivity and 99.3%) specificity compared to the reference test.

### Detection of HR-HPV in the group with normal cytology

A total of the results of 720 pairs of tests was analysed. The new test produced 154 (21.4%>) positive results and 566 (78.6%>) negative results. The reference test produced 165 (22.9%>) positive results and 555 (77.1%>) negative results.

| Test/result | Reference test - positive result | Reference test - negative result | Total |
|---|---|---|---|
| New test - positive result | 145 | 9 | 154 |
| New test - negative result | 20 | 546 | 566 |
| Total | 165 | 555 | 720 |

In the case of 9 (5.8%>) positive results of the new test, the reference test produced a negative result. In the case of 20 (3.5%>) negative results of the new test, the reference test produced a positive result. Kendall's tau b correlation coefficient for both tests equalled
b=0.8841279.

ROC analysis showed that the new test is characterised by 87.9% sensitivity and 98.4% specificity compared to the reference test.

### Detection of HPV 16/18 in the group with normal cytology

A total of the results of 720 pairs of tests was analysed. The new test produced 52 (7.2%>) positive results and 668 (92.8%>) negative results. The reference test produced 53 (7.4%o) positive results and 667 (92.6%o) negative results.

| Test/result | Reference test positive result | Reference test negative result | Total |
|---|---|---|---|
| New test - positive result | 49 | 3 | 52 |
| New test - negative result | 4 | 664 | 668 |
| Total | 53 | 667 | 720 |

In the case of 3 (5.8%>) positive results of the new test, the reference test produced a negative result. In the case of 4 (0.6%>) negative results of the new test, the reference test produced a positive result. Kendall's tau b correlation coefficient for both tests equalled
b=0.9281394.

ROC analysis showed that the new test is characterised by 92.5% sensitivity and 99.6%o specificity compared to the reference test.

### Detection of HR-HPV in the group with abnormal cytology (minimum ASC-US)

A total of the results of 536 pairs of tests was analysed. The new test produced 476 (88.8%>) positive results and 60 (11.2%) negative results. The reference test produced 482 (89.9%>) positive results and 54 (10.1%>) negative results.

| Test/result | Reference test positive result | Reference test negative result | Total |
|---|---|---|---|
| New test - positive result | 467 | 9 | 476 |
| New test - negative result | 15 | 45 | 60 |
| Total | 482 | 54 | 536 |

In the case of 9 (1.9%) positive results of the new test, the reference test produced a negative result. In the case of 15 (25.0%) negative results of the new test, the reference test produced a positive result. Kendall's tau b correlation coefficient for both tests equalled
b=0.7658275.

ROC analysis showed that the new test is characterised by 96.9% sensitivity and 83.3%) specificity compared to the reference test.

### Detection of HPV 16/18 in the group with abnormal cytology (minimum ASC-US)

A total of the results of 536 pairs of tests was analysed. The new test produced 178 (33.2%>) positive results and 358 (66.8%>) negative results. The reference test produced 185 (34.5%>) positive results and 351 (65.5%>) negative results.

| Test/result | Reference test - positive result | Reference test - negative result | Total |
|---|---|---|---|
| New test - positive result | 174 | 4 | 178 |
| New test - negative result | 11 | 347 | 358 |
| Total | 185 | 351 | 536 |

In the case of 4 (2.2%) positive results of the new test, the reference test produced a negative result. In the case of 11 (3.1%>) negative results of the new test, the reference test produced a positive result. Kendall's tau b correlation coefficient for both tests equalled b=0.9379317.

ROC analysis showed that the new test is characterised by 94.1% sensitivity and 98.9%) specificity compared to the reference test.

### Conclusions

Analysis of Kendall's tau b coefficients revealed that the results of the new test are characterised by a very high correlation with the results of the reference PapilloCheck test within the scope of HR-HPV detection, ranging from 76.6% to 91.5%. An even higher level of correlation, close to the ideal threshold of 95% (from 92.8%> to 94.2%>), was obtained for the detection of HPV 16/18.
The high diagnostic accuracy of the new test is confirmed by its high sensitivity (from 87.9% to 96.9% for hrHPV and from 92.5% to 94.1% for HPV 16/18) and specificity (from 83.3% to 98.4%) for HR-HPV and from 98.9% to 99.3% for HPV 16/18). The very high specificity of the new test for the detection of HPV 16/18 deserves particular attention; the obtained values prove that a negative result of the test excludes the presence of HPV 16 or HPV 18 infection with a probability of almost 100%. The fact that a high level of correlation, sensitivity and specificity of the new test was observed in both subgroups of patients with normal and abnormal results of cytological exam confirms the versatility of this tool.
To conclude: As a result of the performed investigations, a very high correlation of the results of the hrHPV test, according to the invention, with the results of the well-validated clinically commercial reference test (PapilloCheck) was obtained, and the high sensitivity and specificity of the hrHPV test, according to the invention, in relation to the reference test (PapilloCheck) was confirmed.

### Determination of the clinical effectiveness of the new HR-HPV test for detecting precancerous lesions and cervical cancer

A clinical population-based study, which involved 5000 women, using the new hrHPV test in cytology samples from the cervix collected on a SurePath liquid medium for further statistical analyses of correlation with a microscope image and determination of the clinical effectiveness of the new test was conducted.

The analysis was aimed at determining the clinical usefulness of the new hrHPV test in preventive exams and diagnosis of precancerous lesions and cervical cancer. To determine the diagnostic effectiveness of the new test, a large group of women who presented for routine cytological exams, being a part of cervical cancer prophylaxis, was investigated. The correlation of the obtained molecular genetic results with the microscopic image of cytological slides in a liquid medium, prepared using the standardised SurePath method of the Becton Dickinson company and assessed by qualified cytomorphologists and pathologists according to the Bethesda classification system of 2001, was determined. In order to obtain higher objectification of the microscopic evaluation, the slides were further evaluated using computer-assessed automatic screening (FocalPoint system of the Becton Dickinson company).

The analysis included 5000 pairs of the new test results for the presence of HR-HPV and the results of cytological exams on a liquid medium. The material was collected from consecutive women from the Zachodniopomorskie province and Wielkopolska province who presented for routine cytological exams. Patients whose tests for the presence of HR-HPV were ordered by the attending physician and women with a history of gynaecological and oncological abnormalities were excluded from the analysis. The average age of the investigated women equalled 33.6 ± 10.1 years of age (range: 16-67 years of age). The results of the cytological exam comprised 4825 (96.5%) normal preparations (no abnormal epithelial cells), 77 (1.55%) preparations with lesions corresponding to ASC-US, 6 (0.1%) preparations with lesions corresponding to ASC-H, 77 (1.55%) preparations with lesions corresponding to L-SIL and 15 (0.3%) preparations with lesions corresponding to H-SIL.

Similarly to the described above, DNA for the real-time PCR was isolated from cytology samples (cervical smears) collected into a SurePath liquid medium (Becton Dickinson), using a Cervex Brush Combi brush (Rovers). DNA isolation was performed automatically using magnetic beads in a NucliSense EasyMAG apparatus (Biomerieux). PCR was prepared using a PIRO automated pipetting station (Domier LTF). DNA amplification was performed in a Light Cycler 96 apparatus (Roche). As mentioned above, thin-layer cytological slides, prepared using a PrepStain robot (Becton Dickinson), were evaluated by an experienced team of cytomorphologists and pathologists using computer-assisted screening (Focal Point GS system, Becton Dickinson). The results of cytological and molecular genetic exams were subjected to a statistical analysis.

### Results analysis and statistics of the clinical effectiveness of the new HR-HPV test for detecting precancerous lesions and cervical cancer

In order to determine the clinical usefulness of the new test for HR-HPV, the values of the positive (PPV) and negative (NPV) predictive value in relation to the cytological exam in a liquid medium (SurePath, Becton Dickinson) were used. Thus, the probability of obtaining an abnormal result of a cytological exam in patients having a positive result of the new test and a normal result of a cytological exam with a negative result of the test for the presence of HR- HPV was determined. In case a high negative predictive value of the new test is confirmed, its negative result would permit resignation from a cytological exam, and its positive result could be a criterion for its execution.

The level of correlation between the results of the new hrHPV test and the results of the cytological exam was evaluated based on the value of Kendall's tau b correlation coefficient. The positive predictive value (PPV, the probability of an abnormal result of a cytological exam in an individual with a positive result of the test for the presence of HR-HPV) and negative predictive value (NPV, the probability of a normal result of a cytological exam in an individual with a negative result of the test for the presence of HR-HPV) were evaluated based on ROC analysis. All statistical calculations were performed using the Statistica 10 software package (StatSoft, Tulsa, OK, United States).

### HR-HPV detection versus an abnormal result of the cytological exam (ASCUS minimum)

A total of the results of 5000 pairs of hrHPV tests and cytological exams was analysed. The new test produced 403 (8.1%) positive results and 4597 (91.9%) negative results. The results of the cytological exam comprised 175 (3.5%) abnormal slides and 4825 (96.5%) normal slides.

| Test/result | Abnormal cytology (min. ASCUS) | Normal cytology | Total |
|---|---|---|---|
| New test - positive result | 168 | 235 | 403 |
| New test - negative result | 7 | 4590 | 4597 |
| Total | 175 | 4825 | 5000 |

In the case of 235 (58.3%) patients with a positive result of the new test, the cytological exam produced a normal result. In the case of 7 (0.2%) patients with a negative result of the new test, the cytological exam produced an abnormal result. Kendall's tau b correlation coefficient for the test for HR-HPV presence and the result of a cytological exam equalled b=0.6152294.
ROC analysis showed that the new test is characterised by a positive predictive value (PPV) of 41.7% and a negative predictive value (NPV) of 99.8%, compared to the results of a cytological exam.

### HR-HPV detection versus an abnormal result of the cytological exam (H-SIL)

A total of the results of 5000 pairs of hrHPV tests and cytological exams was analysed. The new test produced 403 (8.1%) positive results and 4597 (91.9%) negative results. The results of the cytological exam comprised 15 (0.3%) slides with lesions corresponding to H-SIL and 4985 (99.7%>) normal slides or slides with intensity lower than H-SIL.

| Test/result | Abnormal cytology (H-SIL) | Normal cytology | Total |
|---|---|---|---|
| New test - positive result | 15 | 388 | 403 |
| New test negative result | 0 | 4597 | 4597 |
| Total | 15 | 4985 | 5000 |

In the case of 388 (96.3%) patients with a positive result of the new test, the cytological exam revealed no lesions corresponding to H-SIL. In patients with a negative result of the new test, the cytological exam revealed no lesions corresponding to H-SIL. Kendall's tau b correlation coefficient for the test for HR-HPV presence and the result of a cytological exam equalled b=0.1852669.

ROC analysis showed that the new test is characterised by a positive predictive value (PPV) of 3.7%) and a negative predictive value (NPV) of 100.0%, compared to the results of a cytological exam.

### HR-HPV 16/18 detection versus an abnormal result of the cytological exam (ASCUS minimum)

A total of the results of 5000 pairs of tests and cytological exams was analysed. The new test produced 169 (3.4%) positive results and 4831 (96.6%) negative results. The results of the cytological exam comprised 175 (3.5%) abnormal slides and 4825 (96.5%) normal slides.

| Test/result | Abnormal cytology (min. ASCUS) | Normal cytology | Total |
|---|---|---|---|
| New test-positive result | 71 | 98 | 169 |
| New test-negative result | 104 | 4727 | 4831 |
| Total | 175 | 4825 | 5000 |

In the case of 98 (58.0%) patients with a positive result of the new test, the cytological exam produced a normal result. In the case of 104 (2.2%) patients with a negative result of the new test, the cytological exam produced an abnormal result. Kendall's tau b correlation coefficient for the test for HPV 16/18 presence and the result of a cytological exam equalled b=0.3919416.

ROC analysis showed that the new test is characterised by a positive predictive value (PPV) of 42.0%) and a negative predictive value (NPV) of 97.8%, compared to the results of a cytological exam.

### HPV 16/18 detection versus an abnormal result of the cytological exam (H-SIL)

A total of the results of 5000 pairs of tests and cytological exams was analysed. The new test produced 169 (3.4%) positive results and 4831 (96.6%) negative results. The results of the cytological exam comprised 15 (0.3%) slides with lesions corresponding to H-SIL and 4985 *(99.1%)* normal slides or slides with intensity lower than H-SIL.

| Test/result | Abnormal cytology (H-SIL) | Normal cytology or H-SIL(-) | Total |
|---|---|---|---|
| New test - positive result | 10 | 159 | 169 |
| New test - negative result | 5 | 4826 | 4831 |
| Total | 15 | 4985 | 5000 |

In the case of 159 (94.1%) patients with a positive result of the new test, the cytological exam revealed no lesions corresponding to H-SIL. In 5 (0.1%) patients with a negative result of the new test, the cytological exam revealed lesions corresponding to H-SIL. Kendall's tau b correlation coefficient for the test for HR-HPV presence and the result of a cytological exam equalled b=0.1921027.

ROC analysis showed that the new test is characterised by a positive predictive value (PPV) of 5.9%) and a negative predictive value (NPV) of *99.9%*, compared to the results of a cytological exam.

### Conclusions

The analysis of the clinical value of the new test revealed that it is characterised by an optimal negative predictive value (from 97.8% to 100%), compared to the result of the cytological exam on a liquid medium. Particularly high NPV values (100%) for the negative result for HR-HPV and *99.9%* for the negative result for HPV 16/18) were obtained with respect to precancerous lesions (H-SIL). The lower level of the positive predictive value is fully understandable given that not every cytological abnormality must be accompanied by an active HPV infection, especially in the group of cytological diagnoses of ASC-US and ASC- H types. It is expected that even more favourable coefficients of the predictive value for the hrHPV test can be achieved compared to traditional cytology, which is characterised by proven lesser effectiveness in detecting abnormal cervical epithelial cells in comparison to cytological methods on a liquid medium (liquid based cytology, LBC).

The obtained results indicate that in the case of a negative result of the new test, there is no need of a cytological exam. Therefore, the use of the new test could potentially allow for resignation from a cytological examination in a large population of HPV-negative women, which would enable significant savings.

However, due to the lower positive predictive value of the test, each case of its positive result should be an indication for cytology. In this context, another advantage of the new test should be emphasised: the fact that it is performed on the same material which is used for a cytological exam on a liquid medium. Accordingly, in the case of a positive result of the hrHPV test, the cytological exam can be performed using the same material, with no need for collection of additional smears from the patient. This significantly shortens the time of the diagnostic procedure and significantly reduces its cost.

To conclude: based on the results of the performed investigations, it was found that the hrHPV test, according to the invention, is characterised by an optimal negative predictive value (from 97.8% to 100%) in comparison with the results of a cytological exam on a liquid medium (SurePath), and the use of the new hrHPV test with high clinical effectiveness can potentially allow for resignation from a cytological exam in a large population of HR-HPV- negative women.

The invention allows for an innovative diagnostic tool, which is a test combining features of cytological and HPV exams. The implementation of the test according to the invention into preventive programmes should significantly increase the effectiveness of the screening for cervical cancer and rationalise the costs of further diagnosis and treatment. Furthermore, the use of a simple HPV test, preferably based on the real-time PCR technique in the material from the cytology on a liquid medium (LBC), will potentially allow for a greater automation and capacity of the diagnostic process in laboratories participating in preventive programmes. The application of the test will enable determination of the risk of cervical cancer and selection of adequate treatment. In addition, the innovative use of LNA technology, which had not been previously used in HPV diagnostics, allows for common access to the diagnostics at the highest clinical level.

## Claims

1. A method of determining whether a sample tests positive for oncogenic human papillomavirus (HPV), the method comprising:
combining the sample with primers for real-time polymerase chain reaction (PCR) amplification of a target region of oncogenic HPV genomes, and a labelled pan-HPV probe capable of emitting a signal upon amplification of the target region, providing conditions for real-time PCR, and measuring for a signal emitted from the labelled pan-HPV probe, wherein detection of the signal indicates that the sample tests positive for oncogenic
HPV,
**characterised in that** the pan-HPV probe is a labelled locked nucleic acid (LNA) molecule comprising sequence ACTTTCGTTT (SEQ ID NO: 25), or the reverse complement thereof.

2. A method according to claim 1, wherein the pan-HPV probe is a labelled 10- nucleotide molecule of sequence consisting of ACTTTCGTTT (SEQ ID NO: 25), or the reverse complement thereof and/or the pan-HPV probe is labelled at its 5' end with a fluorophore and at its 3' end with a quencher, and wherein the signal emitted upon amplification of the target region is fluorescence.

3. A method according to claim 1, wherein the primers are for amplification of a target region of any of HPV16, HPV18, HPV31, HPV33, HPV35, HPV39, HPV45, HPV51, HPV52, HPV56, HPV58, HPV59, HPV66 or HPV68 or/and comprising combining the sample with a combination of primers for amplification of a target region of all of HPV16, HPV18, HPV31, HPV33, HPV35, HPV39, HPV45, HPV51, HPV52, HPV56, HPV58, HPV59, HPV66 orHPV68.

4. A method according to any of the preceding claims, wherein the primers comprise one or more of the following primer pairs:
a) for HPV16 SEQ ID NO: 1 and SEQ ID NO: 14
b) for HPVI8 SEQ ID NO: 2 and SEQ ID NO: 15
c) for HPV33/33n SEQ ID NO: 3 and SEQ ID NO: 14
d) for HPV31 SEQ ID NO: 4 and SEQ ID NO: 14
e) for HPV35 SEQ ID NO: 5 and SEQ ID NO: 14
f) for HPV39 SEQ ID NO: 6 and SEQ ID NO: 14
g) for HPV45 SEQ ID NO: 7 and SEQ ID NO: 15
h) for HPV51 SEQ ID NO: 8 and SEQ ID NO: 16
i) for HPV52 SEQ ID NO: 9 and SEQ ID NO: 14
j) for HPV56/66 SEQ ID NO: 10 and SEQ ID NO: 14
k) for HPV58 SEQ ID NO: 11 and SEQ ID NO: 14
l) for HPV59 SEQ ID NO: 12 and SEQ ID NO: 17
m) for HPV68 SEQ ID NO: 13 and SEQ ID NO: 18
or/and wherein the primers comprise all of the primer pairs (a) to (m).

5. A method according to any of the preceding claims, comprising combining the sample with primers for amplification of a target region of HPV16, HPV18, HPV31, HPV33, HPV35, HPV39, HPV45, HPV51, HPV52, HPV56, HPV58, HPV59, HPV66 and HPV68,
detecting a signal from the labelled pan-HPV probe, and
concluding that the sample tests positive for HPV16, HPV18, HPV31, HPV33, HPY35, HPY39, HPV45, HPY51, HPY52, HPY56, HPV58, HPY59, HPV66 orHPV68.

6. A method according to any of claims 1 to 7, comprising combining the sample with primers for amplification of a target region of HPV16, HPY18, HPV31, HPV33, HPV35, HPV39, HPV45, HPV51, HPV52, HPV56, HPV58, HPV59, HPV66 and HPV68,
detecting no signal from the labelled pan-HPV probe, and
concluding that the sample tests negative for HPV16, HPV18, HPV31, HPV33, HPV35, HPV39, HPV45, HPV51, HPV52, HPV56, HPV58, HPV59, HPV66 and HPV68.

7. A method according to any of the preceding claims, further comprising combining the sample
with primers for amplification of a target region of HPV33n,
and a labelled 33n probe capable of emitting a signal upon amplification of the target region, wherein the 33n probe and the pan-HPV probe carry the same label,
providing conditions for real-time PCR, and
measuring for a signal emitted from the labelled 33n probe or the pan-HPV probe,
wherein detection of the signal indicates that the sample tests positive for oncogenic
HPV,
**characterised in that** the 33n probe is a labelled locked nucleic acid (LNA) molecule comprising sequence ACTTTCGCTT (SEQ ID NO: 26), or the reverse complement thereof or/and the 33n probe is a labelled 10-nucleotide molecule of sequence consisting of ACTTTCGCTT (SEQ ID NO: 26), or the reverse complement thereof.

8. A method according to claim 10, wherein the pan-HPV probe and 33n probe are labelled at the 5' end with a fluorophore and at the 3' end with a quencher, and wherein the signal emitted upon amplification of their respective target regions is fluorescence of the same wavelength.

9. A method according to any of the preceding claims, further comprising determining whether the sample tests positive for HPVI6 or HPVI8, the method comprising
combining the sample with primers for real time PCR amplification of a target region of HPV16 and HPV18 genomes, and a labelled 16/18 probe capable of emitting a signal upon amplification of the target region,
providing conditions for real time PCR, and measuring for
a signal from the labelled 16/18 probe,
wherein detection of a signal indicates that the sample tests positive for HPVI6 or HPV18,
**characterised in that** the 16/18 probe is a labelled locked nucleic acid (LNA) molecule comprising sequence ATATCWGATGACGAG (SEQ ID NO: 27).

10. A method of determining whether a sample tests positive for oncogenic human papillomavirus (HPV) HPV16 and / or HPV18, the method comprising
combining the sample with primers for real-time PCR amplification of a target region of HPV 16 and HPV 18 genomes, and a labelled 16/18 probe capable of emitting a signal upon amplification of the target region,
providing conditions for real-time PCR, and measuring for
a signal from the labelled 16/18 probe,
wherein detection of a signal indicates that the sample tests positive for HPVI6 or HPV18,
**characterised in that** the 16/18 probe is a labelled locked nucleic acid (LNA) molecule comprising sequence ATATCWGATGACGAG (SEQ ID NO: 27), or the reverse complement thereof.

11. A method according to claim 13 or 14, wherein the 16/18 probe is a labelled 15- nucleotide molecule of sequence consisting of ATATCWGATGACGAG (SEQ ID NO: 27), or the reverse complement thereof.

12. A method according to claim 13 or claim 15, wherein the 16/18 probe carries a different label from the pan-HPV probe.

13. A method according to any of claims 13 to 16, wherein the 16/18 probe is labelled at its 5' end with a fluorophore and at its 3' end with a quencher, and wherein the signal emitted upon amplification of the target region is fluorescence.

14. A method according to claim 16 or 17, wherein the 16/18 probe emits a fluorescent signal at a wavelength different from that of the pan-HPV probe.

15. A method according to any of claims 13 to 18, wherein the primers comprise one or both of the following primer pairs:
a) for HPV16 SEQ ID NO: 19 and SEQ ID NO: 20
b) for HPVI8 SEQ ID NO: 21 and SEQ ID NO: 22

16. A method according to any of the preceding claims, further comprising conducting a positive control for the real time PCR, comprising
combining the sample with primers for real time PCR amplification of a control DNA sequence preferably human DNA, present in the sample, and a labelled control probe capable of emitting a signal upon amplification of the control DNA sequence preferably human DNA,
providing conditions for real time PCR, and
measuring for a signal from the control probe,
wherein detection of the signal indicates that the real time PCR is functional.

17. A method according to any of the preceding claims, wherein the sample is a liquid based cytology sample from a human cervical smear.

18. A composition comprising a labelled nucleic acid probe comprising nucleotide sequence ACTTTCGTTT (SEQ ID NO: 25) or the reverse complement thereof and a detectable label.

19. A composition according to claim 23, wherein the probe comprises LNA nucleotides.

20. A composition according to claim 23 or claim 24, wherein the probe is a 10- nucleotide molecule of sequence consisting of ACTTTCGTTT (SEQ ID NO: 25) or the reverse complement thereof labelled with a fluorophore and a quencher.

21. A composition according to any of claims 23 to 25, wherein the probe is labelled at its 5' end with a fluorophore and at its 3' end with a quencher.

22. Use of a composition according to any of claims 23 to 26 in performing real time PCR for detection of HPV.

## Patentansprüche

1. Ein Verfahren zur Bestimmung, ob eine Probe positiv auf onkogenes humanes Papillomavirus (HPV) getestet wird, wobei das Verfahren umfasst:
Kombinieren der Probe mit Primern für die Echtzeit-Polymerase-Kettenreaktion (PCR) -Amplifikation einer Zielregion von onkogenen HPV-Genomen und eine markierte Pan-HPV-Sonde, die bei Amplifikation der Zielregion ein Signal emittieren kann, Bereitstellung von Bedingungen für Echtzeit-PCR,
und Messen für ein Signal, das von der markierten Pan-HPV-Sonde emittiert wird, wobei die Detektion des Signals anzeigt, dass die Probe positiv auf onkogenes HPV getestet wird,
**dadurch gekennzeichnet, dass** die pan-HPV-Sonde ein markiertes verriegeltes Nukleinsäure (LNA) -Molekül, das die Sequenz ACTTTCGTTT (SEQ ID NO: 25) oder das umgekehrte Komplement davon umfasst.

2. Verfahren nach Anspruch 1, wobei die Pan-HPV-Sonde ein markiertes 10-Nukleotidmolekül einer Sequenz bestehend aus ACTTTCGTTT (SEQ ID NO: 25) ist, oder das umgekehrte Komplement davon und / oder die pan-HPV-Sonde ist an ihrem 5'-Ende mit einem Fluorophor und an ihrem 3'-Ende mit einem Quencher markiert, und wobei das bei der Amplifikation der Zielregion emittierte Signal Fluoreszenz ist.

3. Verfahren nach Anspruch 1, wobei die Primer für die Amplifikation einer Zielregion von irgendeinem von HPV16, HPV18, HPV31, HPV33, HPV35, HPV39, HPV45, HPV51, HPV52, HPV56, HPV58, HPV59, HPV66 oder HPV68 sind, oder / und das Kombinieren der Probe mit einer Kombination von Primern zur Amplifikation einer Zielregion von allen von HPV16, HPV18, HPV31, HPV33, HPV35, HPV39, HPV45, HPV51, HPV52, HPV56, HPV58, HPV59, HPV66 oder HPV68 umfassen.

4. Verfahren nach einem der vorhergehenden Ansprüche, wobei die Primer eines oder mehrere der folgenden Primerpaare umfassen:
a) für HPV16 SEQ ID NO: 1 und SEQ ID NO: 14
b) für HPVI8 SEQ ID NO: 2 und SEQ ID NO: 15
c) für HPV33 / 33n SEQ ID NO: 3 und SEQ ID NO: 14
d) für HPV31 SEQ ID NO: 4 und SEQ ID NO: 14
e) für HPV35 SEQ ID NO: 5 und SEQ ID NO: 14
f) für HPV39 SEQ ID NO: 6 und SEQ ID NO: 14
g) für HPV45 SEQ ID NO: 7 und SEQ ID NO: 15
h) für HPV51 SEQ ID NO: 8 und SEQ ID NO: 16
i) für HPV52 SEQ ID NO: 9 und SEQ ID NO: 14 2
j) für HPV56 / 66 SEQ ID NO: 10 und SEQ ID NO: 14
k) für HPV58 SEQ ID NO: 11 und SEQ ID NO: 14
l) für HPV59 SEQ ID NO: 12 und SEQ ID NO: 17
m) für HPV68 SEQ ID NO: 13 und SEQ ID NO: 18
oder / und wobei die Primer alle Primerpaare von (a) bis (m) umfassen.

5. Verfahren nach einem der vorhergehenden Ansprüche, das das Kombinieren der Probe mit Primern zur Amplifikation einer Zielregion von HPV16, HPV18, HPV31, HPV33, HPV35, HPV39, HPV45, HPV51, HPV52, HPV56, HPV58, HPV59, HPV66 und HPV68 umfasst, und das ein Signal von der markierten Pan-HPV-Sonde detektiert, und
das zu dem Schluss kommt, dass die Probe positiv auf
HPV16, HPV18, HPV31, HPV33, HPY35, HPY39, HPV45, HPY51, HPY52, HPY56, HPV58, HPY59, HPV66 oder HPV68 getestet wurde.

6. Verfahren nach einem der Ansprüche 1 bis 7, das das Kombinieren der Probe mit Primern zur Amplifikation einer Zielregion von HPV16, HPY18, HPV31, HPV33, HPV35, HPV39, HPV45, HPV51, HPV52, HPV56, HPV58, HPV59, HPV66 und HPV68 umfasst,
und das kein Signal von der markierten Pan-HPV-Sonde detektiert, und
das zu dem Schluss kommt, dass die Probe negativ auf
HPV16, HPV18, HPV31, HPV33, HPV35, HPV39, HPV45, HPV51, HPV52, HPV56, HPV58, HPV59, HPV66 und HPV68 getestet wurde.

7. Verfahren nach einem der vorhergehenden Ansprüche, das das Kombinieren der Probe mit Primern zur Amplifikation einer Zielregion von HPV33
und eine markierte 33n-Sonde, die in der Lage ist, bei Amplifikation der Zielregion ein Signal zu emittieren, ferner umfasst, wobei die 33n-Sonde und die Pan-HPV-Sonde die gleiche Markierung tragen, Bereitstellung von Bedingungen für Echtzeit-PCR, und
Messen für ein Signal, das von der markierten 33n-Sonde oder der Pan-HPV-Sonde emittiert wird, wobei die Detektion des Signals anzeigt, dass die Probe positiv auf onkogenes HPV getestet wird, **dadurch gekennzeichnet, dass** die 33n-Sonde eine markierte, verriegelte Nukleinsäure- Molekül(LNA)ist, das die ACTTTCGCTT Sequenz(SEQ ID NO: 26) oder das umgekehrte Komplement davon umfasst oder / und die 33n-Sonde ein markiertes 10-Nukleotid-Molekül der Sequenz ist, die aus ACTTTCGCTT (SEQ ID NO: 26) oder das umgekehrte Komplement davon besteht.

8. Verfahren nach Anspruch 10, wobei die pan-HPV-Sonde und 33n-Sonde am 5'-Ende mit einem Fluorophor und am 3'-Ende mit einem Quencher markiert sind, und wobei das bei der Amplifikation ihrer jeweiligen Zielregionen emittierte Signal eine Fluoreszenz der gleichen Wellenlänge ist.

9. Verfahren nach einem der vorhergehenden Ansprüche, das ferner umfasst, zu bestimmen, ob die Probe positiv auf HPVI6 oder HPVI8 getestet wird, wobei das der Probe mit Primern zur Echtzeit-PCR-Amplifikation einer Zielregion von HPV16- und HPV18-Genomen und eine markierte 16/18-Sonde, die bei Amplifikation der Zielregion ein Signal emittieren kann, umfasst,
Bereitstellung von Bedingungen für Echtzeit-PCR und Messen für ein Signal von der markierten 16/18-Sonde, wobei die Detektion eines Signals anzeigt, dass die Probe positiv auf HPVI6 oder HPV18 getestet wird, **dadurch gekennzeichnet, dass** die 16/18-Sonde eine markierte verriegelte Nukleinsäure-Molekül(LNA)ist, das die ATATCWGATGACGAG Sequenz (SEQ ID NO: 27) umfasst.

10. Verfahren zum Bestimmen ob eine Probe positiv auf onkogenes humanes Papillomavirus (HPV) HPV16 und / oder HPV18 getestet wird, wobei das das Kombinieren der Probe mit Primern für die Echtzeit-PCR-Amplifikation einer Zielregion von HPV 16 und HPV 18-Genomen und eine markierte 16/18 Sonde, die bei Amplifikation der Zielregion ein Signal emittieren kann, umfasst;
Bereitstellung von Bedingungen für Echtzeit-PCR und Messen für ein Signal von der markierten 16/18-Sonde, wobei die Detektion eines Signals anzeigt, dass die Probe positiv auf HPVI6 oder HPV18 getestet wird, **dadurch gekennzeichnet, dass** die 16/18-Sonde eine markierte gesperrte Nukleinsäure-Molekül(LNA)ist, das die Sequenz ATATCWGATGACGAG (SEQ ID NO: 27) oder das umgekehrte Komplement davon umfasst.

11. Verfahren nach Anspruch 13 oder 14, wobei die 16/18-Sonde ein markiertes 15-Nukleotid-Molekül ist, das aus ATATCWGATGACGAG Sequenz (SEQ ID NO: 27) oder dem umgekehrten Komplement davon besteht.

12. Verfahren nach Anspruch 13 oder Anspruch 15, wobei die 16/18-Sonde eine andere Markierung von der Pan-HPV-Sonde trägt.

13. Verfahren nach einem der Ansprüche 13 bis 16, wobei die 16/18-Sonde an ihrem 5'-Ende mit einem Fluorophor und an ihrem 3'-Ende mit einem Quencher markiert ist und wobei das Signal bei Amplifikation der Zielregion emittiert wird ist Fluoreszenz.

14. Verfahren nach Anspruch 16 oder 17, wobei die 16/18-Sonde ein Fluoreszenzsignal bei einer Wellenlänge emittiert, die sich von der Wellenlänge der Pan-HPV-Sonde unterscheidet.

15. Verfahren nach einem der Ansprüche 13 bis 18, wobei die Primer eines oder beide der folgenden Primerpaare umfassen:
a) für HPV16 SEQ ID NO: 19 und SEQ ID NO: 20
WO 2015/156693 PCT / PL2015 / 050007
b) für HPVI8 SEQ ID NO: 21 und SEQ ID NO: 2%

16. Verfahren nach einem der vorhergehenden Ansprüche, das ferner das Durchführen einer positiven Kontrolle für die Echtzeit-PCR umfasst sowie das Kombinieren der Probe mit Primern für die Echtzeit-PCR-Amplifikation einer Kontroll-DNA-Sequenz, vorzugsweise menschlicher DNA, die in der Probe vorhanden ist, und eine markierte Kontrollsonde, die bei Amplifikation der Kontroll-DNA-Sequenz (vorzugsweise menschlicher DNA)ein Signal aussenden kann, Bereitstellung von Bedingungen für die Echtzeit-PCR, und Messen für ein Signal von der Kontrollsonde, wobei die Detektion des Signals anzeigt, dass die Echtzeit-PCR funktional ist.

17. Verfahren nach einem der vorhergehenden Ansprüche, wobei die Probe eine auf Flüssigkeit basierende zytologische Probe aus einem menschlichen Zervixabstrich ist.

18. Eine Zusammensetzung, die eine markierte Nukleinsäuresonde umfasst, die die Nukleotidsequenz ACTTTCGTTT (SEQ ID NO: 25) oder das umgekehrte Komplement davon und eine nachweisbare Markierung umfasst.

19. Eine Zusammensetzung nach Anspruch 23, wobei die Sonde LNA-Nukleotide umfasst.

20. Eine Zusammensetzung nach Anspruch 23 oder Anspruch 24, wobei die Sonde ein 10-Nucleotid-Molekül einer Sequenz ist, die aus ACTTTCGTTT (SEQ ID NO: 25) oder dem umgekehrten Komplement davon(das mit einem Fluorophor und einem Quencher markiert ist) besteht.

21. Eine Zusammensetzung nach einem der Ansprüche 23 bis 25, wobei die Sonde an ihrem 5'-Ende mit einem Fluorophor und an ihrem 3'-Ende mit einem Quencher markiert ist.

22. Verwendung einer Zusammensetzung nach einem der Ansprüche 23 bis 26 zur Durchführung einer Echtzeit-PCR zum Detektion von HPV.

## Revendications

1. Procédé pour déterminer si un échantillon est positif pour le papillomavirus humain oncogène (HPV), le procédé comprenant:
combinaison de l'échantillon avec des amorces pour l'amplification en chaîne par polymérase (PCR) en temps réel d'une région cible de génomes de HPV oncogènes, et une sonde pan-HPV marquée capable d'émettre un signal lors de l'amplification de la région cible, fournissant des conditions pour la PCR en temps réel, et mesurer un signal émis par la sonde pan-HPV marquée,
dans lequel la détection du signal indique que l'échantillon est positif pour le HPV oncogène, **caractérisé en ce que** la sonde pan-HPV est une molécule d'acide nucléique verrouillé (LNA) marquée comprenant la séquence ACTTTCGTTT (SEQ ID NO: 25), ou le complément inverse de celle-ci.

2. Procédé selon la revendication 1, dans lequel la sonde pan-HPV est une molécule de 10 nucléotides marquée d'une séquence consistant en ACTTTCGTTT (SEQ ID NO: 25), ou son complément inverse et / ou la sonde pan-HPV est marquée à son extrémité 5 'avec un fluorophore et à son extrémité 3' avec un suppresseur (quencher), et dans lequel le signal émis lors de l'amplification de la région cible est la fluorescence.

3. Procédé selon la revendication 1, dans lequel les amorces sont destinées à l'amplification d'une région cible de HPV16, HPV18, HPV31, HPV33, HPV35, HPV39, HPV45, HPV51, HPV52, HPV56, HPV58, HPV59, HPV66 ou HPV68 ou / et comprenant la combinaison de l'échantillon avec une combinaison d'amorces pour l'amplification d'une région cible de tous les HPV16, HPV18, HPV31, HPV33, HPV35, HPV39, HPV45, HPV51, HPV52, HPV56, HPV58, HPV59, HPV66 ou HPV68.

4. Procédé selon l'une quelconque des revendications précédentes, dans lequel les amorces comprennent une ou plusieurs des paires d'amorces suivantes:
a) pour HPV16 SEQ ID NO: 1 et SEQ ID NO: 14
b) pour HPVI8 SEQ ID NO: 2 et SEQ ID NO: 15
c) pour HPV33 / 33n SEQ ID NO: 3 et SEQ ID NO: 14
d) pour HPV31 SEQ ID NO: 4 et SEQ ID NO: 14
e) pour HPV35 SEQ ID NO: 5 et SEQ ID NO: 14
f) pour HPV39 SEQ ID NO: 6 et SEQ ID NO: 14
g) pour HPV45 SEQ ID NO: 7 et SEQ ID NO: 15
h) pour HPV51 SEQ ID NO: 8 et SEQ ID NO: 16
i) pour HPV52 SEQ ID NO: 9 et SEQ ID NO: 14 2
j) pour HPV56 / 66 SEQ ID NO: 10 et SEQ ID NO: 14
k) pour HPV58 SEQ ID NO: 11 et SEQ ID NO: 14
l) pour HPV59 SEQ ID NO: 12 et SEQ ID NO: 17
m) pour HPV68 SEQ ID NO: 13 et SEQ ID NO: 18
ou / et dans lequel les amorces comprennent toutes les paires d'amorces de (a) à (m).

5. Procédé selon l'une quelconque des revendications précédentes, comprenant la combinaison de l'échantillon avec des amorces pour l'amplification d'une région cible de HPV16, HPV18, HPV31, HPV33, HPV35, HPV39, HPV45, HPV51, HPV52, HPV56, HPV58, HPV59, HPV66. et HPV68,
détecter un signal provenant de la sonde pan-HPV marquée, et
la conclusion que l'échantillon est positif pour
HPV16, HPV18, HPV31, HPV33, HPY35, HPY39, HPV45, HPY51, HPY52, HPY56, HPV58, HPY59, HPV66 ou HPV68.

6. Procédé selon l'une quelconque des revendications 1 à 7, comprenant la combinaison de l'échantillon avec des amorces pour l'amplification d'une région cible de HPV16, HPY18, HPV31, HPV33, HPV35, HPV39, HPV45, HPV51, HPV52, HPV56, HPV58, HPV59, HPV66 et HPV68,
détecter aucun signal de la sonde pan-HPV marquée, et
conclure que l'échantillon est négatif pour
HPV16, HPV18, HPV31, HPV33, HPV35, HPV39, HPV45, HPV51, HPV52, HPV56, HPV58, HPV59, HPV66 et HPV68.

7. Procédé selon l'une quelconque des revendications précédentes, comprenant en outre la combinaison de l'échantillon avec des amorces pour l'amplification d'une région cible de HPV33n,
et une sonde 33n marquée capable d'émettre un signal lors de l'amplification de la région cible, dans laquelle la sonde 33n et la sonde pan-HPV portent le même marqueur, fournissant des conditions pour la PCR en temps réel, et
mesurer un signal émis par la sonde 33n marquée ou la sonde pan-HPV,
dans lequel la détection du signal indique que l'échantillon est positif pour le HPV oncogène,
**caractérisé en ce que** la sonde 33n est une molécule d'acide nucléique verrouillée (LNA) marquée comprenant la séquence ACTTTCGCTT (SEQ ID NO: 26), ou le complément inverse de celle-ci et / et la sonde 33n est une molécule marquée de 10 nucléotides de séquence consistant en ACTTTCGCTT (SEQ ID NO: 26), ou le complément inverse de celle-ci.

8. Procédé selon la revendication 10, dans lequel la sonde pan-HPV et la sonde 33n sont marquées à l'extrémité 5 'avec un fluorophore et à l'extrémité 3' avec un suppresseur (quencher), et dans lequel le signal est émis lors de l'amplification de leurs régions cibles respectives est la fluorescence de la même longueur d'onde.

9. Procédé selon l'une quelconque des revendications précédentes, comprenant en outre la détermination si l'échantillon est positif pour HPVI6 ou HPVI8, le procédé comprenant
combinaison de l'échantillon avec des amorces pour l'amplification PCR en temps réel d'une région cible des génomes HPV16 et HPV18, et une sonde marquée 16/18 capable d'émettre un signal lors de l'amplification de la région cible, fournissant des conditions pour la PCR en temps réel et mesurant signal de la sonde marquée 16/18,
dans lequel la détection d'un signal indique que l'échantillon est positif pour HPVI6 ou HPV18, **caractérisé en ce que** la sonde 16/18 est une molécule d'acide nucléique verrouillé (LNA) marquée comprenant la séquence ATATCWGATGACGAG (SEQ ID NO: 27).

10. Procédé pour déterminer si un échantillon est positif pour le HPV16 et / ou le HPV18 du papillomavirus humain (HPV) oncogène, le procédé comprenant
combiner l'échantillon avec des amorces pour l'amplification PCR en temps réel d'une région cible des génomes HPV 16 et HPV 18, et une sonde marquée 16/18 capable d'émettre un signal lors de l'amplification de la région cible, fournissant des conditions pour la PCR en temps réel, et mesurer pour un signal de la sonde marquée 16/18,
dans lequel la détection d'un signal indique que l'échantillon est positif pour HPVI6 ou HPV18, **caractérisé en ce que** la sonde 16/18 est une molécule d'acide nucléique verrouillé (LNA) marquée comprenant la séquence ATATCWGATGACGAG (SEQ ID NO: 27), ou son complément inverse.

11. Procédé selon la revendication 13 ou 14, dans lequel la sonde 16/18 est une molécule de 15 nucleotides marquée d'une séquence consistant en ATATCWGATGACGAG (SEQ ID NO: 27), ou son complément inverse.

12. Procédé selon la revendication 13 ou la revendication 15, dans lequel la sonde 16/18 porte un marqueur différent de la sonde pan-HPV.

13. Procédé selon l'une quelconque des revendications 13 à 16, dans lequel la sonde 16/18 est marquée à son extrémité 5 'avec un fluorophore et à son extrémité 3' avec un suppresseur (quencher), et dans lequel le signal est émis lors de l'amplification de la région cible est la fluorescence.

14. Procédé selon la revendication 16 ou 17, dans lequel la sonde 16/18 émet un signal fluorescent à une longueur d'onde différente de celle de la sonde pan-HPV.

15. Procédé selon l'une quelconque des revendications 13 à 18, dans lequel les amorces comprennent une ou les deux paires d'amorces suivantes:
a) pour HPV16 SEQ ID NO: 19 et SEQ ID NO: 20
WO 2015/156693 PCT / PL2015 / 050007
b) pour HPVI8 SEQ ID NO: 21 et SEQ ID NO: 2%

16. Procédé selon l'une quelconque des revendications précédentes, comprenant en outre la réalisation d'un contrôle positif pour la PCR en temps réel, comprenant
la combinaison de l'échantillon avec des amorces pour l'amplification PCR en temps réel d'une séquence d'ADN témoin, de préférence de l'ADN humain, présent dans l'échantillon,
et une sonde de contrôle marquée capable d'émettre un signal lors de l'amplification de la séquence d'ADN de contrôle, de préférence de l'ADN humain, fournissant des conditions pour la PCR en temps réel, et mesurant un signal provenant de la sonde de contrôle, dans laquelle la détection du signal indique que la PCR en temps réel est fonctionnelle.

17. Procédé selon l'une quelconque des revendications précédentes, dans lequel l'échantillon est un échantillon de cytologie à base liquide provenant d'un frottis cervical humain.

18. Une composition comprenant une sonde d'acide nucléique marquée comprenant la séquence nucléotidique ACTTTCGTTT (SEQ ID NO: 25) ou son complément inverse et un marqueur détectable.

19. Composition selon la revendication 23, dans laquelle la sonde comprend des nucléotides de LNA.

20. Composition selon la revendication 23 ou la revendication 24, dans laquelle la sonde est une molécule de 10 nucleotides de séquence consistant en ACTTTCGTTT (SEQ ID NO: 25) ou son complément inverse marqué avec un fluorophore et un suppresseur (quencher).

21. Composition selon l'une quelconque des revendications 23 à 25, dans laquelle la sonde est marquée à son extrémité 5 'avec un fluorophore et à son extrémité 3' avec un suppresseur (quencher).

22. Utilisation d'une composition selon l'une quelconque des revendications 23 à 26 pour effectuer une PCR en temps réel pour la détection de HPV.


### SEQUENCE LISTING

<110> Centrum Onkologii - Instytut im. Marii Sk³odowskiej-Curie
   Podolski, Jacek
<120> Screening Test for Detecting the Presence of Oncogenic HPV Viruses
<130> PZ/2433/AG
<150> P.407864
   <151> 2014-04-11
<160> 72
<170> PatentIn version 3.5
<210> 1
   <211> 23
   <212> DNA
   <213> artificial
<220>
   <223> a primer for HPV 16 F
<400> 1
   gcacaggaag caaaacaaca tag 23
<210> 2
   <211> 21
   <212> DNA
   <213> artificial
<220>
   <223> a primer for HPV 18 F
<400> 2
   aggtccacaa tgatgcacaa g 21
<210> 3
   <211> 24
   <212> DNA
   <213> artificial
<220>
   <223> a primer for HPV 33/33n F
<400> 3
   cagaagatga ggatgaaaca gcag 24
<210> 4
   <211> 20
   <212> DNA
   <213> artificial
<220>
   <223> a primer for HPV 31 F
<400> 4
   gaggaacatg cagaggctgt 20
<210> 5
   <211> 20
   <212> DNA
   <213> artificial
<220>
   <223> a primer for HPV 35 F
<400> 5
   atgcacagga ggagcaaaca 20
<210> 6
   <211> 24
   <212> DNA
   <213> artificial
<220>
   <223> a primer for HPV 39 F
<400> 6
   gtgagacagc acaggtactt ttac 24
<210> 7
   <211> 19
   <212> DNA
   <213> artificial
<220>
   <223> a primer for HPV 45 F
<400> 7
   ccatgcgcag gaagttcag 19
<210> 8
   <211> 20
   <212> DNA
   <213> artificial
<220>
   <223> a primer for HPV 51 F
<400> 8
   acaaagaggc tgtgcatcag 20
<210> 9
   <211> 23
   <212> DNA
   <213> artificial
<220>
   <223> a primer for HPV 52 F
<400> 9
   gaaggggagg atgatttaca tgc 23
<210> 10
   <211> 21
   <212> DNA
   <213> artificial
<220>
   <223> a primer for HPV 56/66 F
<400> 10
   gcaagtacaa acagcacatg c 21
<210> 11
   <211> 20
   <212> DNA
   <213> artificial
<220>
   <223> a primer for HPV 58 F
<400> 11
   agcccgagcg ttgtttaatg 20
<210> 12
   <211> 23
   <212> DNA
   <213> artificial
<220>
   <223> a primer for HPV 59 F
<400> 12
   ggccttgttt aatgtgcagg aag 23
<210> 13
   <211> 22
   <212> DNA
   <213> artificial
<220>
   <223> a primer for HPV 68 F
<400> 13
   caaacaggtg acacagtctc ag 22
<210> 14
   <211> 21
   <212> DNA
   <213> artificial
<220>
   <223> a primer for HPV 16/31/33/33n/35/39/52/56/58/66 R
<400> 14
   gtattgccat acccgctgtc t 21
<210> 15
   <211> 22
   <212> DNA
   <213> artificial
<220>
   <223> a primer for HPV 18/45 R
<400> 15
   aacagccata gccactatct ga 22
<210> 16
   <211> 21
   <212> DNA
   <213> artificial
<220>
   <223> a primer for HPV 51 R
<400> 16
   ctgtccggat aactgtccag t 21
<210> 17
   <211> 24
   <212> DNA
   <213> artificial
<220>
   <223> a primer for HPV 59 R
<400> 17
   tgtctggcac tgttaktaac cttc 24
<210> 18
   <211> 21
   <212> DNA
   <213> artificial
<220>
   <223> a primer for HPV 68 R
<400> 18
   tcctgtaatg gcgactttgc t 21
<210> 19
   <211> 25
   <212> DNA
   <213> artificial
<220>
   <223> a primer for HPV 16 F HEX
<400> 19
   taatggatgg ttttatgtag aggct 25
<210> 20
   <211> 24
   <212> DNA
   <213> artificial
<220>
   <223> a primer for HPV 16 R HEX
<400> 20
   ctgcctgtgt taaataatca ttat 24
<210> 21
   <211> 21
   <212> DNA
   <213> artificial
<220>
   <223> a primer for HPV 18 F HEX
<400> 21
   ggctggtttt atgtacaagc t 21
<210> 22
   <211> 21
   <212> DNA
   <213> artificial
<220>
   <223> a primer for HPV 18 R HEX
<400> 22
   cctgttcaca aaatgttcct t 21
<210> 23
   <211> 24
   <212> DNA
   <213> artificial
<220>
   <223> a primer for HBE1 IC F
<400> 23
   gagaacttca aggtgagttc aggt 24
<210> 24
   <211> 24
   <212> DNA
   <213> artificial
<220>
   <223> a primer for HBE1 IC R
<400> 24
   gaagtctgct gttctaacat caag 24
<210> 25
   <211> 10
   <212> DNA
   <213> artificial
<220>
   <223> a sequence of HR-HPV
<400> 25
   actttcgttt 10
<210> 26
   <211> 10
   <212> DNA
   <213> artificial
<220>
   <223> a sequence of HPV 33n
<400> 26
   actttcgctt 10
<210> 27
   <211> 15
   <212> DNA
   <213> artificial
<220>
   <223> a sequence of HPV 16/18
<400> 27
   atatcwgatg acgag 15
<210> 28
   <211> 30
   <212> DNA
   <213> artificial
<220>
   <223> a sequence of HBE1
<400> 28
   tgacattaat tgaagctcat aatcttattg 30
<210> 29
   <211> 10
   <212> DNA
   <213> artificial
<220>
   <223> a probe for HR-HPV
<220>
   <221> misc-feature
   <222> (1)..(1)
   <223> 5'-6FAM
<220>
   <221> misc-feature
   <222> (10)..(10)
   <223> BHQ-1-3'
<400> 29
   actttcgttt 10
<210> 30
<400> 30
   000
<210> 31
   <211> 15
   <212> DNA
   <213> artificial
<220>
   <223> a probe for 16/18
<220>
   <221> misc-feature
   <222> (1)..(1)
   <223> 5'-HEX
<220>
   <221> misc-feature
   <222> (6) .. (6)
   <223> "W" signifies a change of base A/T.
<220>
   <221> misc_feature
   <222> (15)..(15)
   <223> BHQ-1-3'
<400> 31
   atatcwgatg acgag 15
<210> 32
   <211> 30
   <212> DNA
   <213> artificial
<220>
   <223> a probe for IC
<220>
   <221> misc_feature
   <222> (1)..(1)
   <223> 5'-ROX
<220>
   <221> misc_feature
   <222> (30)..(30)
   <223> BHQ-2-3'
<400> 32
   tgacattaat tgaagctcat aatcttattg 30
<210> 33
   <211> 180
   <212> DNA
   <213> artificial
<220>
   <223> region of HPV16 genome
<400> 33
   gcacaggaag caaaacaaca tagagatgca gtacaggttc taaaacgaaa gtatttgggt        60
   agtccactta gtgatattag tggatgtgta gacaataata ttagtcctag attaaaagct       120
   atatgtatag aaaaacaaag tagagctgca aaaaggagat tatttgaaag cgaagacagc       180
<210> 34
   <211> 10
   <212> DNA
   <213> artificial
<220>
   <223> consensus region of HPV genome targeted by pan-HPV probe
<400> 34
   aaacgaaagt 10
<210> 35
   <211> 240
   <212> DNA
   <213> artificial
<220>
   <223> region of HPV 33n genome
<400> 35
   cattgtttaa tatacaggaa ggggaggatg acttaaatgc tgtgtgtgca ctaaagcgaa      60
   agtttgccgc atgttcacaa agtgctgcgg aggacgttgt tgatcgtgct gcaaacccgt     120
   gtagaacgtc tattaataaa aataaagaat gcacatacag aaaacgaaaa atagatgagc     180
   tagaagacag cggatatggc aatactgaag tggaaactca gcagatggta caacaggtag     240
<210> 36
   <211> 10
   <212> DNA
   <213> artificial
<220>
   <223> region of HPV 33n genome targeted by 33n probe
<400> 36
   aagcgaaagt 10
<210> 37
   <211> 240
   <212> DNA
   <213> artificial
<220>
   <223> region of HPV 16 genome
<400> 37
   acgggatgta atggatggtt ttatgtagag gctgtagtgg aaaaaaaaac aggggatgct      60
   atatcagatg acgagaacga aaatgacagt gatacaggtg aagatttggt agattttata     120
   gtaaatgata atgattattt aacacaggca gaaacagaga cagcacatgc gttgtttact     180
   gcacaggaag caaaacaaca tagagatgca gtacaggttc taaaacgaaa gtatttgggt     240
<210> 38
   <211> 15
   <212> DNA
   <213> artificial
<220>
   <223> HPV 16 probe
<400> 38
   atatcagatg acgag 15
<210> 39
   <211> 15
   <212> DNA
   <213> artificial
<220>
   <223> HPV 18 probe
<400> 39
   atatctgatg acgag 15
<210> 40
   <211> 420
   <212> DNA
   <213> artificial
<220>
   <223> Region of HPV 18 genome
<400> 40
   gcagtaagca acaatggctg atccagaagg tacagacggg gagggcacgg ggtgtaacgg 60
   ctggttttat gtacaagcta ttgtagacaa aaaaacagga gatgtaatat ctgatgacga 120
   ggacgaaaat gcaacagaca cagggtcgga tatggtagat tttattgata cacaaggaac 180
   attttgtgaa caggcagagc tagagacagc acaggcattg tttcatgcgc aggaggtcca 240
   caatgatgca caagtgttgc atgttttaaa acgaaagttt gcaggaggca gcaaagaaaa 300
   cagtccatta ggggagcggc tggaggtgga tacagagtta agtccacggt tacaacaaat 360
   atcattaaat agtgggcaaa aaaaggcaaa aaggcggctg ttcacaatat cagatagtgg 420
<210> 41
   <211> 240
   <212> DNA
   <213> artificial
<220>
   <223> Region of HPV 16 genome
<400> 41
   gcacaggaag caaaacaaca tagagatgca gtacaggttc taaaacgaaa gtatttgggt 60
   agtccactta gtgatattag tggatgtgta gacaataata ttagtcctag attaaaagct 120
   atatgtatag aaaaacaaag tagagctgca aaaaggagat tatttgaaag cgaagacagc 180
   gggtatggca atactgaagt ggaaactcag cagatgttac aggtagaagg gcgccatgag 240
<210> 42
   <211> 10
   <212> DNA
   <213> artificial
<220>
   <223> Reverse complement pan-HPV probe sequence, which corresponds to the consensus region targeted by the pan-HPV probe
<400> 42
   aaacgaaagt 10
<210> 43
   <211> 194
   <212> DNA
   <213> artificial
<220>
   <223> Example PCR product obtained from primers HPV 16 F and common reverse primer
<400> 43
   gcacaggaag caaaacaaca tagagatgca gtacaggttc taaaacgaaa gtatttgggt        60
   agtccactta gtgatattag tggatgtgta gacaataata ttagtcctag attaaaagct       120
   atatgtatag aaaaacaaag tagagctgca aaaaggagat tatttgaaag cgaagacagc       180
   gggtatggca atac                                                          194
<210> 44
   <211> 1950
   <212> DNA
   <213> artificial
<220>
   <223> HPV 16 nucleotides 865-2814 E1 gene sequence
<400> 44
   atggctgatc ctgcaggtac caatggggaa gagggtacgg gatgtaatgg atggttttat        60
   gtagaggctg tagtggaaaa aaaaacaggg gatgctatat cagatgacga gaacgaaaat       120
   gacagtgata caggtgaaga tttggtagat tttatagtaa atgataatga ttatttaaca       180
   caggcagaaa cagagacagc acatgcgttg tttactgcac aggaagcaaa acaacataga       240
   gatgcagtac aggttctaaa acgaaagtat ttgggtagtc cacttagtga tattagtgga       300
   tgtgtagaca ataatattag tcctagatta aaagctatat gtatagaaaa acaaagtaga       360
   gctgcaaaaa ggagattatt tgaaagcgaa gacagcgggt atggcaatac tgaagtggaa       420
   actcagcaga tgttacaggt agaagggcgc catgagactg aaacaccatg tagtcagtat       480
   agtggtggaa gtgggggtgg ttgcagtcag tacagtagtg gaagtggggg agagggtgtt 540
   agtgaaagac acactatatg ccaaacacca cttacaaata ttttaaatgt actaaaaact 600
   agtaatgcaa aggcagcaat gttagcaaaa tttaaagagt tatacggggt gagtttttca 660
   gaattagtaa gaccatttaa aagtaataaa tcaacgtgtt gcgattggtg tattgctgca 720
   tttggactta cacccagtat agctgacagt ataaaaacac tattacaaca atattgttta 780
   tatttacaca ttcaaagttt agcatgttca tggggaatgg ttgtgttact attagtaaga 840
   tataaatgtg gaaaaaatag agaaacaatt gaaaaattgc tgtctaaact attatgtgtg 900
   tctccaatgt gtatgatgat agagcctcca aaattgcgta gtacagcagc agcattatat 960
   tggtataaaa caggtatatc aaatattagt gaagtgtatg gagacacgcc agaatggata 1020
   caaagacaaa cagtattaca acatagtttt aatgattgta catttgaatt atcacagatg 1080
   gtacaatggg cctacgataa tgacatagta gacgatagtg aaattgcata taaatatgca 1140
   caattggcag acactaatag taatgcaagt gcctttctaa aaagtaattc acaggcaaaa 1200
   attgtaaagg attgtgcaac aatgtgtaga cattataaac gagcagaaaa aaaacaaatg 1260
   agtatgagtc aatggataaa atatagatgt gatagggtag atgatggagg tgattggaag 1320
   caaattgtta tgtttttaag gtatcaaggt gtagagttta tgtcattttt aactgcatta 1380
   aaaagatttt tgcaaggcat acctaaaaaa aattgcatat tactatatgg tgcagctaac 1440
   acaggtaaat cattatttgg tatgagttta atgaaatttc tgcaagggtc tgtaatatgt 1500
   tttgtaaatt ctaaaagcca tttttggtta caaccattag cagatgccaa aataggtatg 1560
   ttagatgatg ctacagtgcc ctgttggaac tacatagatg acaatttaag aaatgcattg 1620
   gatggaaatt tagtttctat ggatgtaaag catagaccat tggtacaact aaaatgccct 1680
   ccattattaa ttacatctaa cattaatgct ggtacagatt ctaggtggcc ttatttacat 1740
   aatagattgg tggtgtttac atttcctaat gagtttccat ttgacgaaaa cggaaatcca 1800
   gtgtatgagc ttaatgataa gaactggaaa tcctttttct caaggacgtg gtccagatta 1860
   agtttgcacg aggacgagga caaggaaaac gatggagact ctttgccaac gtttaaatgt 1920
   gtgtcaggac aaaatactaa cacattatga 1950
<210> 45
   <211> 21
   <212> DNA
   <213> artificial
<220>
   <223> region of the HPV 16 E1 gene targeted by the reverse primer HPV 16/31/33/33n/35/39/52/56/58/66R
<400> 45
   agacagcggg tatggcaata c 21
<210> 46
   <211> 1974
   <212> DNA
   <213> artificial
<220>
   <223> HPV 18 nucleotides 914 - 2887 E1 gene sequence
<400> 46
   atggctgatc cagaaggtac agacggggag ggcacggggt gtaacggctg gttttatgta      60
   caagctattg tagacaaaaa aacaggagat gtaatatctg atgacgagga cgaaaatgca     120
   acagacacag ggtcggatat ggtagatttt attgatacac aaggaacatt ttgcgaacag     180
   gcagagctag agacagcaca ggcattgttt catgcgcagg aggtccacaa tgatgcacaa     240
   gtgttgcatg ttttaaaacg aaagtttgca ggaggcagca aggaaaacag tccattaggg     300
   gagcggctgg aggtggatac agagttaagt ccacggttac aacaaatatc attaaatagt     360
   gggcaaaaaa aggcaaaaag gcggctgttc acaatatcag atagtggcta tggctgttct     420
   gaagtggaag caacacagat tcaggtaatt acaaatggcg aacagggcgg caatgtatgt     480
   agtggcggca gtacagaggc tatagacaat gggggcacag agggcaacaa cagcagtgta     540
   gacggtacaa gtgacactag caatatagaa aatgtaaatc cacaatgtac catagcacaa     600
   ttaaaagact tgttaaaagt aaacaataaa caaggagcaa tgttagcagt atttaaagac     660
   acatatgggc tatcatttac agatttagtt agaaatttta aaagtgataa aaccacgtgt     720
   acagattggg ttacagctat atttggagta aacccaacaa tagcagaagg atttaaaaca     780
   ctaatacagc catttatatt atatgcccat attcaatgtc ttgactgtaa atggggagta     840
   ttaatattag ccctgttgcg ttacaaatgt ggtaaaagta gactaacagt tgctaaaggt     900
   ttaagtacgt tgttacacgt acctgaaact tgtatgttaa ttcaaccacc aaaattgcga     960
   agtagtgttg cagcactgta ttggtataga acaggaatat caaatattag tgaagtaatg    1020
   ggagacacac ctgagtggat acaaagactt actattatac aacatggaat agatgatagc    1080
   aattttgatt tgtcagaaat ggtacaatgg gcatttgata atgagctgac agatgaaagc    1140
   gatatggcat ttgaatatgc cttattagca gacagcaaca gcaatgcggc tgccttttta    1200
   aaaagcaatt gccaagctaa atatttaaaa gattgtgcca caatgtgcaa acattatagg    1260
   cgagcccaaa aacgacaaat gaatatgtca cagtggatac gatttagatg tttaaaaata    1320
   gatgaagggg gagattggag accaatagtg caatttttgc gataccaaca aatagagttt    1380
   ataacatttt taggagcctt aaaatcattt ttaaaaggaa ctcccaaaaa aaattgttta    1440
   gtattctgtg gaccagcaaa tacaggaaaa tcatattttg gaatgagttt tatacacttt    1500
   atacaaggag cagtaatatc atttgtaaat tctactagtc atttttggtt ggaaccgtta    1560
   acagatacca aggtggccat gttagatgat gcaacaacca cgtgttggac atactttgat     1620
   acctatatga gaaatgcttt agatggcaat cccataagta ttgatagaaa gcacaaacca     1680
   ttaatacaac taaaatgtcc tccaatacta ctaaccacaa atatacatcc agcaaaggat     1740
   aacagatggc catatttaga aagtagaata acagtatttg aatttccaaa tgcatttcca     1800
   tttgataaaa atggcaatcc agtatatgaa ataaatgaca aaaattggaa atgttttttt     1860
   gaaaggacat ggtccagatt agatttgcac gaggaagagg aagatgcaga caccgaagga     1920
   aaccctttcg gaacgtttaa gtgcgttgca ggacaaaatc atagaccact atga           1974
<210> 47
   <211> 22
   <212> DNA
   <213> artificial
<220>
   <223> Region of HPV18 E1 genome targeted by reverse primer HPV 18/45 R
<400> 47
   tcagatagtg gctatggctg tt 22
<210> 48
   <211> 2002
   <212> DNA
   <213> artificial
<220>
   <223> HPV 33 genome comprising nucleotides 879 - 2813 E1 gene sequence
<400> 48
   atggccgatc ctgaaggtac aaatggggct gggatggggt gtactggttg gtttgaggta 60
   gaagcagtca tagagagaag aacaggagat aatatttcag aagatgagga tgaaacagca 120
   gatgacagtg gcacggattt actagagttt atagatgatt ctatggaaaa tagtatacag 180
   gcagacacag aggcagcccg ggcattgttt aatatacagg aaggggagga tgacttaaat 240
   gctgtgtgtg cactaaagcg aaagtttgcc gcatgttcac aaagtgctgc ggaggacgtt 300
   gttgatcgtg ctgcaaaccc gtgtagaacg tctattaata aaaataaaga atgcacatac 360
   agaaaacgaa aaatagatga gctagaagac agcggatatg gcaatactga agtggaaact 420
   cagcagatgg tacaacaggt agaaagtcaa aatggcgaca caaacttaaa tgacttagaa 480
   tctagtgggg tgggggatga ttcagaagta agctgtgaga caaatgtaga tagctgtgaa 540
   aatgttacat tgcaggaaat tagtaatgtt ctacatagta gtaatacaaa agcaaatata 600
   ttatataaat ttaaagaggc ctatggaata agttttatgg aattagtaag accatttaaa 660
   agtgataaaa caagctgtac agattggtgt ataacaggat atggaattag tccatcagta 720
   gcagaaagtt taaaagtatt aattaaacag catagtttgt atactcattt acaatgttta 780
   acttgcgata gaggaataat aatattattg ttaattagat ttaggtgtag caaaaacagg 840
   ttaacagtag caaaactaat gagtaattta ttatcaatac ctgaaacatg tatggttata 900
   gagccaccaa agttacggag ccaaacatgt gcattgtatt ggtttagaac agcaatgtca 960
   aacattagtg atgtacaagg tacaacacct gaatggatag atagactaac tgttttacaa 1020
   catagcttta atgataatat atttgattta agtgaaatgg tacagtgggc atacgataac 1080
   gagttaacgg acgatagtga cattgcatat tattatgcac aacttgcaga ttcaaatagt 1140
   aatgctgctg catttttaaa aagtaactca caagcaaaaa tagtaaagga ctgtggaata 1200
   atgtgtagac attataaaaa agcagaaaaa cgtaaaatgt caataggaca atggatacaa 1260
   agtagatgtg aaaaaacaaa tgatggagga aattggagac caatagtaca gttgttaaga 1320
   tatcaaaaca ttgaatttac agcattttta ggtgcattta aaaagttttt aaaaggtata 1380
   ccaaaaaaaa gctgtatgct aatttgtgga ccagcaaata caggaaagtc atattttgga 1440
   atgagtttaa tacagttttt aaaagggtgt gttatatcat gtgtaaattc taaaagtcac 1500
   ttttggttgc agccattatc agatgcaaaa ataggaatga tagatgatgt aacgccaata 1560
   agttggacat atatagatga ttacatgaga aatgcgttag atggaaatga aatttcaata 1620
   gatgtgaaac atagggcatt agtgcaatta aaatgtccac cactgcttct tacctcaaat 1680
   acaaatgcag gcacagactc tagatggcca tatttacata gtagattaac agtatttgaa 1740
   tttaaaaatc cattcccatt tgatgaaaat ggtaacccag tgtatgtaat aaatgatgaa 1800
   aattggaaat cctttttctc aaggacgtgg tgcaaattag atttaataga ggaagaggac 1860
   aaggaaaacc atggaggaaa tatcagcacg tttaaatgca gtgcaggaga aaatactaga 1920
   tctttacgaa gctgataaaa ctgatttacc atcacaaatt gaacattgga aactgatacg 1980
   catggagtgt gctttatttt at 2002
<210> 49
   <211> 21
   <212> DNA
   <213> artificial
<220>
   <223> HPV 39 sequence targeted by the Reverse primer HPV16/31/33/33n/35/39/52/56/58/66R
<400> 49
   agacagcgga tatggcaata c 21
<210> 50
   <211> 21
   <212> DNA
   <213> artificial
<220>
   <223> Reverse complement of SEQ ID NO: 49
<400> 50
   gtattgccat atccgctgtc t 21
<210> 51
   <211> 1891
   <212> DNA
   <213> artificial
<220>
   <223> HPV31 genome sequence comprising nucleotides 862 - 2751 E1 gene sequence
<400> 51
   aatggctgat ccagcaggta cagatgggga ggggacggga tgcaatggtt ggttttatgt      60
   agaagcagta attgacagac agacagggga caacatttca gacgacgaaa atgaagacag     120
   tagtgatact ggggaggata tggttgactt tattgacaat tgtaatgtat acaacaatca     180
   ggcagaagca gagacagcac aggcattgtt tcatgcacag gaagcggagg aacatgcaga     240
   ggctgtgcag gttctaaaac gaaagtatgt aggtagtcct ttaagtgata ttagtagttg     300
   tgtggattat aatattagtc cacggttaaa agctatatgc atagaaaata acagtaaaac     360
   agcaaaacga agactttttg aacttccaga cagcgggtat ggcaatactg aagtggaagc     420
   gcagcagatg gtacaggtag aggagcaaca aacaacatta agttgtaatg gtagtgacgg     480
   gacacatagt gaacgagaca atgaaactcc aacacgtaat atattgcaag tgttaaaaac     540
   tagcaatggt aaagctgcta tgttaggtaa atttaaagaa ttatatggtg taagttttat     600
   ggaactaatt aggccatttc aaagcaataa aagcacatgt gctgattggt gtgtagctgc     660
   gtttggagtt acaggtacag ttgcagaagg atttaaaacc ctattgcaac catattgttt     720
   gtattgccat ttacaaagtt tagcatgttc ctggggcatg gttatgttaa tgcttgtaag     780
   atttaaatgt gcaaaaaata gaataacaat tgaaaaatta ttagaaaagt tattgtgtat     840
   atctacaaat tgtatgttaa ttcagccacc caaattacgt agtacagctg cagcattata     900
   ttggtacaga acaggaatgt caaacattag tgatgtatat ggtgaaacac cagaatggat     960
   agaaagacaa acagtattac agcatagttt taatgacaca acatttgatt tgtcccaaat    1020
   ggtacaatgg gcatatgaca atgatgttat ggatgatagt gaaattgcct atagatatgc    1080
   acaattagct gacagtgata gtaatgcatg tgcattttta aaaagtaatt cgcaggcaaa    1140
   aatagttaaa gattgtggaa caatgtgtag acattataaa cgagcagaaa aacgacaaat    1200
   gtctatggga cagtggatta aaagtagatg tgacaaagtt agtgacgaag gtgactggag    1260
   ggacatagta aagtttttaa gatatcaaca aatagaattt gtgtcatttt tatctgcatt    1320
   aaagctgttt ttaaaaggag tgccaaagaa aaactgtatt ttaatacatg gtgcacctaa    1380
   tacaggtaaa tcatattttg gaatgagcct tatgagcctc ttacaaggat gtataatatc    1440
   atatgcaaat tcaaaaagtc atttttggtt acaaccactg gctgatgcta aaataggcat    1500
   gttagatgat gctacaacgc catgttggca ttatatagac aattacctac gaaatgcact     1560
   agatggcaac cctgtatcta tagatgtaaa gcataaagct ttaatgcagt taaaatgtcc     1620
   tcctttattg attacatcta atataaatgc aggtaaggat gacagatggc catacctaca     1680
   tagcagacta gtggttttta catttccaaa tccatttcca tttgacaaaa acggaaatcc     1740
   agtatatgaa ttaagtgata aaaactggaa atcctttttc tcaaggacgt ggtgcagatt     1800
   aaatttgcac gaggaagagg acaaagaaaa cgatggagac tctttctcaa cgtttaaatg     1860
   tgtgtcagga caaaatatta gaacattatg a                                    1891
<210> 52
   <211> 1914
   <212> DNA
   <213> artificial
<220>
   <223> HPV35 nucleotides 868 - 2781 E1 gene sequence
<400> 52
   atggctgatc ctgcaggtac agatgaaggg gaggggacgg gatgtaatgg atggtttttt     60
   gtagaagcag tagttagtag acgtacgggg gatccagtgt cagaggacga aaatgaagat    120
   gactgtgaca gggggaggga tatggtggac tttataaatg atacagatat attaaacata    180
   caggcagaaa cagagacagc acaagcatta tttcatgcac aggaggagca aacacacaaa    240
   gaggctgtac aggtcctaaa acgaaagtat gctagtagtc cacttagcag cgtgagctta    300
   tgtgttaata ataacataag tccacgttta aaagctattt gcattgaaaa taaaaataca    360
   gcagcaaagc gacgattatt tgaactacca gacagcggtt atggcaattc tgaagtggaa    420
   atacagcaga tacaacaggt agaggggcat gatacagttg aacaatgtag tatgggcagt    480
   ggggatagta taacctctag tagcgatgaa agacatgaag agactccaac gcgagacata    540
   atacaaatac taaaatgtag taatgcaaac gcagctatgt tggctaaatt taaagaccta    600
   tttggtatta gttttacaga acttattaga ccatttaaga gtgataaatc cacatgtaca    660
   gattggtgtg tggccgcatt tggaatagcc ccaagtgtgg cggaaagttt aaaaacatta    720
   attaaaccat attgtttata tgtacatata caatgtttat cgtgttcatg gggtatggta    780
   attctagcat tattacgatt taaatgtgca aaaaacagaa caacaattga aaaactatta    840
   tcaaaattgc tatgtatttc agctgcaagt atgctaatac aaccaccaaa attacgtagt    900
   accccagctg cgttatattg gtttaaaaca gcaatgtcaa atattagtga ggttgatgga    960
   gaaacaccag aatggattca aagacaaaca gtattacagc atagttttaa tgatgcaata   1020
   tttgacctat ctgaaatggt acaatgggca tatgacaatg attttataga tgatagtgat   1080
   atagcatata aatatgcaca attggcagaa actaatagta atgcatgtgc ttttttaaaa    1140
   agtaattcgc aagctaaaat tgtaaaagat tgtgcaacaa tgtgtagaca ttataaacga 1200
   gctgaaaaaa gagaaatgac aatgtcacag tggattaaaa ggcgatgtga aaaggtggac 1260
   gatgacggtg actggaggga catagtacga tttttaagat atcaacaagt agattttctg 1320
   gcatttttat ctgcactaaa aaatttttta catggtgtgc ctaaaaaaaa ttgcatactt 1380
   atatatggag caccaaacac aggtaaatca ttatttggaa tgagtctaat gcatttctta 1440
   caaggagcta ttatatccta tgtaaattct aaaagccatt tttggttgca gccattatat 1500
   gatgccaaaa tagctatgtt agatgatgct acatcgccat gttgggcata tatagaccaa 1560
   tatttaagaa atgcactaga tggaaatcct atttcattag atgtaaagca taaagcatta 1620
   gtgcaattaa aatgcccacc tttacttatt acatcaaata taaatgcagg caaagatgac 1680
   aggtggccat acttacatag cagggtagtg gtctttacat ttcacaatga attcccattt 1740
   gataaaaatg gaaacccagt gtatgggctt aatgataaaa actggaaatc ctttttctca 1800
   aggacgtggt gcagattaaa tttgcacgag gaagaggaca aagaaaatga tggagacgct 1860
   ttcccagcgt ttaagtgtgt gtcaggacaa aatactagaa cattacgaga ctga 1914
<210> 53
   <211> 21
   <212> DNA
   <213> artificial
<220>
   <223> Region of HPV53 sequence targeted by the reverse primer HPV16/31/33/33n/35/39/52/56/58/66R
<400> 53
   agacagcggt tatggcaatt c 21
<210> 54
   <211> 21
   <212> DNA
   <213> artificial
<220>
   <223> Reverse complement of SEQ ID NO: 53
<400> 54
   gaattgccat aaccgctgtc a 21
<210> 55
   <211> 1943
   <212> DNA
   <213> artificial
<220>
   <223> HPV 39 nucleotides 928 - 2871 E1 gene sequence
<400> 55
   tggccaatcg tgaaggtaca gacggggatg ggtcgggatg taacggatgg tttctagtac 60
   aggcaatagt agataaacaa acaggcgaca cagtgtcgga ggatgaggat gaaaatgcaa 120
   cagatacagg ttcagacttg gcagacttta ttgatgattc cacagatatt tgtgtacagg 180
   cagagcgtga gacagcacag gtacttttac atatgcaaga ggcccaaagg gatgcacaag 240
   cagtgcgtgc cttaaaacga aagtatacag acagcagtgg cgacactaga ccgtatggaa 300
   aaaaagtagg caggaatacc aggggaacac tacaggaaat ttcattaaat gtaagcagta 360
   cgcaggcaac acaaacggtg tattccgtgc cagacagcgg atatggcaat atggaagtgg 420
   aaacagctga agtggaggag gtaactgtag caactaatac aaatggggat gctgaagggg 480
   aacatggcgg cagtgtacgg gaggagtgca gtagtgtgga tagtgctata gatagtgaaa 540
   accaggatcc caaatctcca actgcacaaa ttaaattatt gttacaatcc aataacaaaa 600
   aggctgcaat gctaacacaa tttaaagaaa catatggact atcctttact gacctggtac 660
   gtacgtttaa aagtgataaa acaacatgta cagactgggt ggcagccata tttggagtac 720
   atccaactat tgcagaagga tttaaaacat taatcaacaa atatgcctta tatacacata 780
   tacaaagctt agacacaaaa caaggagtac taattttaat gctaataaga tatacatgtg 840
   gaaaaaatag ggttactgta ggaaagggat taagtacatt gttacatgtt ccagaaagtt 900
   gtatgcttct ggagcctcct aaactgcgca gccctgtagc agcactatat tggtatcgca 960
   caggtatatc caatattagt gtggtaacag gggatacgcc agaatggata caacgattaa 1020
   ctgttataca acatggaata gatgatagtg tatttgacct atcggacatg gtacaatggg 1080
   catttgacaa tgaatatact gatgaaagtg acatagcatt taattatgca atgttagcag 1140
   attgtaacag taatgctgca gcctttttaa aaagtaactg ccaggcaaaa tatgtaaaag 1200
   attgtgcaac aatgtgtaaa cattacaagc gagcacaaaa aaggcaaatg tccatgtctc 1260
   aatggataaa atttaggtgt agtaaatgtg atgaaggcgg ggactggaga cccatagtac 1320
   aattcttaag atatcaagga atagaattta tatccttttt atgtgcatta aaggaatttt 1380
   taaagggtac tcccaaaaaa aactgtatag ttatatatgg acctgcgaat acaggaaagt 1440
   cacatttttg tatgagcctt atgcattttt tacagggcac agttatttca tatgtaaact 1500
   ccaccagcca cttttggcta gaaccacttg cagatgcaaa actagcaatg ttagatgatg 1560
   caaccggtac ctgctggtca tatttcgata attatatgag aaatgcatta gatgggtatg 1620
   caataagttt agataggaaa tataaaagtt tactacaaat gaaatgtcca ccattattaa 1680
   taacctccaa taccaatcct gtggaagacg ataggtggcc atatttacgt agtaggctaa 1740
   cagtgtttaa atttcctaat gcatttccat ttgaccaaaa caggaatcca gtgtacacaa 1800
   tcaatgataa aaactggaaa tgtttttttg aaaagacttg gtgcagatta gacttgcagc 1860
   aggacgagga tgaaggagac aatgatgaaa acactttcac aacgtttaaa tgtgttacag 1920
   gacaaaatac tagaatacta tga 1943
<210> 56
   <211> 1931
   <212> DNA
   <213> artificial
<220>
   <223> HPV 45 nucleotides 914 - 2845 E1 gene sequence
<400> 56
   tggcggatcc agaaggtacc gacggggagg gaacggggtg taatggctgg ttctttgtag      60
   aaacaattgt agagaaaaaa acaggggatg taatatcaga tgatgaggat gaaactgcaa     120
   cagatacagg gtcggatatg gtagatttta ttgacacaca attatccatt tgtgaacagg     180
   cagagcaaga gacagcacag gcattgttcc atgcgcagga agttcagaat gatgcacagg     240
   tgttgcatct tttaaaacga aagtttgcag gaggcagcaa ggaaaacagt ccattagggg     300
   agcagctaag tgtggatacg gatctaagtc cacggttaca agaaatttca ttaaatagtg     360
   ggcacaaaaa agcaaaacga cggttgttta caatatcaga tagtggctat ggctgttctg     420
   aagtggaagc tgcagagact caggtaactg taaacactaa tgcggaaaat ggcggcagtg     480
   tacatagtac acaaagtagt ggtggggata gtagtgacaa tgcagaaaat gtagatccgc     540
   attgcagtat tacagaacta aaggagctat tacaagcaag taacaaaaag gctgcaatgc     600
   tggcagtatt taaagacata tatgggctgt catttacgga tttggttaga aattttaaaa     660
   gtgataaaac aacatgtaca gattgggtaa tggctatatt tggagttaat ccaacggtag     720
   cagaaggctt taaaacatta attaaaccag caacgttata cgcccatatc caatgtttag     780
   attgtaaatg gggagtatta atattagctt tattaagata taaatgtggc aaaaatagac     840
   taactgttgc aaaaggctta agcacattgt tgcacgtacc tgaaacatgt atgttaattg     900
   aaccaccaaa attgcgaagt agtgttgcag cattatactg gtatagaaca ggtatatcca     960
   atattagtga agtaagtgga gacacacctg agtggataca aagactgaca attattcaac    1020
   atggtattga cgatagtaat tttgatttgt cagacatggt gcaatgggca tttgataatg    1080
   accttacaga tgaaagtgat atggcatttc aatatgccca attagcagac tgcaacagta    1140
   atgcagctgc atttttaaaa agtaactgcc aagccaaata tttaaaagat tgtgctgtaa    1200
   tgtgtagaca ttataaaaga gcacaaaaac gccaaatgaa tatgtctcaa tggattaaat    1260
   atagatgttc caaaatagat gaaggtgggg attggagacc catagtacaa ttcctaagat    1320
   atcagggagt agaatttatt agctttttaa gggcactaaa ggaatttctt aaaggaacac    1380
   caaaaaaaaa ttgtatactg ttatatggac ctgcaaatac aggaaaatcg tattttggaa    1440
   tgagttttat acatttccta caaggtgcaa taatatcatt tgtaaattca aacagccatt    1500
   tttggttaga accgttagca gatactaagg tagccatgtt ggatgatgcc acacacacgt    1560
   gttggacata ttttgataat tatatgagaa atgcattaga tggtaatcct ataagtatag     1620
   acagaaagca taaaccatta ttacagctaa aatgtcctcc aatcctatta acatccaata     1680
   ttgatccagc aaaagataat aaatggccat atttagaaag tagggtgacg gtatttacat     1740
   ttccacatgc atttccattt gataaaaatg gtaatccagt atatgaaata aatgataaaa     1800
   attggaaatg tttttttgaa aggacatggt ccagattaga tttgcacgag gacgatgaag     1860
   atgcagacac cgaaggaatc cctttcggaa cgtttaagtg cgttacagga caaaatacta     1920
   gaccactatg a                                                         1931
<210> 57
   <211> 1800
   <212> DNA
   <213> artificial
<220>
   <223> HPV 51 nucleotides 841 - 2640 comprising the E1 gene sequence
<400> 57
   ttgcccgtgt tgtgcgaaca actagcaacg gcgatggact gtgaaggtac agaggatgag 60
   ggggcggggt gtaatgggtg gttttttgtt gaagcaatag tagaaaaaaa aacaggagat 120
   aatgtttcgg atgatgagga tgaaaatgca gatgatacag gatctgattt aataaacttt 180
   atagatagtg aaactagtat ttgcagtcag gcggaacagg agacagcacg ggcgttgttt 240
   caggcccaag aattacaggc aaacaaagag gctgtgcatc agttaaaacg aaagtttcta 300
   gtcagcccgc gaagcagccc attaggagac attacaaatc aaaacaacac acacagccat 360
   agtcaggcaa acgagtcaca agttaaaagg agattactgg acagttatcc ggacagcgga 420
   tatggcaata cacaagtgga aactgtggaa gcaacgttgc aggtagatgg gcaacatggc 480
   ggttcacaga acagtgtgtg tagtagcggg gggggcagtg ttatggatgt ggaaacaaca 540
   gaaagctgtg caaatgtaga actaaacagt atatgtgaag tattaaaaag cagtaatgca 600
   aaagcaacgt taatggcaaa atttaaagag ttgtatggta ttagttataa tgagttggta 660
   cgggtgttta aaagtgataa aacatgttgt atagattggg tttgtgcatt gtttggcgtt 720
   tccccaatgg tagcagaaaa tttaaaaaca ctaattaagc cattttgcat gtactaccat 780
   atacaatgtt tatcatgtga ttggggcacc attgtattaa tgctaattag gttttcatgt 840
   gcaaaaaaca gaacaacaat tgctaagtgt ttaagtacat tagtaaatat cccacaatca 900
   caaatgttta tagaaccacc aaaattacgt agtacacctg tggcattata tttttataga 960
   acaggcatat caaacattag caatacatat ggagagacac ctgaatggat tacacgacaa 1020
   acgcaactac aacatagttt tgaggatagt acctttgaat tatcacaaat ggtgcaatgg 1080
   gcatttgacc atgaagtatt agatgatagt gaaatagcat ttcattatgc acaattagca 1140
   gatatagata gtaatgctgc agcgttttta aagagtaatt gccaagcaaa atatgtaaaa 1200
   gattgtggga ccatggcacg gcattacaaa cgagcacaaa gaaaatcatt atctatgtca 1260
   gcctggataa ggtatagatg tgatagagca aaggatggag gcaactggag agaaattgct 1320
   aaatttttaa gatatcaagg tgtaaacttt atgtccttta ttcaaatgtt taaacagttt 1380
   ttaaaaggaa caccaaaaca caattgcata gtcatatatg gcccaccaaa cacaggcaag 1440
   tcattatttg caatgagcct aatgaagttt atgcaagggt ccattatttc atatgtaaac 1500
   tctggtagtc atttttggtt acagccacta gaggatgcta aaatagcatt gttagatgat 1560
   gctacgtatg ggtgttggac atatattgat cagtatttaa gaaacttttt agatggtaat 1620
   ccatgtagta tagatagaaa acataggagt ttaatacaat tagtatgtcc accattacta 1680
   ataacgtcaa acataaatcc acaagaggat gcaaacctaa tgtatttaca tacaagggta 1740
   acagtattaa agtttttaaa tacatttcca tttgataaca atgggaatgc tgtgtataca 1800
<210> 58
   <211> 21
   <212> DNA
   <213> artificial
<220>
   <223> sequence to which the reverse primer for HPV 51 hybridises
<400> 58
   actggacagt tatccggaca g 21
<210> 59
   <211> 1943
   <212> DNA
   <213> artificial
<220>
   <223> HPV 52 nucleotides 864 - 2807 E1 gene sequence
<400> 59
   tggaggaccc tgaaggtaca gagggcgaaa gggagggatg tacaggctgg tttgaagtaa     60
   aggcaataat agaaaaacaa acaggagata acatttcaga ggacgaggat gaaaatgcat    120
   atgatagtgg aacagatcta atagatttta tagatgattc aaatataaat aatgaacagg    180
   cagaacatga ggcagcccgg gcattgttta atgcacagga aggggaggat gatttacatg    240
   ctgtgtctgc agtaaaacga aagtttacaa gcagtccgga aagtgctggg caagatggtg    300
   tagaaaaaca tggtagtccg cgtgcaaaac acatttgtgt aaatacagag tgtgttttac    360
   caaaacgcaa accatgtcac gtagaagaca gcggctatgg caatagtgaa gtggaagcgc    420
   agcagatggc agaccaggta gacgggcaaa atggcgactg gcaaagtaac agtagtcaat    480
   caagtggggt gggggctagt aattcagatg taagttgtac tagtatagag gacaatgagg    540
   aaaatagtaa tagaacgcta aaaagcatac aaaatattat gtgcgaaaat agcataaaaa    600
   caacggtatt atttaaattt aaagaaacat atggtgttag ctttatggaa ttagtaagac 660
   catttaaaag taatagaagt agttgtacag attggtgtat tataggaatg ggagtaacac 720
   catcagttgc agaaggatta aaagtattaa tacagcccta tagcatatat gcccatttgc 780
   aatgtttaac atgtgacaga ggcgtgctta tactgctgct aattaggttt aaatgtggaa 840
   aaaacagatt aacagtgtcc aaactaatgt cacagctgtt aaatatacca gaaacacata 900
   tggtaataga accaccaaaa ttacgaagtg ctacctgtgc attatattgg tatagaacag 960
   gtttgtctaa tattagtgag gtatatggta ccaccccaga atggatagaa caacaaacag 1020
   tattacagca tagctttgac aatagcatat ttgattttgg agaaatggtg caatgggcat 1080
   atgatcatga tataacagat gatagtgaca tagcatataa atatgcacag ttagcagatg 1140
   taaatagcaa tgctgcagca ttcctaaaaa gcaattcgca agcaaaaata gtaaaggact 1200
   gtgcaaccat gtgtagacat tataaacggg cagaaagaaa acatatgaat attggacaat 1260
   ggatacagta tagatgtgat agaatagatg atggtggaga ttggaggcct atagtaagat 1320
   ttttaagata tcaagacata gaatttacag cctttttaga cgcatttaaa aaatttttaa 1380
   aaggtatacc taaaaaaaat tgtttagtat tatatggacc tgcaaacaca ggaaaatcat 1440
   attttggaat gagtttaatt aggttcttaa gtggatgtgt aatatcctat gtaaactcaa 1500
   aaagccattt ttggctacaa ccattaacag atgcaaaagt gggtatgata gatgatgtaa 1560
   cacctatatg ttggacatat atagatgatt atatgagaaa tgcactggat ggaaatgata 1620
   tatcagtaga tgtaaagcat agagccttag tacaaataaa atgcccacca ttaattttaa 1680
   caacaaatac aaatgcagga acagatccta ggtggccata tttacatagt agattggttg 1740
   tgtttcattt caaaaaccca tttccatttg atgaaaatgg caatcccata tatgaaatta 1800
   acaacgaaaa ttggaaatcc tttttctcaa ggacgtggtg caaattagat ttaatacagg 1860
   aagaggacaa ggaaaacgat ggagtcgata ccggcacgtt taaatgcagt gcaggaaaaa 1920
   atactagatc tatacgaagc tga 1943
<210> 60
   <211> 21
   <212> DNA
   <213> artificial
<220>
   <223> Region of HPV 52 that is targeted by the reverse primer
<400> 60
   agacagcggc tatggcaata g 21
<210> 61
   <211> 21
   <212> DNA
   <213> artificial
<220>
   <223> the reverse complement of SEQ ID NO: 60
<400> 61
   ctattgccat agccgctgtc t 21
<210> 62
   <211> 1911
   <212> DNA
   <213> artificial
<220>
   <223> HPV 56 nucleotides 895 - 2805 E1 gene sequence
<400> 62
   atggcgtcac ctgaaggtac agatggggag gggaagggat gttgtggatg gtttgaagta      60
   gaggcaattg tagaaaaaaa aacaggagat aaaatatcag atgatgaaag tgacgaggag     120
   gatgaaatag atacagattt agatggattt atagacgatt catatataca aaatatacag     180
   gcagacagag agacagctca acaattgttg caagtacaaa cagcacatgc agataaacag     240
   acgttgcaaa aactaaaacg aaagtatata gctagtccat taagtgatat tagtaatcag     300
   caaactgtgt gccgggaagg agtaaaacgg aggcttattt tatcagacct acaagacagc     360
   gggtatggca atacattgga aactctggaa acaccagaac aggtagatga agaggtacag     420
   ggacgtgggt gcgggaatac acaaaatgga ggctcacaaa acagtaccta tagtaacaat     480
   agtgaggact ctgtaataca tatggatatt gatagaaaca atgaaacgcc aacacaacaa     540
   ttgcaggact tgtttaaaag tagcaattta caaggtaaat tatattataa atttaaagaa     600
   gtgtatggta ttccattttc agaattggtg cgtacgttta aaagtgatag tacatgttgc     660
   aatgattgga tatgtgctat atttggtgtt aatgaaacat tagccgaggc actaaaaact     720
   ataataaaac cacactgtat gtattatcat atgcaatgtt taacatgtac atggggggtt     780
   atagtaatga tgctaattag atatacatgt ggcaaaaaca gaaaaacaat tgcaaaagca     840
   ttaagctcaa tattaaatgt accacaggag caaatgttaa ttcaaccacc aaaaatacga     900
   agtcctgctg tagctttata tttttataaa acagcaatgt caaatattag tgatgtgtat     960
   ggagacacac cagaatggat acaaagacaa acacaattgc aacacagttt acaggatagt    1020
   caatttgaat tatctaaaat ggtgcagtgg gcatttgata atgaagtaac agatgatagc    1080
   caaattgcgt ttcaatatgc acaattagca gatgtagaca gcaatgcaca agccttttta    1140
   aaaagcaata tgcaggcaaa atatgtaaag gattgtggaa taatgtgtag acattataaa    1200
   agggcacaac agcaacaaat gaatatgtgc cagtggataa agcacatatg tagtaaaaca    1260
   gatgaagggg gtgattggaa acccattgta caatttttaa gatatcaagg ggtcgatttc    1320
   atttcatttc taagttactt taaattattt ctacaaggaa cacctaaaca taactgtttg    1380
   gtactttgtg gaccgccaaa tacaggtaaa tcatgctttg ctatgagtct tataaagttt     1440
   tttcaagggt ctgtcatttc atttgtgaat tcacaaagcc acttttggtt gcagccatta     1500
   gacaatgcta aacttgggtt gttggatgat gcaacagaaa tatgttggaa atatatagac     1560
   gattatttaa ggaatttggt agatggaaat cctataagtt tagatagaaa acataaacaa     1620
   ttagtacaaa taaaatgtcc accattacta attacaacca atataaatcc tatgctagat     1680
   gctaaattac gatatttaca cagtagaatg ttagtgtttc agtttcaaaa tccatttcca     1740
   ttagataata atggtaatcc tgtatatgaa ttaagtaatg taaactggaa atgtttcttt     1800
   acaaggacgt ggtccagatt aaatttggat aacgacgagg acaaagaaaa caatggagac     1860
   gctttcccaa cgtttaaatg tgtgccagaa caaaatacta gactgttttg a              1911
<210> 63
   <211> 1893
   <212> DNA
   <213> artificial
<220>
   <223> HPV 66 nucleotides 895 - 2787 E1 gene sequence
<400> 63
   atggcatcac ctgaaggtac aaatggggag gggacgggat gttgtggatg gtttcaggta      60
   gaagcaattg tagaaagaaa aacgggggat acaatatcag atgatgaaag cgaggaggag     120
   aatgaaacag atacagattt agatggattt atagacaata cacttataaa caatacacag     180
   gaagacaggg agacagctca acaattattg caagtacaaa cagcacatgc agatgcacag     240
   acgttgcaaa aactaaaacg aaagtatata ggtagtccct taagtgatat tagtaatcag     300
   caaactgtgt accgggagga agtaaaacga aggctaatat tatcagaaga cagtgggtat     360
   ggcaatacat tggaaacatt ggaaacatca caacaggtag aatacgaaaa gggaaatggg     420
   tgcgggagct cacaaaatgg aggctcgcaa aacagtaact gtagtgagca ctcggtggta     480
   aatatggata tagatacaaa tatggaaaca ccaacacacc aattgcagga actatttaaa     540
   agtagtaacg cacaaggaag attacatttt aaatttaaag aagtgtatgg agtgccatat     600
   acagagttgg tgcgaacatt taaaagcgat agtacatgtt gtaacgattg gatatgtgca     660
   atatttggtg ttaatgaaac attagcagaa gcgttaaaaa ctatactaaa accacaatgt     720
   gtgtactatc atatgcaatg cctaacatgt tcatgggggg taattgtaat gatgctaatt     780
   agatatatat gtggaaaaaa cagaaaaaca attacaaaat cgctaagctc aattttaaat     840
   gtaccacaag agcaaatgtt aattcaacca ccaaaactac gaagtcctgc tgtggcatta     900
   tatttttata aaacagcaat gtcaaatatt agtgaggtgt atggggaaac accagaatgg     960
   atacaaagac agacacaatt gcaacacagt ttacaagaca atcaatttga attgtctaaa    1020
   atggtacagt gggcatttga taatgacgta acagatgata gccaaattgc ctttttatat    1080
   gcacaactag cggacataga tagtaatgca caagcatttt tagaaagtaa tatgcaagca 1140
   aaatatgtaa aggattgtgg aataatgtgt agacattata aaagggcaca gcaacagcaa 1200
   atgaatatgt gccagtggat aaagcatata tgtagtaaag tagatgaagg gggtgattgg 1260
   aaacccattg cgcaattttt acgatatcaa ggggtcgact tcatttcatt tttaagttat 1320
   tttaaattat ttttacaagg cacgcctaaa cataattgtt tggtactgtg tggaccacca 1380
   aatacaggta aatcatgttt tgctatgagc cttataaatt ttttccaagg gtcagtcatt 1440
   tcatttgtta attcacaaag ccacttttgg ttacagccac tagacaatgc caaattaggg 1500
   ttgctggatg atgcaacaga tacgtgttgg agatatatag atgattatct aagaaattta 1560
   ttagatggga atcccataag tttagatagg aaacataaac aattagtaca aataaaatgt 1620
   cctccagtca ttattacaac taatgtaaat cctatgcaag atgcaaaatt aagatatcta 1680
   cacagtagaa tttcagtgtt taagtttgaa aatccatttc cattagataa caatggtaat 1740
   cctgtgtatg aattgagtaa tgtaaattgg aaatgttttt ttgaaaggac atggtccaga 1800
   ttaaattcgg ataacgacga ggacaaagaa aacaatggag actctatccc aacgtttaga 1860
   tgcgtgccag aacaaaatac taggctgtta tga 1893
<210> 64
   <211> 21
   <212> DNA
   <213> artificial
<220>
   <223> target sequence of HPV 66 for the reverse primer. HPV16/31/33/33N/35/39/52/56/58/66R
<400> 64
   agacagtggg tatggcaata c 21
<210> 65
   <211> 21
   <212> DNA
   <213> artificial
<220>
   <223> reverse complement of SEQ ID NO: 64
<400> 65
   gtattgccat acccactgtc t 21
<210> 66
   <211> 1935
   <212> DNA
   <213> artificial
<220>
   <223> HPV 58 nucleotides 883 - 2817 E1 gene sequence
<400> 66
   atggatgacc ctgaaggtac aaacggggta ggggcgggct gtactggctg gtttgaggta 60
   gaagcagtaa tagaacgaag aacaggagat aatatttcag atgatgagga cgaaacagca 120
   gacgatagtg gtacagattt aatagagttt atagatgatt cagtacaaag tactacacag 180
   gcagaagcag aggcagcccg agcgttgttt aatgtacagg aaggggtgga cgatataaat 240
   gctgtgtgtg cactaaaacg aaagtttgca gcatgctcag aaagtgctgt agaggactgt 300
   gtggaccggg ccgcaaatgt gtgtgtatcg tggaaatata aaaataaaga atgcacacac 360
   agaaaacgaa aaattattga gctagaagac agcggatatg gcaatactga agtggaaact 420
   gagcagatgg cacaccaggt agaaagccaa aatggcgacg cagacttaaa tgactcgcag 480
   tctagtgggg tgggggctag ttcagatgta agcagtgaaa cggatgtaga cagttgtaat 540
   actgttccat tacaaaatat tagtaatatt ttacataaca gtaatactaa agcaacgcta 600
   ttatataaat tcaaagaagc ttatggagta agttttatgg aattagttag accatttaaa 660
   agtgataaaa caagctgtac agattggtgt ataacagggt atggaataag tccctccgta 720
   gcagaaagtt taaaagtact aattaaacag cacagtatat atacacacct acaatgttta 780
   acgtgtgaca gaggaattat attattattg ttaattagat ttaaatgtag caaaaataga 840
   ttaactgtgg caaaattaat gagtaattta ctatcaattc ctgaaacatg tatgattatt 900
   gagccaccaa aattacgaag tcaagcatgt gccttatatt ggtttagaac agcaatgtca 960
   aatataagtg atgtgcaagg gacaacacca gaatggatag atagattaac agtgttacag 1020
   catagcttta atgatgatat atttgattta agtgaaatga tacaatgggc atatgataat 1080
   gacattacag atgatagtga cattgcatat aaatatgcac agttagcaga tgttaatagt 1140
   aatgcagcag catttttaag aagcaatgca caagcaaaaa tagtaaaaga ctgtggcgtt 1200
   atgtgcagac attataaaag agcagaaaag cgtggtatga caatgggaca atggatacaa 1260
   agtaggtgtg aaaaaacaaa tgatggaggt aattggagac caatagtaca atttttaaga 1320
   tatcaaaata ttgaatttac agcattttta gttgcattta aacagttttt acaaggtgta 1380
   ccaaaaaaaa gttgtatgtt actgtgtggc ccagcaaata cagggaaatc atattttgga 1440
   atgagtttaa tacatttctt aaaaggatgc attatttcat atgtaaattc caaaagtcat 1500
   ttttggttgc agccattatc agatgctaaa ctaggtatga tagatgatgt aacagccata 1560
   agctggacat atatagatga ttatatgaga aatgcattag atggtaacga catttcaata 1620
   gatgtaaaac atagggcatt agtacaatta aaatgtccac cattaataat tacctcaaat 1680
   acaaatgcag gcaaagattc acgatggcca tatttgcaca gtagactaac agtatttgaa 1740
   tttaacaatc catttccatt tgatgcaaat ggtaatccag tgtataaaat aaatgatgaa 1800
   aattggaaat cctttttctc aaggacgtgg tgcaaattag gcttaataga ggaagaggac 1860
   aaggaaaacg atggaggaaa tatcagcacg tttaagtgca gtgcaggaca aaatcctaga 1920
   catatacgaa gctga 1935
<210> 67
   <211> 1934
   <212> DNA
   <213> artificial
<220>
   <223> HPV 59 nucleotides 872 - 2806 E1 gene sequence
<400> 67
   tggccgattc ggaaggtaca gatggggaag ggacggggtg caatggatgg ttttttgtgc 60
   aggcaatagt agataaaaaa acaggtgaca aaatttcaga tgacgaggat gaaaatgcaa 120
   cagatacagg ttcagacttg gtagacttta ttgatgatac cacaacaatt tgtgtacagg 180
   cagagcgcga gacagcacag gccttgttta atgtgcagga agcccaaagg gatgcacggg 240
   aaatgcatgt tttaaaacga aagtttgggt gcagtataga aaacagtagt gagaaagcgg 300
   cggcaggaac aaaagctaag tcaccattac aagaaatatc agtaaatgtt aaccacccaa 360
   aagtaaagag aaggttacta acagtgccag acagcggcta tggctattct gaagtggaaa 420
   tgctcgagac tcaggtaacc gtggagaata ctggaaatgg ggacagcaat ggcagtgttt 480
   gtagcgacag tcaaatagac ggtagcgaca gcagtaacat ggatgttgaa aacatagttc 540
   caacatcccc cactaatcaa ttgttacagt tattacatag caaaaataag aaagcagcta 600
   tgtatgcaaa atttaaagaa ttgtatgggt tatcatttca agatttggtt aggacattta 660
   aaagtgacag aactacctgt agcgattggg taaccgccat ttttggtgta aatccaactg 720
   tagcagaagg atttaaaaca ttaatacaac cctatgtgct atatgcacat atacaatgtt 780
   tagattgtgc atggggagta gtaatattag cattattaag atataaatgt ggaaaaaata 840
   gaataacagt tgcaaaagga cttagcacat tactacatgt accagatacg tgcatgttaa 900
   ttgaaccacc caaattgcgt agtggtgttg cagcattata ttggtacaga acaggaatgt 960
   ccaatattag tgaagttata ggggaaacgc ccgaatggat acaaagacta acaattatac 1020
   aacatggagt tgatgatagc gtgtttgacc tgtcagaaat gatacaatgg gcgtttgata 1080
   atgacctaac agatgaaagt gatattgcat atgaatatgc attaatagca gatagtaata 1140
   gtaacgccgc tgcattttta aaaagcaact gccaggcaaa atacctaaaa gattgtgcag 1200
   ttatgtgtag gcattataaa agagcacaaa aaagacaaat gagtatgtca cagtggataa 1260
   aatggagatg tgataaaata gaagaggggg gagattggaa acccatagta caatttttaa 1320
   gatatcaagg agtagaattt ataacgtttt tatgtgcatt aaaagatttt ttaaaaggta 1380
   ccccaaaaag aaattgcatt gtgctgtgtg ggccagcaaa tacaggcaag tcatactttg 1440
   gaatgagcct gctacatttc ttacaaggaa ctgtaatttc acatgtaaat tcaaatagtc 1500
   acttttggct agaaccttta acagatgcta aattagctat gctagacgat gcaacagata      1560
   gttgttggac atattttgat acatatatgc gaaatgctct ggatggcaat cctataagtg      1620
   tagatagaaa gcataggcac ctagtacaaa ttaaatgtcc accaatgctt attacatcaa      1680
   atacaaatcc agttacagat aacaggtggc catacctaaa tagcagacta atggtattta      1740
   aatttccaaa caaatttcca tttgacaaaa atagaaatcc agtatataca attaatgaca      1800
   gaaactggaa atgttttttt gaaaggacgt ggtgcagatt agatttgaac gaggaagagg      1860
   atgatgcaga cagtgatgga caccctttcg cagcgtttaa gtgtgttaca ggatcaaata      1920
   ttagaacatt atga                                                        1934
<210> 68
   <211> 24
   <212> DNA
   <213> artificial
<220>
   <223> region targeted by the reverse primer for HPV 59, m denotes a nucleotide change c/a
<400> 68
   gaaggttamt aacagtgcca gaca 24
<210> 69
   <211> 1923
   <212> DNA
   <213> artificial
<220>
   <223> HPV 68 nucleotides 824 - 2746 E1 gene sequence
<400> 69
   atggccaatt gtgaaggtac agatggggac gggacggggt gtaacggatg gtttttcgta        60
   caagcaatag tagataaaca aacaggtgac acagtctcag aggatgagga tgaaaacgcg       120
   acagatacag gttcagacat ggtagatttc attgatgatg ctacagatat ttgtatacag       180
   gcagagcgtg agacagcaca ggtactgtta aatatgcaac aggcccaaag ggatgcacaa       240
   acagtgcgtg ccctaaaacg aaagtataca gacagtatag aaagcagccc tttagcaaag       300
   tcgccattac aggaactatc aataaatgta agcagtacac aggcaagaca accggcgtat       360
   acagtgccgg acagcggcta tggcaatatg gaagtggaaa ctcactcgga ggtaactgta       420
   gcaactaata caaatggggc ggacggggag gatgaagggg aaaatggcga cagcatacgg       480
   gaggactgta gtagtgtaga cagtgctata gatagtgaaa accaggatcc taaatcacct       540
   actactcaac taaaagtatt attacaatgt aataataaaa aagctgcaat gttaacagaa       600
   tttaaaaaag tatatggatt gtcctttaat gacctagtac gtacatttaa aagtgataag       660
   accacatgta cggactgggt agcagcaata tttggagtaa atccaaccat tgccgaaggg       720
   tttaaaacac taattaaaca atatgcatta tatacccata tacaatgttt agatacaaaa     780
   aacggaatat taatattaat gttaataaga tataaatgtg ggaaaaatag aataacagta     840
   ggaaaaggat taagtacatt gttgcatgtt ccagacagct gtatgctttt gcagccacca     900
   aaattgcgta gccctgttgc agcattgtat tggtatagaa caggaatatc taatattagt     960
   gaggtgtgtg gagacacgcc agaatggata aaaagattaa ctataataca acatggaata    1020
   gatgatagtg tatttgatct atcagacatg gtacaatggg catttgataa tgagttaaca    1080
   gatgaaagtg atatagcatt ttcatatgct atgttggcag attgtaatag taatgctgca    1140
   gcgtttttaa aaagcaactg ccaagcaaaa tatgtaaaag attgtgcaac aatgtgtaga    1200
   cattacaaac gggcacaaaa acgacaaatg tcaatgccgc aatggattaa attcagatgc    1260
   agtaaatgtg atgaaggtgg tgattggcga ccaattgtac agtttttaag atatcagggg    1320
   ctagaattca taacattctt atgtgcatta aaagattttt taaaaggcac gccaaaacgt    1380
   aattgtatag ttatacatgg gccaccaaat actggcaagt catatttttg catgagtctt    1440
   atacatttct tgcaaggcac aataatttca tatgtaaatt cagctagtca cttttggtta    1500
   gagccacttg cagatgcaaa aatagccatg ttagatgacg caacaggtac atgttggtca    1560
   tattttgata attacatgag aaatgcatta gatggtaacc caataagttt agatagaaaa    1620
   cacagacacc taatacaaat aaaatgtcca ccaatgctaa taacatcaaa tactaaccct    1680
   gtggaagaca ataggtggcc gtatttacat agcagactaa ctgtatttaa atttcctaat    1740
   gcatttccat ttgaccaaaa caggaaccca gtgtatacaa tgaatgataa aaactggaaa    1800
   tgtttttttg aaaagacttg gtgcaaatta gacttgcagc aggacgagga tgaaggagac    1860
   aatgatgaaa acactttccc aacgtttaaa tgtgttacag gagaaaatat tagaacatta    1920
   tga                                                                  1923
<210> 70
   <211> 21
   <212> DNA
   <213> artificial
<220>
   <223> region of HPV68 to which the reverse primer for HPV68 hybridises
<400> 70
   agcaaagtcg ccattacagg a 21
<210> 71
   <211> 24
   <212> DNA
   <213> artificial
<220>
   <223> Target sequence for HPV 16 R HEX
<400> 71
   ataatgatta tttaacacag gcag 24
<210> 72
   <211> 21
   <212> DNA
   <213> artificial
<220>
   <223> Target sequence for HPV18 R HEX
<400> 72
   aaggaacatt ttgtgaacag g 21